(19) Europäisches Patentamt
European Patent Office
Office européen des brevets

(11) EP 2 385 124 B1

(12) **EUROPEAN PATENT SPECIFICATION**

(45) Date of publication and mention of the grant of the patent:
**11.09.2013 Bulletin 2013/37**

(51) Int Cl.:
***C07K 14/705** (2006.01)*

(21) Application number: **10186068.2**

(22) Date of filing: **12.05.2000**

(54) **Peptides or peptide analogues for modulating the binding of a PDZ protein and a PL protein**

Peptide oder Peptidanaloga zur Modulation der Bindung zwischen einem PDZ-Protein und einem PL-Protein

Peptides ou analogues de peptides pour moduler l'interaction entre und protéine PDZ et une protéine PL

(84) Designated Contracting States:
**AT BE CH CY DE DK ES FI FR GB GR IE IT LI LU MC NL PT SE**
Designated Extension States:
**AL LT LV MK RO SI**

(30) Priority:
**14.05.1999 US 134114 P**
**14.05.1999 US 134117 P**
**14.05.1999 US 134118 P**
**21.10.1999 US 160860 P**
**29.10.1999 US 162498 P**
**13.12.1999 US 170453 P**
**14.01.2000 US 176195 P**
**14.02.2000 US 182296 P**
**11.04.2000 US 547276**
**11.04.2000 US 196460 P**
**11.04.2000 US 196528 P**
**11.04.2000 US 196527 P**
**11.04.2000 US 196267 P**

(43) Date of publication of application:
**09.11.2011 Bulletin 2011/45**

(62) Document number(s) of the earlier application(s) in accordance with Art. 76 EPC:
**00932386.6 / 1 178 817**

(73) Proprietor: **Arbor Vita Corporation**
**Fremont, CA 94555 (US)**

(72) Inventor: **Lu, Peter**
**Fremont, CA 94555 (US)**

(74) Representative: **Wilkinson, Marc George et al**
**avidity IP**
**Kestrel House**
**Falconry Court**
**Baker's Lane**
**Epping, Essex CM16 5DQ (GB)**

(56) References cited:
**WO-A1-98/23781**
**WO-A1-2005/068496**
**WO-A2-99/06551**
**WO-A2-99/40189**
**CA-A1- 2 273 622**
**FR-A1- 2 717 081**
**US-A- 5 877 282**

- **DATABASE PROTEIN [Online] NCBI; 19 March 1999 (1999-03-19), "Glutamate receptor, ionotropic, N-methyl D-aspartate 2A [Homo sapiens]", XP002659803, Database accession no. NP_000824.1**
- **VIVES E ET AL: "A TRUNCATED HIV-1 TAT PROTEIN BASIC DOMAIN RAPIDLY TRANSLOCATES THROUGH THE PLASMA MEMBRANE AND ACCUMULATES IN THE CELL NUCLEUS", JOURNAL OF BIOLOGICAL CHEMISTRY, THE AMERICAN SOCIETY OF BIOLOGICAL CHEMISTS, INC, US, vol. 272, no. 25, 20 June 1997 (1997-06-20), pages 16010-16017, XP002940007, ISSN: 0021-9258, DOI: 10.1074/JBC.272.25.16010**
- **MORRIS M C ET AL: "A peptide carrier for the delivery of biologically active proteins into mammalian cells", NATURE BIOTECHNOLOGY, NATURE PUBLISHING GROUP, NEW YORK, NY, US, vol. 19, 1 December 2001 (2001-12-01), pages 1173-1176, XP002559236, ISSN: 1087-0156, DOI: 10.1038/NBT1201-1173**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 385 124 B1

- VIVES E ET AL: "TAT PEPTIDE INTERNALIZATION: SEEKING THE MECHANISM OF ENTRY", CURRENT PROTEIN AND PEPTIDE SCIENCE, BENTHAM SCIENCE PULBISHERS, NL, vol. 4, no. 2, 1 April 2003 (2003-04-01), pages 125-132, XP001204952, ISSN: 1389-2037, DOI: 10.2174/1389203033487306

## Description

### 1. FIELD OF THE INVENTION

The present invention relates to peptides and peptide analogues, and methods for using such compositions to regulate activities of cells of the hematopoietic system. In one aspect, the invention provides methods of modulating metabolism (e.g., activation) of hematopoietic cells (e.g., T cells and B cells) by antagonizing an interaction between a PDZ domain containing protein and a protein that binds a PDZ domain. In one aspect, it relates to fusion peptides containing an amino acid sequence corresponding to the carboxyl terminus of a surface receptor expressed by a hematopoietic cell and a transmembrane transporter sequence; such fusion peptides are useful in regulating hematopoietic cells by inhibiting cell activation.

### 2. BACKGROUND OF THE INVENTION

PDZ domains of proteins are named after three prototypical proteins: PSD95, Drosophila large disc protein and Zonula Occludin 1 protein (Gomperts et al., 1996, Cell 84:659-662). PDZ domain-containing proteins are involved in synapse formation by organizing transmembrane neurotransmitter receptors through intracellular interactions. PDZ domains contain the signature sequence GLGF. In the nervous system, typical PDZ domain-containing proteins contain three PDZ domains, one SH3 domain and one guanylate kinase domain. Examples of intracellular PDZ domain-containing proteins include LIN-2, LIN-7 and LIN-10 at the pre-synapse, and PSD95 at the post-synapse.

PDZ domains have been shown to bind the carboxyl termini of transmembrane proteins in neuronal cells. Songyang et al. reported that proteins capable of binding PDZ domains contain a carboxyl terminal motif sequence of E- S/T- X- V/I (Songyang et al., 1997, Science 275: 73) . X- ray crystallography studies have revealed the contact points between the motif sequence and PDZ domains (Doyle et al., 1996, Cell 88: 1067- 1076) . While the interaction between PDZ domains and ion channels in neurons have been studied extensively, such interactions have had limited studies in other biological systems, especially the hematopoietic system.

The hematopoietic system is composed of different cell types that perform distinct functions. Many of its diverse function requires coordinated movement of cell surface receptors including ion channels, adhesion surface molecules to coordinate cell-cell interaction, and cytokine receptors. Despite their diverse functional activities, all hematopoietic cells are believed to develop from a multipotent bone marrow hematopoietic stem cell. Such stem cell has been shown to express a surface marker termed CD34. During differentiation, the stem cell gives rise to progenitor cells in each of several specific hematopoietic cell lineages. The progenitor cells then undergo a series of morphological and functional changes to produce mature functionally committed hematopoietic cells.

Among the functions performed by hematopoietic cells, certain cell types are involved exclusively in immunity. For example, lymphocytes, which include T cells, B cells and natural killer (NK) cells, are effectors in immune responses. Monocytes and granulocytes (i.e., neutrophils, basophils and eosinophils) play a role in non-specific forms of defense. Lymphocytes, monocytes and granulocytes are collectively referred to as white blood cells or leukocytes. On the other hand, other hematopoietic cells perform functions that are unrelated to the immune system. For example, erythrocytes are involved in gas transport, and cells of the thrombocytic series are involved in blood clotting.

T cells and B cells recognize antigens and generate an immune response. T cells recognize antigens by heterodimeric surface receptors termed the T cell receptor (TCR). The TCR is associated with a series of polypeptides collectively referred to as CD3 complex. B cells recognize antigens by surface immunoglobulins (Ig), which are also secretory molecules. In addition, a large number of co-stimulatory surface receptors have been identified in T cells and B cells, which augment cellular activation during antigen-induced activation.

In addition to the T cell antigen receptor/CD3 complex (TCR/CD3), other molecules expressed by T cells which mediate an activation signal, include but are not limited to, CD2, CD4, CD5, CD6, CD8, CD18, CD27, CD28, CD43, CD45, CD152 (CTLA-4), CD154, MHC class I, MHC class II, CDw137 (4-1BB), CDw150, and the like (Barclay et al., The Leucocyte Antigen Facts Book, 1997, Second edition, Academic Press; Leucocyte Typing, 1984, Bernard et al. (eds.), Springer-Verlag; Leukocyte Typing II, 1986, Reinherz et al. (eds.), Springer-Verlag; Leukocyte Typing III, 1987, McMichael (ed.), Oxford University Press; Leukocyte Typing IV, 1989, Knapp et al. (eds.), Oxford University Press; CD Antigens, 1996, VI Internat. Workshop and Conference on Human Leukocyte Differentiation Antigens. http://www.ncbi.nlm. nih.gov/prow). Cell surface antigens that work together with TCR/CD3 are often referred to as co-receptors in the art.

Specific antibodies have been generated against all of the aforementioned T cell surface antigens. Other molecules that bind to the aforementioned T cell surface receptors include antigen-binding antibody derivatives such as variable domains, peptides, superantigens, and their natural ligands such as CD58 (LFA-3) for CD2, HIV gp120 for CD4, CD27L for CD27, CD80 or CD86 for CD28 or CD152, ICAM1, ICAM2 and ICAM3 for CD11a/CD18, 4-1BBL for CDw137.

Activation molecules expressed by B cells, include but are not limited to, surface Ig, CD18, CD19, CD20, CD21, CD22, CD23, CD40, CD45, CD80, CD86 and ICAM1. Similarly, natural ligands of these molecules and antibodies directed to them as well as antibody derivatives may be used to deliver an activation signal to B cells.

However, prior to the present invention, it was not known that signal transduction following stimulation of any leukocyte receptor was mediated by receptor interactions with PDZ domain- containing proteins. Therefore, it was not even contemplated in the art that an interference of leukocyte surface receptor/PDZ domain interactions could regulate leukocyte activation.

Document WO 98/23781 A1 discloses peptides modulating the binding of a PDZ protein comprising a C-terminal PDZ-domain binding sequence D-X-V.

## 3. **SUMMARY OF THE INVENTION**

The present invention discloses a fusion peptide or peptide analogue thereof for modulating the binding of a PDZ protein and a PL protein comprising

(i) a transmembrane transporter amino acid sequence capable of facilitating the entry of a peptide linked thereto into a cell through the plasma membrane,
(ii) an optional, flexible polylinker peptide sequence, and
(iii) a carboxyl terminal peptide sequence comprising a PDZ domain binding motif at the C- terminal, the PDZ domain binding motif sequence comprising
X*- S/T- D/E- V/I/L, wherein X* is any non- aromatic amino acid.

Preferred embodiments of the invention are described in the appended claims.

Also described herein is a method of modulating a biological function of a cell, e.g., an endothelial cell or hematopoietic cell (such as a a leukocyte, e.g., T cell or B cell) , by introducing into the cell an antagonist that inhibits binding of a PDZ protein and a PL protein in the cell, or a agonist that enhances binding of a PDZ protein and a PL protein in the cell. In various embodiments the PL protein is an adhesion protein, an adaptor protein, or an intracellular protein. In embodiments it is CD6, CD49E, CD49F, CD138, Clasp- 1, Clasp- 4, VCAM1, Clasp- 2, CD95, DNAM- 1, CD83, CD44, CD4, CD97, CD3n, DOCK2, CD34, FceRIb, or FasLigand. In an embodiment the PL protein is characterized by a carboxy- terminal amino acid motif that is X- S- X- A, X- A- D/E- V, X- V/I/L- X*- V, or X- S/T- X- F (where X is any amino acid and X* is any non- aromatic amino acid) . In embodiments, the PL protein is expressed by T lymphocytes or B lymphocytes. In some embodiments of this method, the PDZ protein is CASK, MPP1, DLG1, PSD95, NeDLG, SYN1a, TAX43, LDP, LIM, LIMK, AF6, PTN- 4, prIL16, 41.8, RGS12, DVL1, TAX 40, TIAM1, MINT1, K303, TAX2, or KIAA561.

In some aspects, the cell is a leukocyte and the biological function is cell activation, cell proliferation, maintenance of cell structure, cell metabolic activity, or cytokine production. In some embodiments, the method further includes detecting a change in leukocyte activation.

In preferred aspects, the antagonist is an agent that inhibits the binding of a PL peptide to a PDZ domain polypeptide in an "A" assay, in a "G" assay, or in both an A assay and a G assay. The antagonist can be a polypeptide, such as a polypeptide having at the carboxyterminus at least two residues that are the same as the carboxy- terminal two residues of a PL protein, such as a PL protein is expressed in a hematopoietic or endothelial cell that is an adhesion protein, an adaptor protein, or an intracellular protein. In a further aspect, at least the carboxy- terminal four residues of the polypeptide are the same as the carboxy- terminal four residues of the PL protein. In a further aspect, the PL protein has a carboxy- terminal amino acid motif selected from X- S- X- A, X- A- D/E- V, X- V/I/L, -X*- V, or X- S/T- X- F, where X is any amino acid and X* is any non- aromatic amino acid. In a further aspect, the PL protein is CD6, CD49E, CD49F, CD138, Clasp- 1, Clasp- 4, VCAM1, Clasp- 2, CD95, DNAM- 1, CD83, CD44, CD97, CD3n, DOCK2, CD34, FceRIb, or FasLigand.

In a related aspect, the antagonist is a peptide mimetic of a PL inhibitor sequence peptide. In another related aspect the antagonist is a fusion polypeptide having a PL sequence and transmembrane transporter amino acid sequence (such as HIV tat, Drosophila antenapedia, herpes simplex virus VP22 or anti-DNA CDR 2 and 3).

In another aspect, the disclosure provides a method of determining whether a test compound is an inhibitor of binding between a PDZ protein and a PL protein by contacting a PDZ domain polypeptide having a sequence from the PDZ protein, and a PL peptide under conditions in which they form a complex, in the presence and in the absence of a test compound, and detecting the formation of the complex in the presence and absence of the test compound, where less complex formation in the presence of the test compound than in the absence of the compound indicates that the test compound is an inhibitor of a PDZ protein -PL protein binding. The PL peptide has a sequence that includes the a C-terminal sequence of a PL protein, such as CD6, CD49E, CD49F, CD138, Clasp-1, Clasp-4, VCAM1, Clasp-2, CD95, DNAM-1, CD83, CD44, CD97, CD3n, DOCK2, CD34, FceRIb, or FasLigand. In some embodiments, the PDZ domain polypeptide is a fusion polypeptide.

In a related aspect, the disclosure provides a method of determining whether a test compound is an agonist of binding between a PDZ protein and a PL protein by contacting a PDZ domain polypeptide, and a PL peptide under conditions in which they form a complex, in the presence and in the absence of a test compound, and detecting the formation of the complex in the presence and absence of the test compound, where more complex formation in the presence of the test compound than in the absence of the compound indicates that the test compound is an agonist of a PDZ protein -PL protein binding.

The disclosure further provides an inhibitor of binding of a PDZ protein and a PL protein. In an aspect, the inhibitor is characterized in that it reduces binding of a peptide selected from the group consisting of a PL peptide selected from the group consisting of CD6, CD49E, CD49F, CD138, Clasp- 1, Clasp- 4, VCAM1, Clasp- 2, CD95, DNAM- 1, CD83, CD44, CD97, CD3n, DOCK2, CD34, FceRIb, and FasLigand and a PDZ domain polypeptide. In various aspects, the inhibitor is a peptide comprising a sequence that is from 3 to about 20 residues of a C- terminal sequence of a PL protein selected from CD6, CD49E, CD49F, CD138, Clasp- 1, Clasp- 4, CAM1, Clasp- 2, CD95, DNAM- 1, CD83, CD44, CD97, CD3n, DOCK2, CD34, FceRIb, and FasLigand; a peptide having a motif X- S- X- A, X- A- D/E- V, X- V/I/L- X*- V, or X- S/T- X- F, (where X is any amino acid and X* is any non- aromatic amino acid) ; a peptide mimetic, ; or a small organic molecule. The disclosure also provides a pharmaceutical composition containing the inhibitor.

The disclosure also provides a method for treating a disease characterized by leukocyte activation by administering a therapeutically effective amount of an inhibitor of a PL-PDZ interaction. In aspects, the disease is characterized by an inflammatory or humoral immune response or is an autoimmune disease. The disclosure further provides a method of reducing inflammation in a subject, by administering an agent that inhibits binding of a PDZ protein and a PL protein, where the PL protein is an adhesion protein, an adaptor protein, or an intracellular protein.

The disclosure also provides use of an inhibitor of the binding of a PDZ protein and a PL protein to inhibit leukocyte activation or to treat a disease mediated by hematopoietic cells, such as a disease is characterized by an inflammatory or humoral immune response. The disclosure also provides use of an inhibitor of the binding of a PDZ protein and a PL protein in the preparation of a medicament for treatment of a disease mediated by hematopoietic cells.

The disclosure also provides a method of modulating a biological function of a hematopoietic cell, comprising introducing into the cell an antagonist that inhibits binding of a PDZ protein and a PL protein in the cell as deduced from Table 2, for example, where the PL protein is DNAM-1 and the PDZ protein is MPP1, MPP2, DLG1, NeDLG, PSD95, LIM, AF6, 41.8 or RGS12, the PL protein is LPAP and the PDZ protein is DLG1 or MINT1, or the PL protein is DNAM-1 and the PDZ protein is PSD95 or MPP2.

The present disclosure also relates to peptides and peptide analogues that bind PDZ domains in hematopoietic cells. In particular, it relates to fusion peptides and peptide analogues containing a hematopoietic cell surface receptor carboxyl terminal sequence and a transmembrane transporter sequence which facilitates entry of the peptides into a target cell. The disclosure also relates to methods of using such compositions in inhibiting leukocyte activation as measured by cytokine production, cell proliferation, apoptosis and cytotoxicity.

It is an aspect of the disclosure to administer a therapeutically effective amount of the aforementioned fusion peptides or peptide analogues as pharmaceutical compositions to a subject to inhibit undesirable leukocyte-mediated events.

It is also an aspect of the disclosure to administer a therapeutically effective amount of the aforementioned fusion peptides or peptide analogues as pharmaceutical compositions to a subject to treat an autoimmune disorder or to prevent transplantation rejection of a solid organ transplant.

## 4. **BRIEF DESCRIPTION OF THE DRAWINGS**

FIGURES 1A- 1D show the results of exemplary assays in which the binding of biotinylated peptides having a sequence of the carboxyl- terminus ("c- terminus") of various leukocyte proteins to PDZ domains (i.e., GST- PDZ domain fusion proteins) was determined using the "G" assay described *infra.* The PDZ domains are: PSD95 (Fig. 1A) ; NeDLG (Fig. 1B) ; DLG1 (Fig 1C) ; and 41.8 (Fig. 1D) . These and other PDZ domain fusion proteins are described *infra* (e.g., TABLE 2) . In the figure, peptides 1- 31 refer to the biotinylated PL, peptide used in the assay, and are identified in the Key, *infra.* "Peptide IDs" are defined in TABLE 3. Key:

| # | Test Protein | Peptide IDs |
|---|---|---|
| 1 | Clasp-2 | AA2L |
| 2 | FceRIb | AA25L |
| 3 | CDW128B | AA29.2 |
| 4 | KV1.3 | AA33L |
| 5 | Neurexin | AA38L |
| 6 | DOCK2 | AA40L |
| 7 | CC CKR-1R | AA41L |
| 8 | CC CKR-2 | AA42L |
| 9 | CC CKR-4 | AA44L |
| 10 | BLR-1 | AA45L |
| 11 | CD49E | AA11L |
| 12 | CD97 | AA14L |
| 13 | VCAM1 | AA17L |
| 14 | CD138 | AA18L |
| 15 | DNAM-1 | AA22L |
| 16 | CDW128A | AA29.1L |
| 17 | CC CKR-3 | AA43L |
| 18 | Clasp-1 | AA1L-R |
| 19 | CD46 (Form 1) | AA10L |
| 20 | CD95 | AA13L |
| 21 | CDW125 | AA28L |
| 22 | CD83 | AA47L |
| 23 | CD62E | AA48L |
| 24 | CD3n | AA4L |
| 25 | Clasp-4 | AA3L-V |
| 26 | CD44 | AA9L |
| 27 | CD166 | AA20L |
| 28 | CD62E | AA48L |
| 29 | CD5 | AA49L |
| 30 | CD148 | AA55L |
| 31 | DOCK2 | AA40L |

**FIGURES 2A and 2B** show the Apparent Affinity Determination for PDZ-Ligand Interactions. Varying concentrations of biotinylated CLASP-2 (Fig. 2A; **TABLE 4**) or Fas (Fig. 2B; **TABLE 4**) C-terminal peptides were reacted with immobilized (plate bound) GST polypeptide or GST-PDZ fusion proteins (GST-DLG1, GST-NeDLG, and GST-PSD95). The binding to GST alone (< 0.2 OD units) was subtracted from the binding to the fusion proteins to obtain the signal at each peptide concentration. This signal was then normalized by dividing the signal at each peptide concentration by the maximum signal observed for each peptide-PDZ pair (i.e. the signal obtained at 30 uM Clasp 2 peptide or 100 uM Fas peptide; 0.4 -1.0 OD units for Clasp 2 and 1.2 - 2.0 OD units for Fas). The normalized signals were then plotted and fit to a saturation binding curve, yielding an apparent affinity of 21 uM for DLG1-Clasp 2 interaction, 7.5 uM for NeDLG-Clasp 2 interaction, 45 uM for PSD95-Clasp 2 interaction, 54 uM for DLG1-Fas interaction, 54 uM for NeDLG-Fas interaction, and 85 uM for PSD95-Fas interaction. Data are means of duplicate data points, with standard errors between duplicate data points < 20%.

**FIGURES 3A-3F** show inhibition of PDZ - PL peptide interactions. A fixed concentration of biotinylated C-terminal peptide having a sequence based on the C-terminal sequence of a cell surface receptor protein (Clasp 2, CD46, Fas, and KV 1.3; see **TABLE 4**) was bound to immobilized GST polypeptide or the GST-fusion protein indicated at the top left of each frame, in the presence or absence of the competitor peptides indicated in the legend of each frame and the level of inhibition determined. Fig. 3A- DLG1; Fig. 3B- PSD95; Fig. 3C NeDLG; Fig. 3D - DLG1, Fig. 3E, PSD95; Fig. F - 41.8. In Fig. 3A-B the competitor peptides are present at 100 uM; in Figs. 3C-F the competitor is present at the indicated concentration.

**FIGURES 4A and 4B** shows the results of introduction of a Tat-CD3 fusion peptide on T cell activation. Antigen-specific T cell activation was measured by cytokine production. Fusion peptides containing tat and a T cell surface molecule carboxyl terminus inhibited $\gamma$-interferon (IFN) production by a T cell line in response to myelin basic protein (MBP) stimulation. The level of inhibition was determined by first subtracting the binding of the labeled peptide to GST alone from the binding to the fusion protein and dividing by the signal in the absence of competitor peptide.

<u>**Tables**</u>

| | |
|---|---|
| Table 1 | Amino Acid Classification |
| Table 2 | Protein-Ligand Pairs |
| Table 3 | PDZ Domains |
| Table 3A | Note on Table 3 |
| Table 4 | PL Peptides |
| Table 5A&B | Exemplary PL Motifs |

5. **DEFINITIONS**

5.1 A "fusion protein" or "fusion polypeptide" as used herein refers to a composite protein, i.e., a single contiguous amino acid sequence, made up of two (or more) distinct, heterologous polypeptides which are not normally fused together in a single amino acid sequence. Thus, a fusion protein can include a single amino acid sequence that contains two entirely distinct amino acid sequences or two similar or identical polypeptide sequences, provided that these sequences are not normally found together in the same configuration in a single amino acid sequence found in nature. Fusion proteins can generally be prepared using either recombinant nucleic acid methods, i.e., as a result of transcription and translation of a recombinant gene fusion product, which fusion comprises a segment encoding a polypeptide of the invention and a segment encoding a heterologous protein, or by chemical synthesis methods well known in the art.

5.2 A "fusion protein construct" as used herein is a polynucleotide encoding a fusion protein.

5.3 As used herein, the term "PDZ domain" refers to protein sequence (i.e., modular protein domain) of approximately 90 amino acids, characterized by homology to the brain synaptic protein PSD-95, the Drosophila septate junction protein Discs-Large (DLG), and the epithelial tight junction protein ZO1 (ZO1). PDZ domains are also known as Discs-Large homology repeats ("DHRs") and GLGF repeats). PDZ domains generally appear to maintain a core consensus sequence (Doyle, D. A., 1996, Cell 85: 1067-1076).

PDZ domains are found in diverse membrane-associated proteins including members of the MAGUK family of guanylate kinase homologs, several protein phosphatases and kinases, neuronal nitric oxide synthase, and several dystrophin-associated proteins, collectively known as syntrophins.

Exemplary PDZ domain-containing proteins and PDZ domain sequences are shown in TABLE 3. The term "PDZ domain" also encompasses variants (e.g., naturally occuring variants) of the sequences of TABLE 3 (e.g., polymorphic variants, variants with conservative substitutions, and the like). Typically, PDZ domains are substantially identical to those shown in TABLE 3, e.g., at least about 70%, at least about 80%, or at least about 90% amino acid residue identity when compared and aligned for maximum correspondence.

5.4 As used herein, the term "PDZ protein" refers to a naturally occurring protein containing a PDZ domain, e.g., a human protein. Exemplary PDZ proteins include CASK, MPP1, DLG1, PSD95, NeDLG, TAX33, SYN1a, TAX43, LDP, LIM, LIMK1, LIMK2, MPP2, NOS1, AF6, PTN-4, prIL16, 41.8kD, KIAA0559, RGS12, KIAA0316, DVL1, TAX40, TIAM1, MINT1, KIAA0303, CBP, MINT3, TAX2, KIAA0561. Exemplary PDZ proteins are listed in TABLE 2 and TABLE 3.

5.5 As used herein, the term "PDZ-domain polypeptide" refers to a polypeptide containing a PDZ domain, such as a fusion protein including a PDZ domain sequence, a naturally occurring PDZ protein, or an isolated PDZ domain peptide.

5.6 As used herein, the term "PL protein" or "PDZ Ligand protein" refers to a naturally occurring protein that forms a molecular complex with a PDZ-domain, or to a protein whose carboxy-terminus, when expressed separately from the full length protein (e.g., as a peptide fragment of 4-25 residues, e.g., 16 residues), forms such a molecular complex. The molecular complex can be observed *in vitro* using the "A assay" or "G assay" described *infra,* or *in vivo.* Exemplary PL proteins listed in **TABLE 2** are demonstrated to bind specific PDZ proteins. This definition is not intended to include anti-PDZ antibodies and the like.

5.7 As used herein, a "PL sequence" refers to the amino acid sequence of the C-terminus of a PL protein (e.g., the C-terminal 2, 3, 4, 5, 6, 7, 8, 9, 10, 12, 14, 16, 20 or 25 residues) ("C-terminal PL sequence") or to an internal sequence known to bind a PDZ domain ("internal PL sequence")..

5.8 As used herein, a "PL peptide" is a peptide of having a sequence from, or based on, the sequence of the C-terminus of a PL protein. Exemplary PL peptides (biotinylated) are listed in **TABLE 4.**

5.9 As used herein, a "PL fusion protein" is a fusion protein that has a PL sequence as one domain, typically as the C-terminal domain of the fusion protein. An exemplary PL fusion protein is a tat-PL sequence fusion.

5.10 As used herein, the term "PL inhibitor peptide sequence" refers to PL peptide an amino acid sequence that (in the form of a peptide or PL fusion protein) inhibits the interaction between a PDZ domain polypeptide and a PL peptide (e.g., in an A assay or a G assay).

5.11 As used herein, a "PDZ-domain encoding sequence" means a segment of a polynucleotide encoding a PDZ domain. In various embodiments, the polynucleotide is DNA, RNA, single stranded or double stranded.

5.12 As used herein, the terms "antagonist" and "inhibitor," when used in the context of modulating a binding interaction (such as the binding of a PDZ domain sequence to a PL sequence), are used interchangeably and refer to an agent that reduces the binding of the, e.g., PL sequence (e.g., PL peptide) and the, e.g., PDZ domain sequence (e.g., PDZ protein, PDZ domain peptide).

5.13 As used herein, the terms "agonist" and "enhancer," when used in the context of modulating a binding interaction (such as the binding of a PDZ domain sequence to a PL sequence), are used interchangeably and refer to an agent that increases the binding of the, e.g., PL sequence (e.g., PL peptide) and the, e.g., PDZ domain sequence (e.g., PDZ protein, PDZ domain peptide).

5.14 As used herein, the terms "peptide mimetic, " "peptidomimetic," and "peptide analog" are used interchangeably and refer to a synthetic chemical compound which has substantially the same structural and/or functional characteristics of an PL inhibitory or PL binding peptide of the invention. The mimetic can be either entirely composed of synthetic, non-natural analogues of amino acids, or, is a chimeric molecule of partly natural peptide amino acids and partly non-natural analogs of amino acids. The mimetic can also incorporate any amount of natural amino acid conservative substitutions as long as such substitutions also do not substantially alter the mimetic's structure and/or inhibitory or binding activity. As with polypeptides of the invention which are conservative variants, routine experimentation will determine whether a mimetic is within the scope of the invention, i.e., that its structure and/or function is not substantially altered. Thus, a mimetic composition is within the scope of the invention if it is capable of binding to a PDZ domain and/or inhibiting a PL-PDZ interaction.

Polypeptide mimetic compositions can contain any combination of nonnatural structural components, which are typically from three structural groups: a) residue linkage groups other than the natural amide bond ("peptide bond") linkages; b) non-natural residues in place of naturally occurring amino acid residues; or c) residues which induce secondary structural mimicry, i.e., to induce or stabilize a secondary structure, e.g., a beta turn, gamma turn, beta sheet, alpha helix conformation, and the like.

A polypeptide can be characterized as a mimetic when all or some of its residues are joined by chemical means other than natural peptide bonds. Individual peptidomimetic residues can be joined by peptide bonds, other chemical bonds or coupling means, such as, e.g., glutaraldehyde, N- hydroxysuccinimide esters, bifunctional maleimides, N, N=- dicyclohexylcarbodiimide (DCC) or N, N=- diisopropylcarbodiimide (DIC) . Linking groups that can be an alternative to the traditional amide bond ("peptide bond") linkages include, e.g., ketomethylene (e.g., -C (=O)- $CH_2$- for- C (=O)- NH- ) , aminomethylene ($CH_2$- NH) , ethylene, olefin (CH=CH) , ether ($CH_2$- O) , thioether ($CH_2$- S) , tetrazole ($CN_4$- ) , thiazole, retroamide, thioamide, or ester (see, e.g., Spatola (1983) in Chemistry and Biochemistry of Amino Acids, Peptides and Proteins, Vol. 7, pp 267- 357, A Peptide Backbone Modifications, Marcell Dekker, NY) .

A polypeptide can also be characterized as a mimetic by containing all or some non-natural residues in place of naturally occurring amino acid residues. Nonnatural residues are well described in the scientific and patent literature; a few exemplary nonnatural compositions useful as mimetics of natural amino acid residues and guidelines are described below.

Mimetics of aromatic amino acids can be generated by replacing by, e.g., D- or L- naphylalanine; D- or L- phenylglycine; D- or L- 2 thieneylalanine; D- or L- 1, -2, 3-, or 4- pyreneylalanine; D- or L- 3 thieneylalanine; D- or L- (2- pyridinyl)- alanine; D- or L- (3- pyridinyl)- alanine; D- or L- (2- pyrazinyl)- alanine; D- or L- (4- isopropyl)- phenylglycine; D- (trifluoromethyl)- phenylglycine; D- (trifluoromethyl)- phenylalanine; D- p- fluorophenylalanine; D- or L- p- biphenylphenylalanine; K- or L- p- methoxybiphenylphenylalanine; D- or L- 2- indole (alkyl) alanines; and, D- or L- alkylainines, where alkyl can be substituted or unsubstituted methyl, ethyl, propyl, hexyl, butyl, pentyl, isopropyl, iso- butyl, sec- isotyl, iso- pentyl, or a non- acidic amino acids. Aromatic rings of a nonnatural amino acid include, e.g., thiazolyl, thiophenyl, pyrazolyl, benzimidazolyl, naphthyl, furanyl, pyrrolyl, and pyridyl aromatic rings.

Mimetics of acidic amino acids can be generated by substitution by, e.g., non- carboxylate amino acids while maintaining a negative charge; (phosphono) alanine; sulfated threonine. Carboxyl side groups (e.g., aspartyl or glutamyl) can also be selectively modified by reaction with carbodiimides (R=- N- C- N- R=) such as, e.g., 1- cyclohexyl- 3 (2- morpholinyl- (4- ethyl) carbodiimide or 1- ethyl- 3 (4- azonia- 4, 4- dimetholpentyl) carbodiimide. Aspartyl or glutamyl can also be converted to asparaginyl and glutaminyl residues by reaction with ammonium ions.

Mimetics of basic amino acids can be generated by substitution with, e.g., (in addition to lysine and arginine) the amino acids ornithine, citrulline, or (guanidino)- acetic acid, or (guanidino) alkyl- acetic acid, where alkyl is defined above. Nitrile derivative (e.g., containing the CN- moiety in place of COOH) can be substituted for asparagine or glutamine. Asparaginyl and glutaminyl residues can be deaminated to the corresponding aspartyl or glutamyl residues.

Arginine residue mimetics can be generated by reacting arginyl with, e.g., one or more conventional reagents, including, e.g., phenylglyoxal, 2, 3- butanedione, 1, 2- cyclohexanedione, or ninhydrin, preferably under alkaline conditions.

Tyrosine residue mimetics can be generated by reacting tyrosyl with, e.g., aromatic diazonium compounds or tetranitromethane. N-acetylimidizol and tetranitromethane can be used to form O-acetyl tyrosyl species and 3-nitro derivatives, respectively.

Cysteine residue mimetics can be generated by reacting cysteinyl residues with, e.g., alpha- haloacetates such as 2- chloroacetic acid or chloroacetamide and corresponding amines; to give carboxymethyl or carboxyamidomethyl derivatives. Cysteine residue mimetics can also be generated by reacting cysteinyl residues with, e.g., bromotrifluoroacetone, alphabromo- beta- (5- imidozoyl) propionic acid; chloroacetyl phosphate, N- alkylmaleimides, 3- nitro- 2- pyridyl disulfide; methyl 2- pyridyl disulfide; p- chloromercuribenzoate; 2- chloromercuri- 4 nitrophenol; or, chloro- 7- nitrobenzo- oxa- 1, 3- diazole.

Lysine mimetics can be generated (and amino terminal residues can be altered) by reacting lysinyl with, e.g., succinic or other carboxylic acid anhydrides. Lysine and other alpha-amino-containing residue mimetics can also be generated by reaction with imidoesters, such as methyl picolinimidate, pyridoxal phosphate, pyridoxal, chloroborohydride, trinitrobenzenesulfonic acid, O-methylisourea, 2,4, pentanedione, and transamidase-catalyzed reactions with glyoxylate.

Mimetics of methionine can be generated by reaction with, e.g., methionine sulfoxide. Mimetics of proline include, e.g., pipecolic acid, thiazolidine carboxylic acid, 3- or 4- hydroxy proline, dehydroproline, 3- or 4- methylproline, or 3, 3, -dimethylproline. Histidine residue mimetics can be generated by reacting histidyl with, e.g., diethylprocarbonate or parabromophenacyl bromide.

Other mimetics include, e.g., those generated by hydroxylation of proline and lysine; phosphorylation of the hydroxyl groups of seryl or threonyl residues; methylation of the alpha-amino groups of lysine, arginine and histidine; acetylation of the N-terminal amine; methylation of main chain amide residues or substitution with N-methyl amino acids; or amidation of C-terminal carboxyl groups.

A component of a natural polypeptide (e.g., a PL polypeptide or PDZ polypetide) can also be replaced by an amino acid (or peptidomimetic residue) of the opposite chirality. Thus, any amino acid naturally occurring in the L-configuration (which can also be referred to as the R or S, depending upon the structure of the chemical entity) can be replaced with the amino acid of the same chemical structural type or a peptidomimetic, but of the opposite chirality, generally referred to as the D- amino acid, but which can additionally be referred to as the R- or S- form.

The mimetics of the disclosure can also include compositions that contain a structural mimetic residue, particularly a residue that induces or mimics secondary structures, such as a beta turn, beta sheet, alpha helix structures, gamma turns, and the like. For example, substitution of natural amino acid residues with D-amino acids; N-alpha-methyl amino acids; C-alpha-methyl amino acids; or dehydroamino acids within a peptide can induce or stabilize beta turns, gamma turns, beta sheets or alpha helix conformations. Beta turn mimetic structures have been described, e.g., by Nagai (1985) Tet. Lett. 26:647-650; Feigl (1986) J. Amer. Chem. Soc. 108:181-182; Kahn (1988) J. Amer. Chem. Soc. 110:1638-1639; Kemp (1988) Tet. Lett. 29:5057-5060; Kahn (1988) J. Molec. Recognition 1:75-79. Beta sheet mimetic structures have been described, e.g., by Smith (1992) J. Amer. Chem. Soc. 114:10672-10674. For example, a type VI beta turn induced by a cis amide surrogate, 1,5-disubstituted tetrazol, is described by Beusen (1995) Biopolymers 36: 181-200. Incorporation of achiral omega-amino acid residues to generate polymethylene units as a substitution for amide bonds is described by Banerjee (1996) Biopolymers 39:769-777. Secondary structures of polypeptides can be analyzed by, e.g., high-field 1H NMR or 2D NMR spectroscopy, see, e.g., Higgins (1997) J. Pept. Res. 50:421-435. See also, Hruby (1997) Biopolymers 43:219-266, Balaji, et al., U.S. Pat. No. 5,612,895.

5.15 As used herein, "peptide variants" and "conservative amino acid substitutions" refer to peptides that differ from a reference peptide (e.g., a peptide having the sequence of the c arboxy-terminus of a specified PL protein) by substitution of an amino acid residue having similar properties (based on size, polarity, hydrophobicity, and the like). Thus, insofar as the compounds that are encompassed within the scope of the invention are partially defined in terms of amino acid residues of designated classes, the amino acids may be generally categorized into three main classes: hydrophilic amino acids, hydrophobic amino acids and cysteine-like amino acids, depending primarily on the characteristics of the amino acid side chain. These main classes may be further divided into subclasses. Hydrophilic amino acids include amino acids having acidic, basic or polar side chains and hydrophobic amino acids include amino acids having aromatic or apolar side chains. Apolar amino acids may be further subdivided to include, among others, aliphatic amino acids. The definitions of the classes of amino acids as used herein are as follows:

"Hydrophobic Amino Acid" refers to an amino acid having a side chain that is uncharged at physiological pH and that is repelled by aqueous solution. Examples of genetically encoded hydrophobic amino acids include Ile, Leu and Val. Examples of non-genetically encoded hydrophobic amino acids include t-BuA.

"Aromatic Amino Acid" refers to a hydrophobic amino acid having a side chain containing at least one ring having a conjugated $\pi$- electron system (aromatic group) . The aromatic group may be further substituted with groups such as alkyl, alkenyl, alkynyl, hydroxyl, sulfanyl, nitro and amino groups, as well as others. Examples of genetically encoded aromatic amino acids include Phe, Tyr and Trp. Commonly encountered non- genetically encoded aromatic amino acids include phenylglycine, 2- naphthylalanine, $\beta$- 2- thienylalanine, 1, 2, 3, 4- tetrahydroisoquinoline- 3-carboxylic acid, 4- chloro- phenylalanine, 2- fluorophenylalanine, 3- fluorophenylalanine and 4- fluorophenylalanine.

"Apolar Amino Acid" refers to a hydrophobic amino acid having a side chain that is generally uncharged at physiological pH and that is not polar. Examples of genetically encoded apolar amino acids include Gly, Pro and Met. Examples of non-encoded apolar amino acids include Cha.

"Aliphatic Amino Acid" refers to an apolar amino acid having a saturated or unsaturated straight chain, branched or cyclic hydrocarbon side chain. Examples of genetically encoded aliphatic amino acids include Ala, Leu, Val and Ile. Examples of non-encoded aliphatic amino acids include Nle.

"Hydrophilic Amino Acid" refers to an amino acid having a side chain that is attracted by aqueous solution. Examples of genetically encoded hydrophilic amino acids include Ser and Lys. Examples of non-encoded hydrophilic amino acids include Cit and hCys.

"Acidic Amino Acid" refers to a hydrophilic amino acid having a side chain pK value of less than 7. Acidic amino acids typically have negatively charged side chains at physiological pH due to loss of a hydrogen ion. Examples of genetically encoded acidic amino acids include Asp and Glu.

"Basic Amino Acid" refers to a hydrophilic amino acid having a side chain pK value of greater than 7. Basic amino acids typically have positively charged side chains at physiological pH due to association with hydronium ion. Examples of genetically encoded basic amino acids include Arg, Lys and His. Examples of non-genetically encoded basic amino acids include the non-cyclic amino acids ornithine, 2,3-diaminopropionic acid, 2,4-diaminobutyric acid and homoarginine.

"Polar Amino Acid" refers to a hydrophilic amino acid having a side chain that is uncharged at physiological pH, but which has a bond in which the pair of electrons shared in common by two atoms is held more closely by one of the atoms. Examples of genetically encoded polar amino acids include Asx and Glx. Examples of non-genetically encoded polar amino acids include citrulline, N-acetyl lysine and methionine sulfoxide.

"Cysteine-Like Amino Acid" refers to an amino acid having a side chain capable of forming a covalent linkage with a side chain of another amino acid residue, such as a disulfide linkage. Typically, cysteine-like amino acids generally have a side chain containing at least one thiol (SH) group. Examples of genetically encoded cysteine-like amino acids include Cys. Examples of non-genetically encoded cysteine-like amino acids include homocysteine and penicillamine.

As will be appreciated by those having skill in the art, the above classification are not absolute -- several amino acids exhibit more than one characteristic property, and can therefore be included in more than one category. For example, tyrosine has both an aromatic ring and a polar hydroxyl group. Thus, tyrosine has dual properties and can be included in both the aromatic and polar categories. Similarly, in addition to being able to form disulfide linkages, cysteine also has apolar character. Thus, while not strictly classified as a hydrophobic or apolar amino acid, in many instances cysteine can be used to confer hydrophobicity to a peptide.

Certain commonly encountered amino acids which are not genetically encoded of which the peptides and peptide analogues of the invention may be composed include, but are not limited to, β- alanine (b- Ala) and other omega-amino acids such as 3- aminopropionic acid (Dap) , 2, 3- diaminopropionic acid (Dpr) , 4- aminobutyric acid and so forth; α- aminoisobutyric acid (Aib) ; ε- aminohexanoic acid (Aha) ; δ- aminovaleric acid (Ava) ; N- methylglycine or sarcosine (MeGly) ; ornithine (Orn) ; citrulline (Cit) ; t- butylalanine (t- BuA) ; t- butylglycine (t- BuG) ; N- methylisoleucine (Melle) ; phenylglycine (Phg) ; cyclohexylalanine (Cha) ; norleucine (Nle) ; 2- naphthylalanine (2- Nal) ; 4- chlorophenylalanine (Phe (4- Cl) ) ; 2- fluorophenylalanine (Phe (2- F) ) ; 3- fluorophenylalanine (Phe (3- F) ) ; 4- fluorophenylalanine (Phe (4- F) ) ; penicillamine (Pen) ; 1, 2, 3, 4- tetrahydroisoquinoline- 3- carboxylic acid (Tic) ; β- 2- thienylalanine (Thi) ; methionine sulfoxide (MSO) ; homoarginine (hArg) ; N- acetyl lysine (AcLys) ; 2, 3- diaminobutyric acid (Dab) ; 2, 3- diaminobutyric acid (Dbu) ; p- aminophenylalanine (Phe ($pNH_2$) ) ; N- methyl valine (MeVal) ; homocysteine (hCys) and homoserine (hSer) . These amino acids also fall conveniently into the categories defined above.

The classifications of the above-described genetically encoded and non-encoded amino acids are summarized in **TABLE 1**, below. It is to be understood that **TABLE 1** is for illustrative purposes only and does not purport to be an exhaustive list of amino acid residues which may comprise the peptides and peptide analogues described herein. Other amino acid residues which are useful for making the peptides and peptide analogues described herein can be found, e.g., in Fasman, 1989, CRC Practical Handbook of Biochemistry and Molecular Biology, CRC Press, Inc., and the references cited therein. Amino acids not specifically mentioned herein can be conveniently classified into the above-described categories on the basis of known behavior and/or their characteristic chemical and/or physical properties as compared with amino acids specifically identified.

**TABLE 1**

| Classification | Genetically Encoded | Genetically Non-Encoded |
|---|---|---|
| Hydrophobic | | |
| Aromatic | F, Y, W | Phg, Nal, Thi, Tic, Phe(4-Cl), Phe(2-F), Phe(3-F), Phe(4-F), Pyridyl Ala, Benzothienyl Ala |
| Apolar | M, G, P | |
| Aliphatic | A, V, L, I | t-BuA, t-BuG, Melle, Nle, MeVal, Cha, bAla, MeGly, Aib |
| Hydrophilic | | |
| Acidic | D, E | |
| Basic | H, K, R | Dpr, Om, hArg, Phe(p-$NH_2$), DBU, $A_2BU$ |
| Polar | Q, N, S, T, Y | Cit, AcLys, MSO, hSer |
| Cysteine-Like | C | Pen, hCys, p-methyl Cys |

5.16 As used herein, a "detectable label" has the ordinary meaning in the art and refers to an atom (e.g., radionuclide), molecule (e.g., fluorescein), or complex, that is or can be used to detect (e.g., due to a physical or chemical property), indicate the presence of a molecule or to enable binding of another molecule to which it is covalently bound or otherwise associated. The term "label" also refers to covalently bound or otherwise associated molecules (e.g., a biomolecule such as an enzyme) that act on a substrate to produce a detectable atom, molecule or complex. Detectable labels suitable for use in the present invention include any composition detectable by spectroscopic, photochemical, biochemical, immunochemical, electrical, optical or chemical means. Labels useful in the present invention include biotin for staining with labeled streptavidin conjugate, magnetic beads (e.g., DynabeadsTM), fluorescent dyes (e.g., fluorescein, Texas red, rhodamine, green fluorescent protein, enhanced green fluorescent protein, and the like), radiolabels (e.g., 3H, 125I, 35S, 14C, or 32P), enzymes ( e.g., hydrolases, particularly phosphatases such as alkaline phosphatase, esterases and glycosidases, or oxidoreductases, particularly peroxidases such as horse radish peroxidase, and others commonly used in ELISAs), substrates, cofactors, inhibitors, chemiluminescent groups, chromogenic agents, and colorimetric labels such as colloidal gold or colored glass or plastic (e.g., polystyrene, polypropylene, latex, etc.) beads. Patents teaching the use of such labels include U.S. Patent Nos. 3,817,837; 3,850,752; 3,939,350; 3,996,345; 4;277,437; 4,275,149; and 4,366,241. Means of detecting such labels are well known to those of skill in the art. Thus, for example, radiolabels and chemiluminescent labels may be detected using photographic film or scintillation counters, fluorescent markers may be detected using a photodetector to detect emitted light (e.g., as in fluorescence-activated cell sorting). Enzymatic labels are typically detected by providing the enzyme with a substrate and detecting the reaction product produced by the action of the enzyme on the substrate, and colorimetric labels are detected by simply visualizing the colored label. Thus, a label is any composition detectable by spectroscopic, photochemical, biochemical, immunochemical, electrical, optical or chemical means. The label may be coupled directly or indirectly to the desired component of the assay according to methods well known in the art. Non-radioactive labels are often attached by indirect means. Generally, a ligand molecule (e.g., biotin) is covalently bound to the molecule. The ligand then binds to an anti-ligand (e.g., streptavidin) molecule which is either inherently detectable or covalently bound to a signal generating system, such as a detectable enzyme, a fluorescent compound, or a chemiluminescent compound. A number of ligands and anti-ligands can be used. Where a ligand has a natural anti-ligand, for example, biotin, thyroxine, and cortisol, it can be used in conjunction with the labeled, naturally occurring anti-ligands. Alternatively, any haptenic or antigenic compound can be used in combination with an antibody. The molecules can also be conjugated directly to signal generating compounds, e.g., by conjugation with an enzyme or fluorophore. Means of detecting labels are well known to those of skill in the art. Thus, for example, where the label is a radioactive label, means for detection include a scintillation counter, photographic film as in autoradiography, or storage phosphor imaging. Where the label is a fluorescent label, it may be detected by exciting the fluorochrome with the appropriate wavelength of light and detecting the resulting fluorescence. The fluorescence may be detected visually, by means of photographic film, by the use of electronic detectors such as charge coupled devices (CCDs) or photomultipliers and the like. Similarly, enzymatic labels may be detected by providing the appropriate substrates for the enzyme and detecting the resulting reaction product. Also, simple colorimetric labels may be detected by observing the color associated with the label. It will be appreciated that when pairs of fluorophores are used in an assay, it is often preferred that they have distinct emission patterns (wavelengths) so that they can be easily distinguished.

5. 17 As used herein, the term "substantially identical" in the context of comparing amino acid sequences, means that the sequences have at least about 70%, at least about 80%, or at least about 90% amino acid residue identity when compared and aligned for maximum correspondence. An algorithm that is suitable for determining percent sequence identity and sequence similarity is the FASTA algorithm, which is described in Pearson, W.R. & Lipman, D.J., 1988, Proc. Natl. Acad. Sci. U.S.A. 85: 2444. See also W. R. Pearson, 1996, Methods Enzymol. 266: 227-258. Preferred parameters used in a FASTA alignment of DNA sequences to calculate percent identity are optimized, BL50 Matrix 15: -5, k-tuple = 2; joining penalty = 40, optimization = 28; gap penalty -12, gap length penalty =-2; and width = 16.

5.18 As used herein, "hematopoietic cells" include leukocytes including lymphocytes (T cells, B cells and NK cells), monocytes, and granulocytes (i.e., neutrophils, basophils and eosinophils), macrophages, dendritic cells, megakaryocytes, reticulocytes, erythrocytes, and $CD34^+$ stem cells.

## 6. DETAILED DESCRIPTION OF THE INVENTION

The present inventors have discovered that interactions between PDZ proteins and PL proteins play an important and extensive role in the biological function of hematopoietic cells and other cells involved in the immune response. Although PDZ-PL interactions were known in the nervous system (i.e., in neurons), their universal importance in hematopoietic cell function, especially in function of T cells and B cells, and their fundamental role in modulation of the immune response has not been recognized. In particular, the present inventors have surprisingly discovered that cell adhesion molecules that mediate cell-cell interaction in the hematopoietic system are PDZ-binding proteins (PL proteins) and bind to PDZ proteins. The inventors have identified numerous interactions between PDZ proteins and PL proteins present in immune system cells, and the invention provides reagents and methods for affecting biological function in the immune system by inhibiting these interactions. As used herein, the term "biological function" in the context of a cell, refers to a detectable biological activity normally carried out by the cell, e.g., a phenotypic change such as proliferation, cell activation (e.g., T cell activation, B cell activation, T-B cell conjugate formation), cytokine release, degranulation, tyrosine phosphorylation, ion (e.g., calcium) flux, metabolic activity, apoptosis, changes in gene expression, maintenance of cell structure, cell migration, adherance to a substrate, signal transduction, cell-cell interactions, and others described herein or known in the art.

In one aspect, the present invention relates to peptides or peptide analogues as defined in the claims. Mimetics, pharmaceutical compositions, and methods of using such compositions to regulate the biological activities of hematopoietic cells, e.g. T cells and B cells, or other cells (e.g., endothelial cells) that necessary for immune function are also described herein. The disclosure further relates to methods of using the compositions to modulate hematopoietic cell activation and immune function, as well as assays for such inhibitors.

**TABLE 2** summarizes an extensive analysis of protein interactions in T cells and B cells. PDZ proteins, the vast majority of which were not previously known to be expressed in immune system cells, are listed in the top row of **TABLE 2.** The first column of the table lists PL proteins. Positions in the matrix denoted by the letter "A" or "G" indicate that an interaction between the PDZ protein and the PL has been detected in novel binding assays (described in detail in, e.g., Section 6.2, *infra*). A blank cell indicates that no interaction was detected using the assays of the invention. An asterisk (*) denotes a PL-PDZ interaction previously reported in the scientific literature.

# PDZ-LIGAND/PDZ INTERACTION SUMMARY

## TABLE 2

| PDZ LIGAND | CODE | SEQ | CASK | MPP1 | DLG1 | PSD95 | NeDLG | TAX33 | SYN1a | TAX 43 | LDP | LIM | LIMK1 | LIMK2 | MPP2 |
|---|---|---|---|---|---|---|---|---|---|---|---|---|---|---|---|
| CD6 | AA6L | ISAA | | | | | | | | | | | | | |
| CD49E (alpha-4) | AA11L | TSDA | | | | | | | | | | | | | |
| CD49F (Aform. alpha6) | AA12L | TSDA | | | | | | | | | | | | | |
| CD166 (CD6L) | AA20L | KTEA | | | | | | | | | | | | | |
| CD148 | AA55L | KTIA | | | | | | | | | | | | | |
| CC CKR-2 | AA42L | KEGA | | | | | | | | | | | | | |
| CD138 (syndecan) | AA18L | EFYA | * | | | | | | | | | | | | |
| CD148 (DEP-1) | AA19L | GYIA | | | | | | | | | | | | | |
| CD98 (2F4) | AA15L | PYAA | | | | | | | | | | | | | G |
| CLASP-1 | AA1L | SAEV | | | G | A | G | | | | | | | | |
| CLASP-4 | AA3L-V | YAEV | | | A | A | A | | | | A | | | | |
| NMDA | AA34.2L | ESDV | | A | A/G | A/G | A/G | | G | A | | | A | | G |
| VCAM1 | AA17L | KSKV | | A | A | | A | | | | A | | | | |
| CLASP-2 | AA2L | SSVV | | | A/G | A/G | A/G | | | | | | | | |
| CD95 (Apo-1/Fas) | AA13L | QSLV | | | A/G | A/G | A/G | | | | | | | | |
| KV1.3 | AA33L | FTDV | | | A/G* | A/G* | A/G | | | | | | A | | |
| DNAM-1 | AA22L | KTRV | | A | A | A/G | A | | | | | A | | | G |
| CD83 | AA47L | TELV | | | A | A | A | | | | | | | | |
| CD44 (long form) | AA9L | KIGV | | G | | | | | | | | | | | |
| Neurexin | AA38L | EYYV | G* | A* | A/G | A/G | G | | A | A | | | A | | |
| CD97 (CD55L) | AA14L | ESGI | | | A | | | | | | | | | | |
| Glycophorin C | AA37L | EYFI | | * | G | G | G | | | | | | | | A |
| CDW128A (IL8RA) | AA29.1L | SSNL | | | A | | A | | | | | | | | |
| CD3n | AA4L | SSQL | | | A | A | | | | | | | | | |
| LPAP | AA30L | VTAL | | | A | | | | | | | | | | |
| CD46 (form 1) | AA10L | FTSL | | | A/G | A/G | G | | | | | | | | |
| CDW128B (IL8RB) | AA29.2L | STTL | | | A/G | A | A/G | | | | | | | | |
| DOCK2 | AA40L | STDL | | | A | A/G | G | | G | | | | | | |
| CD34 | AA7L | DTEL | | | A | A | G | | | | | | | | |
| CD5 | AA49L | AQRL | | | | | | | | | | | | | |
| CC CKR-4 | AA44L | HDAL | | | | | | | | | | | | | |
| FceRIb | AA25L | PIDL | | | | | | | | | | | | | |
| FasLigand | AA23L-M | LYKL | | | | | | | | | | | | | |
| CD62E | AA48L | SYIL | | | | | | | | | | | | | |
| CC CKR-1R | AA41L | SAGF | | | | | | | | | | | | | |
| CDW125 (IL5R) | AA28L | DSVF | | | | | | | | | | | | | |
| BLR-1 | AA45L | LTTF | | | | | | | | | | | | | |
| CC CKR-3 | AA43L | SIVF | | | | | | | | | | | | | |
| | | | CASK | MPP1 | DLG1 | PSD95 | NeDLG | TX33 | SYN1a | TX 43 | LDP | LIM | LIMK | LIMK2 | MPP2 |

* Interactions described in the scientific literature

## PDZ-LIGAND/PDZ INTERACTION SUMMARY

## TABLE 2 CONTINUED

| NOS1 | AF6 | PTN-4 | prIL16 | 41.8 | K559 | RGS12 | K316 | DVL1 | TAX 40 | TIAM1 | MINT1 | K303 | CBP | MINT3 | TAX 2 | K561 | PDZ LIGAND |
|---|---|---|---|---|---|---|---|---|---|---|---|---|---|---|---|---|---|
| | | | | A | | | | | | | | | | | | | CD6 |
| | | | | A/G | | | | | | | | | | | | | CD49E (alpha-4) |
| | | | | A/G | | | | | | | | | | | | | CD49F (Aform, alpha6) |
| | | | | | | | | | | | | | | | | | CD166 (CD6L) |
| | | | | | | | | | | | | | | | | | CD148 |
| | | | | | | | | | | | | | | | | | CC CKR-2 |
| | | | | A/G | | | | | | A | | | | | | | CD138 (syndecan) |
| | | | | | | | | | | | | | | | | | CD148 (DEP-1) |
| | | | | | | | | | | | | | | | | | CD98 (2F4) |
| | | | | | | | | | | | | | | | | | CLASP-1 |
| | A | | | A | | | | | | | A | | | | | | CLASP-4 |
| | | A/G | | A/G | | A/G | | A | | | A/G | | | | A | G | NMDA |
| | | | | A | | | | | | A | | A | | | | | VCAM1 |
| | | | | A | | | | | | | | | | | | | CLASP-2 |
| | | | | A/G | | | | | | | | | | | | | CD95 (Apo-1/Fas) |
| | | | | A | | A | | A | | | G | | | | | | KV1.3 |
| | A | | | A | | A | | | | | | | | | | | DNAM-1 |
| | | | | | | | | | | | | | | | | | CD83 |
| | | | G | | | | | | | | G | | | | | | CD44 (long form) |
| | A | | A | A | | A | | A | A | A | A/G | | | | | | Neurexin |
| | | | | A | | | | | | | | | | | | | CD97 (CD55L) |
| | A | | | A | | | | | | | A | | | | | | Glycophorin C |
| | | | | | | | | | | | | | | | | | CDW128A (IL8RA) |
| | | | | A/G | | | | | | | A/G | | | | | | CD3n |
| | | | | | | | | | | | G | | | | | | LPAP |
| | | | | | | | | | | | | | | | | | CD46 (form 1) |
| | | | | A | | * | | | | | | | | | | | CDW128B (IL8RB) |
| | | | | | | | | | | | | | | | | G | DOCK2 |
| | | | | | | | | | | | | | | | | | CD34 |
| | | | | | | | | | | | | | | | | | CD5 |
| | | | | | | | | | | | | | | | | | CC CKR-4 |
| | | | | | | | | | | | A | | | | | | FceRIb |
| | | | | | | | | | | | | | | | | G | FasLigand |
| | | | | | | | | | | | | | | | | | CD62E |
| | | | | | | | | | | | | | | | | | CC CKR-1R |
| | | G | | | | G | | | | | | | | | | | CDW125 (IL5R) |
| | | | | | | | | | | | G | | | | | | BLR-1 |
| | | | | | | | | | | | | | | | | | CC CKR-3 |
| NOS1 | AF6 | PTN-4 | prIL16 | 41.8 | K559 | RSG12 | K316 | DVL1 | TX 40 | TIAM1 | MINT1 | K303 | CBP | MINT3 | TX 2 | K561 | |

* Interactions described in the scientific literature

As discussed in detail herein, the PDZ proteins listed in **TABLE 2** are naturally occurring proteins containing a PDZ domain. The present invention is particularly directed to the detection and modulation of interactions between PDZ proteins and PL proteins in hematopoietic cells. Exemplary PL proteins are listed in **TABLE 2**. Notably, as discussed *infra,* many of these PL proteins have not previously been recognized as such in any cell system. A variety of PL protein classes are known, and the PL proteins described herein can be characterized as (1) "PL adhesion proteins" (2) "PL ion channel proteins" (3) "PL adaptor proteins" (4) "PL intracellular proteins" and (5) "PL cytokine receptor proteins."

As used herein, an adhesion protein is a cell surface protein involved in cell-cell interaction by direct contact with cell surface molecules (e.g., transmembrane proteins or surface proteins) on a different cell. Thus, when a cell expressing a PL adhesion protein contacts an appropriate other cell, the PL adhesion protein localizes at the interface of the two cells and directly contacts a cell surface molecule on the second cell. A cell-cell interface is a region where the plasma membranes of two different cells are in close (generally <10 nm, often about 1 nm) apposition. Typically, direct molecular contact means interaction of molecules at distances where Van der Walls forces are significant, generally less than about 1 nm. Exemplary PL adhesion proteins include CD6; CD49E (alpha-4); CD49F (a form, alpha6); CD138 (syndecan); CLASP-1; CLASP-4; VCAM1; CLASP-2; DNAM-1; CD83; CD44 (long form); CD97; (CD55L); CD3n; DOCK2; CD34; and FceRlb. Thus, in one embodiment, the PL proteins of the invention are PL adhesion proteins. In an embodiment, the invention provides methods and reagents, as detailed herein, for inhibiting interactions between PL adhesion proteins and PDZ proteins to modulate an immune response. In an embodiment, the inhibition or modulation occurs in a hematopoietic cell. In a related embodiment, the inhibition or modulation occurs in an endothelial cell. In a related embodiment, the inhibition or modulation occurs in an endothelial cell. In a related embodiment, the inhibition or modulation occurs in an epithelial cells, keratinocytes, hepatocytes, cardiac myocytes.

As used herein, an ion channel protein means a transmembrane protein that itself catalyzes the passage of an ion from aqueous solution on one side of a lipid bilayer membrane to aqueous solution on the other side (e.g., by forming a small pore in the membrane). One exemplary PL ion channel proteins is Kv1.3. Thus, in one embodiment, the PL proteins of the invention are PL ion channel proteins. In an embodiment, the invention provides methods and reagents, as detailed herein, for inhibiting interactions between PL ion channel proteins and PDZ proteins to modulate an immune response. In an embodiment, the inhibition or modulation occurs in a hematopoeitic cell. In a related embodiment, the inhibition or modulation occurs in an endothelial cell.

As used herein, an intercellular (i.e., cytosolic) protein has the normal meaning in the art and refers to a protein that is not membrane bound, e.g., has no transmembrane domain. Thus, in one embodiment, the PL proteins of the invention are PL intercellular proteins. Exemplary PL intercellular proteins include Glycophorin C and LPAP. In an embodiment, the invention provides methods and reagents, as detailed herein, for inhibiting interactions between PL cytoplasmic proteins and PDZ proteins to modulate an immune response. In an embodiment, the inhibition or modulation occurs in a hematopoeitic cell. In a related embodiment, the inhibition or modulation occurs in an endothelial cell.

As used herein a cytokine receptor has the normal meaning in the art and refers to a membrane protein with an extracellular domain that specifically binds a cytokine. Exemplary PL cytokine receptor proteins include CDW 125 (IL5R), CDW128A (IL8RA), and BRL-1. Thus, in one embodiment, the PL proteins of the invention are PL cytokine proteins. In an embodiment, the invention provides methods and reagents, as detailed herein, for inhibiting interactions between PL cytokine proteins and PDZ proteins to modulate an immune response. In an embodiment, the inhibition or modulation occurs in a hematopoeitic cell. In a related embodiment, the inhibition or modulation occurs in an endothelial cell.

As used herein, an adaptor protein means a molecule (e.g., protein) that contributes to the formation of a multimolecular complex by binding two or more other biomolecuics. The binding of the two or more other molecules by the adaptor molecule/protein generally involves direct molecular contact between the adaptor protein and each of the two or more other moiccules.. One exemplary PL adaptor protein is LPAP. Thus, in one embodiment, the PL proteins of the invention are PL adaptor proteins. In an embodiment, the invention provides methods and reagents, as detailed herein, for inhibiting interactions between PL adaptor proteins and PDZ proteins to modulate an immune response. In an embodiment, the inhibition or modulation occurs in a hematopoeitic cell. In a related embodiment, the inhibition or modulation occurs in an endothelial cell.

In various embodiments, the PL proteins of the invention are characterized by specific C-terminal (i.e., PL domain) amino acid sequences or amino acid motifs, as described elsewhere in this disclosure.

In various embodiments of the invention, the PL proteins of the invention bind a PDZ protein expressed in T lymphocytes, B lymphocytes, or both T and B lymphocytes. In an embodiment, the PL protein binds a PDZ protein expressed in endothelial cells. In various embodiments, the PL proteins and/or the PDZ protein to which it binds are not expressed in the nervous system (e.g., neurons).

In various embodiments of the invention, the PL protein of the invention binds only one PDZ protein listed in **TABLE 2**. In other embodiment, the PL protein binds 1 to 3, 3 to 5, or more than 5 different PDZ proteins listed in **TABLE 2**.

In various embodiments of the invention, the PL protein is expressed or up-regulated upon cell activation (e.g., in activated B lymphocytes, T lymphocytes) or upon entry into mitosis (e.g., up-regulation in rapidly proliferating cell populations).

In various embodiments of the invention, the PL protein is (i) a protein that mediates immune cell (e.g., hematopoietic cell) activation or migration, (ii) a protein that does not mediate apoptosis in a cell type (iii) a protein that is other than a G-protein coupled seven transmembrane helix receptor, (iv) a protein that is G-protein coupled seven transmembrane helix receptor but not a cytokine receptor or (v) a protein that is not a G-protein coupled seven transmembrane helix receptor and is a cytokine receptor.

6.1 Detection of PDZ Domain-Containing Proteins Expressed in Hematopoietic Cells

As noted *supra,* the present inventors surprisingly discovered that numerous PDZ proteins are expressed in immune system cells, and play a fundamental biological role in modulation of the immune response. PDZ proteins DLG1 and TIAM-1 have been previously described to be in T cells. The present inventors discovered, using a BLAST search of the Human EST database and the experiments described *infra,* that several additional PDZ proteins are present in hematopoietic cells including MPP1, P-DLG, VELI-1, PSD95, syntenin in T cells and CASK, DLG1, DLG2, ZIP KINASE, syntrophin 2, P-dlg, PSD95, and syntenin in B cells.

To determine the full extent of PDZ proteins' involvement in hematopoietic function, the inventors embarked on a systematic investigation of PDZ proteins in T and B cells. A comprehensive list of PDZ domain-containing proteins was retrieved from the Sanger Centre database (Pfam) searching for the keyword, PDZ. The corresponding cDNA sequences were retrieved from GenBank using the NCBI "entrez" database (hereinafter, "GenBank PDZ protein cDNA sequences"). The DNA portion encoding PDZ domains was identified by alignment of cDNA and protein sequence using CLUSTALW. Based on the DNA/protein alignment information, primers encompassing the PDZ domains were designed. The expression of certain PDZ-containing proteins in immune cells was detected by polymerase chain reaction ("PCR") amplification of cDNAs obtained by reverse transcription ("RT") of immune cell derived RNA (i.e., "RT-PCR"). PCR, RT-PCR and other methods for analysis and manipulation of nucleic acids are well known and are described generally in Sambrook et al., (1989) MOLECULAR CLONING: A LABORATORY MANUAL, 2ND ED., VOLS. 1-3, Cold Spring Harbor Laboratory hereinafter, "Sambrook"); and Ausubel et al., CURRENT PROTOCOLS IN MOLECULAR BIOLOGY, Greene Publishing and Wiley-Interscience, New York (1997), as supplemented through January 1999 (hereinafter "Ausubel").

In the experiments summarized in **TABLE 2**, T-cells (Jurkat E6 cell line) and B-cells (MV 4-11 cell line) were tested for expression of specific PDZ domain containing genes by RT-PCR. RNA was prepared using the "trizol" RNA preparation kit (GIBCO-BRL; Cat. # 15596-018) according to the manufacturer's recommendations. Briefly, 1-5 x 10' lymphoblasts were harvested by centrifugation at 200 x g for 10 minutes at 20 ° C. Cells were resuspended in 100 μl PBS buffer and 1 ml of TRIZOL reagent was added per 5 x $10^6$ cells. The cells resuspension was mixed and after 5 minutes incubation at room temperature (RT), chloroform was added at 0.2 ml per ml TRIZOL. The resuspension was vigorously shaken and incubated for 3 more minutes at RT. Samples were then centrifuged at 12000 x g for 15 minutes at 4 ° C, the aqueous phase was recovered and RNA was precipitated with 2-propanol. The precipitate was collected by centrifugation at 12000 x g for 15 minutes at 4 ° C, washed with 75% ethanol, finally recollected by another spin at 12000 x g for 15 minutes at 4 ° C, air dried and resuspended in an appropriate volume of DEPC treated water.

RNA concentration and purity were determined by the measurement of 260/280 nm light absorption by the nucleic acid. For cDNA synthesis, the SUPERSCRIPT II reverse transcriptase cDNA kit (GIBCO-BRL; Cat. # 18064-014) was used. RNA input per 200 μl cDNA reaction sample was 10 μg. Prior to cDNA synthesis RNA was treated with 1 unit/μl DNAse I in 110 μl water at 37° C for 20 minutes. DNase I was then inactivated by a 10 minutes incubation at 70 ° C. Random primer was used for cDNA priming; 10 μl of random hexamer primer (100 ng/μl) was added, samples were heated to 70 ° C for 5 minutes and chilled on ice. Subsequently 40 μl SUPERSCRIPT II "first strand" buffer, 20 μl of 0.1 M DDT, 10 μl of a 10 mM of mix of deoxynucleotide triphosphates (dATP, dCTP, dGTP, dUTP) and 10 μl of SUPERSCRIPT II reverse transcriptase were added and cDNA synthesis was done for 45 minutes at 42 ° C. Reavtions were stopped by a 5 minutes incubation at 95 ° C and typically, 2-4 μl of such cDNA samples were used for PCR.

A portion of the cDNA (typically, 1/5 of a 20 μl reaction) was used for PCR. PCR was conducted using primers designed to amplify specifically PDZ domain-containing regions of PDZ proteins of interest. Oligonucleotide primers were designed to amplify one or more PDZ-encoding domains. The DNA sequences encoding the various PDZ domains of interest were identified by inspection (i.e., conceptual translation of the PDZ protein cDNA sequences obtained from GenBank, followed by alignment with the PDZ domain amino acid sequence). **TABLE 3** shows the PCR primers, the PDZ-encoded domains amplified, and the GenBank accession number of the PDZ-domain containing proteins. To facilitate subsequent cloning of PDZ domains, the PCR primers included endonuclease restriction sequences at their ends to allow ligation with pGEX-3X cloning vector (Pharmacia, GenBank XXI13852) in frame with glutathione-S transferase (GST).

**TABLE 3** lists proteins detected in the aforementioned assays. The results showed that PDZ proteins are widely utilized in T and B cells in both lineage specific as well as lineage independent manner. INADL2/3 (PDZ dom.), KIAA0316, and 26s subunit p27 were detected in T cells, but not B cells. mCASK, KIAA0559, PTN-4, and X11 beta were detected in B cells, but not T cells. AF6, BAII associated prot., Cytohesin bind. Prot., DLG1, DLG5 (pdlg), DVL1, DVL3, GTPase, hypoth. 41.8 kd, KIAA147, KIAA0300, KIAA0303, KIAA0380, KIAA0440, KIAA0545, KIAA0561, LIMK1, LIMK2, LIM domain prot, LIM protein, MINT1, MINT3, MPP1, MPP2, NE-DLG, NOS1, novel serine protease, PTN-3, prIL 16, PSD95, RGS12, serine protease, SYNTENIN, SYNTR 1 alpha, TAX1, TAX2, TAX33, TAX40, Tax43 (SYN, Beta1), TIAM wwp3, and X11 prot. were detected in both T cells and B cells.

TABLE3 PDZ DOMAINS

Key:
Gene names and corresponding gene products are provided. In some cases, cDNA sequences representing the same gene have several database entries under different accession numbers and names. Accession numbers shown correspond to the gene name used in this description, and numbering of nucleotides and amino acids correlates to those Genbank entries. Amino acid sequences shown correspond to the cloned DNA portions of PDZ domain containing genes. Linker amino acid sequences (e.g., amino acids encoded by DNA flanking the cloning site of the pGEX-3X cloning vector). are in italics

| GENE SYMBOL | PROTEIN | ACC.# | AMINO ACID SEQUENCE* | CLON. SITES | FORWARD PRIMER | REVERSE PRIMER |
|---|---|---|---|---|---|---|
| CASK | CASK | Y17138 | AA495-584; PDZ domain 1 (of 1)<br>*HV*TRVRLVQFQKNTDEPMGITLK MNELNHCIVARIMHGGMIHRQGT LHVGDEIREINGISVANQTVEQL QKMLREMRGSITFKIVPSYRTQS *LNSS* | Bam HI / Eco RI | 6CAF<br>5'- TCGGATCCAT GTGACCAGAG TTCGG-3'<br>N1471-1494 | 7CAR<br>5'- TCGGAATTCAG ACTGAGTGCGG TA-3'<br>N1761-1738 |
| MPP1 | 55 Kd erythrocyte membrane protein | M64925 | AA101-186; PDZ domain 1 (of 1)<br>RKVRLIQFEKVTEEPMGITLKLN EKQSCTVARILHGGMIHRQGSLH VGDEILEINGTNVTNHSVDQLQK AMKETKGMISLKVIPNQ*REFIVT D* | Bam HI / Bam HI | 62MPF<br>5'- GGGATCCGGA AAGTGCGACT CATAC-3'<br>N296-320 | 63MPR<br>5'- ACGGATCCGCT GGTTGGGAATT ACTT-3'<br>N568-543 |
| | | | | | | |

(continued)

| GENE SYMBOL | PROTEIN | ACC.# | AMINO ACID SEQUENCE* | CLON. SITES | FORWARD PRIMER | REVERSE PRIMER |
|---|---|---|---|---|---|---|
| DLG1 | human homolog of Drosophila discs large protein | U13897 | AA275-477; PDZ domains 1-2 (of 3)<br><br>*Q*VNGTDADYEYEEITLERGNSGL GFSIAGGTDNPHIGDDSSIFITK IITGGAAAQDGRLRVNDCILRVN EVDVRDVTHSKAVEALKEAGSIV RLYVKRRKPVSEKIMEIKLIKGP KGLGFSIAGGVGNQHIPGDNSIY VTKIIEGGAAHKDGKLQIGDKLL AVNNVCLEEVTHEEAVTALKNTS DFVYLKVAKPTSMYMNDGYA*PNS S* | Bam HI / Eco RI | 1DF<br><br>5′-TCGGATCCAGGTTAATGGCTCAGATG-3′<br>N815-841 | 2DR<br><br>5′-CGGAATTCGGTGCATAGCCATC-3′<br>N1442-1421 |

(continued)

| GENE SYMBOL | PROTEIN | ACC.# | AMINO ACID SEQUENCE* | CLON. SITES | FORWARD PRIMER | REVERSE PRIMER |
|---|---|---|---|---|---|---|
| PSD95 | human postsynaptic density protein 95 | U83192 | AA387-724;<br><br>PDZ domains 1-3 (of 3)<br>*LEG*EGEMEYEEITLERGNSGLGF SIAGGTDNPHIGDDPSIFITKII PGGAAAQDGRLRVNDSILFVNEV DVREVTHSAAVEALKEAGSIVRL YVMRRKPPAEKVMEIKLIKGPKG LGFSIAGGVGNQHIPGDNSIYVT KIIEGGAAHKDGRLQIGDKILAV NSVGLEDVMHEDAVAALKNTYDV VYLKVAKPSNAYLSDSYAPPDIT TSYSQHLDNEISHSSYLGTDYPT AMTPTSPRRYSPVAKDLLGEEDI PREPRRIVIHRGSTGLGFNIVGG EDGEGIFISFILAGGPADLSGEL RKGDQILSVNGVDLRNASHEQAA IALKNAGQTVTIIAQYKPE*FIV* | Bam HI / Eco RI | 8PSF<br><br>5'-TCGGATCCTTGAGGGGGAGATGGA-3'<br>N1150-1173 | 11PSR<br><br>5'-TCGGAATTCGCTATACTCTTCTGG-3'<br>N2191-2168 |

(continued)

| GENE SYMBOL | PROTEIN | ACC.# | AMINO ACID SEQUENCE* | CLON. SITES | FORWARD PRIMER | REVERSE PRIMER |
|---|---|---|---|---|---|---|
| NeDLG | presynaptic protein sao102 (neuroendocrine-dlg) | U49089 | AA205-1171; PDZ domains 1-2 (of 3)<br>*Q*YEEIVLERGNSGLGFSIAGGIDNPHVPDDPGIFITKIIPGGAAAMDGRLEVNDCVLRVNEDEVSEVVHSRAVEALKEAGPVMRLVVRRRQPPPETIMEVNLLKGPKGLGFSIAGGIGNQHIPGDNSIYITKIIEGGAAQKDGRLQIGDRLLAVNNTNLQDVRHEEAVASLKNTSDMVYLKVAKPGS*PR* | Bam HI / Eco RI | 71NEDF<br>5'-CAGGATCCAATATGAGGAAATCGTACTTG-3'<br>N608-635 | 72NEDR<br>5'-TTGAATTCGAGGCTGCCTGGCTTGGC-3'<br>N1186-1161 |
| | | | | | | |
| TAX33 | tax interaction protein 33 | AF028826 | AA73-162; PDZ domain 1 (of 1)<br>HSHPRVVELPKTDEGLGFNVMGGKEQNSPIYISRIIPGGVAERHGGLKRGDQLLSVNGVSVEGEHHEKAVELLKAAKDSVKLVVRYTPKVL*EFIVTN* | Bam HI / Eco RI | 92TAF<br>5'-GTGGGATCCACTCCCACCCTCGAGTAG-3'<br>N208-234 | 93TAR<br>5'-CATGAATTCCAGAACTTTTGGGTGTATCGC-3'<br>N497-468 |
| SYN 1 α | alpha1-syntrophin | U40571 | AA96-189 PDZ domain 1 (of 1)<br>QRRRVTVRKADAGGLGISIKGGRENKMPILISKIFKGLAADQTEALFVGDAILSVNGEDLSSATHDEAVQVLKKTGKEVVLEVKYMKDVSPYFK*NSS* | Bam HI / Eco RI | 124SYF<br>5'-TACGGATCCAGCGGCCGCCGCGTGAC-3'<br>N279-301 | 125SYR<br>5'-GTAGAATTCTTGAAATACGGTGAGAC-3'<br>N576-551 |
| | | | | | | |

(continued)

| GENE SYMBOL | PROTEIN | ACC.# | AMINO ACID SEQUENCE* | CLON. SITES | FORWARD PRIMER | REVERSE PRIMER |
|---|---|---|---|---|---|---|
| TAX43 | human tax interaction protein 43 | AF028828 | AA15-85 PDZ domain 1 (of 1)<br>QKRGVKVLKQELGGLGISIKGGK ENKMPILISKIFKGLAADQTQAL YVGDAILSVNGADLRDATHDEAV QAL*QFIVTN* | Bam HI / Eco RI | 97TAF<br>5′-TCTGGATCCAGAAGCGTGGCGTGAAGG-3′<br>N37-63 | 98TAR<br>5′-CGGAATTCAACGCCTGCACCGCCTC-3′<br>N267-231 |
| LDP | lim domain protein clp-36 | U90878 | AA46-88 PDZ domain 1 (of 1)<br>RGMTTQQIDLQGPGPWGFRLVGG KDFEQPLAISRVTPGSKAAL*ASS* | Bam HI / Eco RI | 146LIF<br>5′-CCAGGATCCGCGGAATGACCACCCAGC-3′<br>N129-155 | 147LIR<br>5′-CATGAATTCGCTAGAGCCGCCTTGCTT-3′<br>N276-239 |
| | | | | | | |
| LIM | Human LIM protein | AF061258 | AA29-112; PDZ domain 1 (of 1)<br>*L*SNYSVSLVGPAPWGFRLQGGKD FNMPLTISSLKDGGKAAQANVRI GDVVLSIDGINAQGMTHLEAQNK IKGCTGSLNMTLQRAS*C* | Bam HI / Eco RI | 182LF<br>5′-TTAGGATCCTGAGCAAGTACAGTGTGTCAC-3′<br>N86-115 | 183LR<br>5′-CTTGAATTCAGCAGATGCTCTTTGCAGAGTC-3′<br>N350-320 |
| LIMK1 | human LIM domain kinase 1 | NM_ 002314 | AA194-291; PDZ domain 1 (of 1) | SMA I | 52LIFP | 53LIRP |

(continued)

| GENE SYMBOL | PROTEIN | ACC.# | AMINO ACID SEQUENCE* | CLON. SITES | FORWARD PRIMER | REVERSE PRIMER |
|---|---|---|---|---|---|---|
| | | | *T*VTLVSIPASSHGKRGLSVSIDPPHGPPGCGTEHSHTVRVQGVDPGCMSPDVKNSIHVGDRILEINGTPIRNVPLDEIDLLIQETSRLLQLTIEHD*PGIHRD* | | 5'-CTGCCCGGGACCGTCACCCTGGTGTCC-3' N570-597 | 5'-TCGCCCGGGTCATGCTCGAGGGTC-3' N874-851 |
| LIMK2 | human LIM domain kinase 2 | D45906 | AA185-275; PDZ domain 1 (of 1) PYSVTLISMPATTEGRRGFSVSVESACSNYATTVQVKEVNRMHISPNNRNAIHPGDRILEINGTPVRTLRVEEVEDAISQTSQTLQLLIEH*EFIVTN* | Bam HI / Eco RI | 185LF 5'-AGCGGATCCCCTACTCTGTCACGCTCATC-3' N545-573 | 186LR 5'-GACGAATTCATGTTCAATCAACAGCTGAAG-3' N834-805 |
| | | | | | | |
| MPP2 | maguk p55 subfamily member 2 (DLG2) | X82895 | AA185-273; PDZ domain 1 (of 1) *Q*PVPPDAVRMVGIRKTAGEHLGVTFRVEGGELVIARILHGGMVAQQGLLHVGDIIKEVNGQPVGSDPRALQELLRNASGSVILKILPNYQ*VFIVTD* | Bam HI / Eco RI | 142MF 5'-TCAGGATCCAGCCTGTACCTCCCGATGC-3' N542-569 | 143MR 5'-ATGGAATTCCTGGTAGTTGGGCAGGATC-3' N828-801 |

(continued)

| GENE SYMBOL | PROTEIN | ACC.# | AMINO ACID SEQUENCE* | CLON. SITES | FORWARD PRIMER | REVERSE PRIMER |
|---|---|---|---|---|---|---|
| NOS1 | human neuronal nitric oxide synthase | U17327 | AA239-988; PDZ domain 1 (of 1)<br>IQPNVISVRLFKRKVGGLGFLVK ERVSKPPVIISDLIRGGAAEQSG LIQAGDIILAVNGRPLVDLSYDS ALEVLRGIASETHVVLILRGP*EF IVTD* | Bam HI / Eco RI | 155NOF<br>5'-AGCGGATCCAGCCCAATGTCATTTC-3'<br>N711-733 | 156NOR<br>5'-GAAGAATTCAGGGCCCCTCAGAATG-3'<br>N994-970 |
| | | | | | | |
| AF6 | af-6 protein | U02478 | AA985-1077; PDZ domain 1 (of 1)<br>LRKEPEIITVTLKKQNGMGLSIV AAKGAGQDKLGIYVKSVVKGGAA DVDGRLAAGDQLLSVDGRSLVGL SQERAAELMTRTSSVVTLEVAKQ *GEFIVTD* | Bam HI / Eco RI | 66AFF<br>5'-TCGGATCCTGAGGAAAGAACCTGAA-3'<br>N2946-2970 | 67AFR<br>5'-TAGAATTCACCCTGCTTTGCTACTTC-3'<br>N3239-3214 |
| PTN-4 | proteintyrosine phosphatase meg1 | M68941 | AA774-862; PDZ domain 1 (of 1)<br>LIRMKPDENGRFGFNVKGGYDQK MPVIVSRVAPGTPADLCVPRLNE GDQVVLINGRDIAEHTHDQVVLF IKASCERHSGELMLLVRPNA*EFI VTD* | Bam HI / Eco RI | 247PTF<br>5'-ATCGGATCCTAATCAGAATGAAACCTG-3'<br>N2312-2338 | 248PTR<br>5'-ATCGAATTCAGCATTAGGTCGAACTAG-3'<br>N2595-2569 |

(continued)

| GENE SYMBOL | PROTEIN | ACC.# | AMINO ACID SEQUENCE* | CLON. SITES | FORWARD PRIMER | REVERSE PRIMER |
|---|---|---|---|---|---|---|
| prIL16 | putative interleukin 16 precursor | S81601 | AA170-383; PDZ domain 1-2 (of 2)<br>HVTILHKEEGAGLGFSLAGGADL ENKVITVHRVFPNGLASQEGTIQ KGNEVLSINGKSLKGTTHHDALA ILRQAREPRQAVIVTRKLTPEAM PDLNSSTDSAASASAASDVSVES TAEATVCTVTLEKMSAGLGFSLE GGKGSLHGDKPLTINRIFKGAAS EQSETVQPGDEILQLGGTAMQGL TRFEAWNIIKALPDGPVTIVIRR KSLQSK*EFIVTD* | Bam HI / Eco RI | 75PRF<br>5'-ACGGGATCCATGTCACCATCTTACAC-3'<br>N503-528 | 76PRR<br>5'-GTGAATTCCTTGGACTGGAGGCTTTTTC-3'<br>N1157-1129 |
| 41.8 kD | hypothetical 41.8 kD protein | AF007156 | AA4-85; PDZ domain 1 (of 1)<br>RDSGAMLGLKVVGGKMTESGRLC AFITKVKKGSLADTVGHLRPGDE VLEWNGRLLQGATFEEVYNIILE SKPEPQVELVVSR*ANSS* | Bam HI / Eco RI | 145HF<br>5'-GTGGGATCCGAGATTCAGGAGCAATGC-3'<br>N4-30 | 146HR<br>5'-CTGGAATTCGCCTTGAAACTACAAGTTC-3'<br>N267-240 |
| K559 | KIAA0559 | AB011131 | AA766-870; PDZ1 (of 1)<br>HYIFPHARIKITRDSKDHTVSGN GLGIRIVGGKEIPGHSGEIGAYI AKILPGGSAEQTGKLMEGMQVLE WNGIPLTSKTYEEVQSIISQQSG EAEICVRLDLNML*SNSS* | Bam HI / Eco RI | 130KIF<br>5'-AAAGGATCCACTACATCTTTCCTCACG-3'<br>N2290-2312 | 131KIR<br>5'-TCACAATTGGATAGCATATTGAGGTCCAG-3'<br>N2623-2595 |

(continued)

| GENE SYMBOL | PROTEIN | ACC.# | AMINO ACID SEQUENCE* | CLON. SITES | FORWARD PRIMER | REVERSE PRIMER |
|---|---|---|---|---|---|---|
| RGS12 | human regulator of G-protein signalling 12 | AF035152 | AA35-103; PDZ domain 1 (of 1)<br>*P*PPRVRSVEVARGRAGYGFTLSG QAPCVLSCVMRGSPADFVGLRAG DQILAVNEINVKKASHEDVVKLI *GNSS* | Bam HI / Eco RI | 64RGF<br>5'-TGGGATCCCGCCCCCAAGGGTGCGGAG-3'<br>N93-119 | 65RGR<br>5'-AGGAATTCCCAATTAATTTCACTAC-3'<br>N316-291 |
| K316 | KIAA0316 | AB002314 | AA197-284; PDZ domain 1 (of 1)<br>PPAPRKVEMRRDPVLGFGFVAGS EKPVVVRSVTPGGPSEGKLIPGD QIVMINDEPVSAAPRERVIDLVR SCKESILLTVIQPYPSPK*IRNSS* | Bam HI / Eco RI | 158KIF<br>5'-AAAGGATCCCTCCGGCTCCTCGGAAG-3'<br>N586-611 | 159KIR<br>5'-TTAGAATTCTGATTTGGGAGAAGGGTAAG-3'<br>N866-839 |
| DVL1 | human dishevelled segment polarity protein homolog | AF006011 | AA248-340; PDZ domain 1 (of 1)<br>*QSTV*LNIVTVTLNMERHHFLGIS IVGQSNDRGDGGIYIGSIMKGGA VAADGRIEPGDMLLQVNDVNFEN MSNDDAVRVLREIVSQTGPISLT VAKCW*EFIVTD* | Bam HI / Eco RI | 1st PCR: 55DVISF<br>5'-TCATCCAGACTCATCCGGAAG-3'<br>N652-673<br>2nd PCR, nested: 37DVF<br>5'-TCGGATCCAAACGGTCACTCTCAAC-3'<br>N723-747 | 1stPCR: 56DVISR<br>5'-GCTCATGTCACTCTTCACCG-3'<br>N1195-1174<br>2nd PCR, nested: 38DVR<br>5'-TCGGAATTCCCAGCACTTGGCTACAG-3'<br>N1029-N1004 |

(continued)

| GENE SYMBOL | PROTEIN | ACC.# | AMINO ACID SEQUENCE* | CLON. SITES | FORWARD PRIMER | REVERSE PRIMER |
|---|---|---|---|---|---|---|
| TAX40 | human tax interaction protein 40 | AF028827 | AA35-137; PDZ domain 1 (of 1)<br>LLPETHRRVRLHKHGSDRPLGFY IRDGMSVRVAPQGLERVPGIFIS RLVRGGLAESTGLLAVSDEILEV NGIEVAGKTLDQVTDMMVANSHN LIVTVKPANQ*ANSS* | Bam HI / Eco RI | 136TF<br>5'-ACGGGATCCTACTGCCTGAGACCCACC-3'<br>N97-123 | 137TR<br>5'-ACGGAATTCCGCTGGTTGGCGGGCTTGAC-3'<br>N421-393 |
| TIAM1 | T- lymphoma invasion and metastasis inducing protein 1 | NM_ 003253 | AA1001-1088; PDZ 1 (of 1)<br>HSIHIEKSDTAADTYGFSLSSVE EDGIRRLYVNSVKETGLASKKGL KAGDEILEINNRAADALNSSMLK DFLSQPSLGLLVRTYPELE*EFIV TD* | Bam HI / Eco RI | 39TF<br>5'-TCGGATCCACAGCATCCACATTGAG-3'<br>N2995-3019 | 40TR<br>5'-TCGGAATTCCTCCAGCTCGGGGT-3'<br>N3275-3253 |
| MINT1 | human X11 protein | L04953 | AA717-894; PDZ domains 1-2 (of 2)<br>SENCKDVFIEKQKGEILGVVIVE SGWGSILPTVIIANMMHGGPAEK SGKLNIGDQIMSINGTSLVGLPL STCQSIIKGLENQSRVKLNIVRC PPVTTVLIRRPDLRYQLGFSVQN GIICSLMRGGIAERGGVRVGHRI IEINGQSVVATPHEKIVHILSNA VGEIHMKTMPAAMYRLL*NSS* | Eco RI / Eco RI | 34MIF<br>5'-CGGAATTCGGAAAACTGTAAAGATG-3'<br>N2149-2167 | 20MR<br>5'-TCGGAATTCAGCAGCCTGTACATCG-3'<br>N2690-2666 |
| | | | | | | |
| K3.03 | KIAA0303 | Ab002301 | AA652-742; PDZ domain 1 (of 1) | Bam HI / Eco RI | 152KIF | 153KIR |

(continued)

| GENE SYMBOL | PROTEIN | ACC.# | AMINO ACID SEQUENCE* | CLON. SITES | FORWARD PRIMER | REVERSE PRIMER |
|---|---|---|---|---|---|---|
| | | | PHQPIVIHSSGKNYGFTIRAIRV YVGDSDIYTVHHIVWNVEEGSPA CQAGLKAGDLITHINGEPVHGLV HTEVIELLLKSGNKVSITTTPF*E* *FIVTD* | | 5'- CTGGGATCCC ACATCAGCCG ATTGTGA-3' N1948-1976 | 5'- TGTGAATTCAA ATGGGGTAGTA GTGATTG-3' N2237-2209 |
| CBP | Cytohesin binding protein HE | AF68836 | AA85-176; PDZ domain 1 (of 1) QRKLVTVEKQDNETFGFEIQSYR PQNQNACSSEMFTLICKIQEDSP AHCAGLQAGDVLANINGVSTEGF TYKQVVDLIRSSGNLLTIETLNG *NSS* | Bam HI / Eco RI | 235CYF 5'- CCTGGATCCA AAGAAAGCTT GTTACTGTG-3' N246-274 | 236CYR 5'- TCAGAATTCCA TTAAGAGTCTC TATC-3' N535-510 |
| | | | | | | |
| MINT3 | human MINT3 | AF029110 | AA11-52; PDZ domain 1 (of 1) PVTTAIIHRPHAREQLGFCVEDG IVRPRPLAPGWGGRAALST*EFIV* *TD* | Bam HI / Eco RI | 188MF 5'- ACTGGATCCC CGTCACCACC GCCATCATC-3' N23-51 | 189MR 5'- CTCGAATTCCG TGCTCAGGGCC GCCCTA-3' N165-138 |
| | | | | | | |

(continued)

| GENE SYMBOL | PROTEIN | ACC.# | AMINO ACID SEQUENCE* | CLON. SITES | FORWARD PRIMER | REVERSE PRIMER |
|---|---|---|---|---|---|---|
| TAX2 | human tax interaction protein 2 | AF028824 | AA54-140; PDZ domain 1 (of 1)<br>RKEVEVFKSEDALGLTITDNGAG YAFIKRIKEGSVIDHIHLISVGD MIEAINGQSLLGCRHYEVARLLK ELPRGRTFTLKLTEPRK*EFIVTD* | Bam HI / Eco RI | 197 TF<br>5'-AGGGGATCCGCAAGGAGGTGGAGGTGTTC-3' | 198 TR<br>5'-TGTGGAATTCCTTGCGAGGCTCCGTGAGC-3'<br>N429-401 |
| | | | | | N154-182 | |
| K561 | KIAA0561 | AB011133 | AA948-1038; PDZ domain 1 (of 1)<br>PPSLSTALARSTASACGRSASTW VIATSTLCTTSSGVWRTEAPPRR RACGLGTSSPTSTGSQCWGWCTW TSWSCCZRAATRYPCGPQPWR*IH RD* | Bam HI / Eco RI | 161KIF<br>5'-CCTGGATCCCCCCATCGTTATCCACAGC-3'<br>N2836-2863 | 162KIR<br>5'-GAGGAATTCTCCAGGGCTGTGGTCCG-3'<br>N3120-3095 |
| *Note concerning TABLE 3 | | | | | | |

In several cases, sequence analysis of the PDZ clones revealed differences to the DNA and/or protein sequence as published in the databases, summarized in **TABLE 3A**.

**TABLE 3A**

| GENE | GENBANK ENTRY*** | ACTUAL CONSTRUCT |
|---|---|---|
| AF6 | N 3060: C | N 3060: T * |
| DLG1 | N 1021: A, = AA 340: Gln | N 1021: G, = AA 340: Arg |
| Lim dom. | N 202: G<br>N 203: C, = AA 68: Arg | N202:C*<br>N 203: G, = AA 68: Gly |
| LIMK1 | N 855: C, = AA 285: Leu | N 855: A, = AA 285: Ile |
| MINT1 | N 2386: G, = AA 796: Glu** | N 2386: A, = AA 796: Lys** |
| NE-DLG | N 713: T<br>N 766: G, = AA 255: Gly<br>N 803: G, = AA 267: Glu<br>N 861: G, = AA 287: Val | N 713: C*<br>N 766: A, = AA 255: Glu<br>N 803: C, = AA 267: Asp<br>N 861: A, = AA 287: Met |
| TIAM1 | N 3224: A | N 3224: G* |

(*) = silent mutation, does not effect the AA sequence;
(**) = MINT1 is the same as X11a. The database entry for X11a shows the same sequence as our actual construct with regard to N 2386 of the MINT1 GenBank entry.
(***)= Nucleotide ("N") and amino acid ("AA") annotations correspond to the numbering as found in the GenBank files.

6.2 Assays for Detection of Interactions Between PDZ-Domain Polypeptides and Candidate PDZ Ligand proteins (PL proteins)

Two complementary assays, termed "A' and "G,"" were developed to detect binding between a PDZ-domain polypeptide and candidate PDZ ligand. In each of the two different assays, binding, is detected between a peptide having a sequence corresponding to the C-terminus of a protein anticipated to bind to one or more PDZ domain (i.e. a candidate PL peptide) and a PDZ-domain polypeptide (typically a fusion protein containing a PDZ domain). In the "A" assay, the candidate, PL peptide is immobilized and binding of a soluble PDZ-domain polypeptide to the immobilized peptide is detected (the "A"' assay is named for the fact that in one embodiment an avidin surface is used to immobilize the peptide). In the "G" assay, the PDZ-domain polypeptide is immobilized and binding of a soluble PL peptide is detected (The "G" assay is named for the fact that in one embodiment a GST-binding surface is used to immobilize the PDZ-domain polypeptide). Preferred embodiments of these assays are described in detail *infra.* However, it will be appreciated by ordinarily skilled practitioners that these assays can be modified in numerous ways while remaining useful for the purposes of the present invention.

6.2.1 Production of Fusion Proteins Containing PDZ-Domains

GST- PDZ domain fusion proteins were prepared for use in the assays of the invention. PCR products containing PDZ encoding domains (as described in §6.1 *supra*) were subcloned into an expression vector to permit expression of fusion proteins containing a PDZ domain and a heterologous domain (i.e., a glutathione- S transferase sequence, "GST") . PCR products (i.e., DNA fragments) representing PDZ domain encoding DNA was extracted from agarose gels using the "sephaglas" gel extraction system (Pharmacia) according to the manufacturer's recommendations.

As noted *supra,* PCR primers were designed to include endonuclease restriction sites to facilitate ligation of PCR fragments into a GST gene fusion vector (pGEX-3X; Pharmacia, GenBank accession no. XXU13852) in-frame with the glutathione-S transferase coding sequence. This vector contains a IPTG inducible lacZ promoter. The pGEX-3X vector was linearized using *Bam* HI and *Eco* RI or, in some cases, *Eco* RI or *Sma* I, as shown in **TABLE** *3*, and dephosphorylated. For most cloning approaches, double digest with Bam HI and Eco RI was performed, so that the ends of the PCR fragments to clone were Bam HI and Eco RI. In some cases, restriction endonuclease combinations used were Bgl II and Eco RI, Bam HI and Mfe I, or Eco RI only, Sma I only, or BamHI only (see **TABLE 3**). When more than one PDZ domain was cloned, the DNA portion cloned represents the PDZ domains and the cDNA portion located between individual domains. Precise locations of cloned fragments used in the assays are indicated in **TABLE 3**. DNA linker sequences between the GST portion and the PDZ domain containing DNA portion vary slightly, dependent on which of the above described cloning sites and approaches were used. As a consequence, the amino acid sequence of the GST-PDZ fusion protein varies in the linker region between GST and PDZ domain. Protein linkers sequences corresponding to different cloning sites/approaches are shown below. Linker sequences (vector DNA encoded) are bold, PDZ domain containing gene derived sequences are in italics.

1) **GST- BamHI**/ *BamHI- PDZ domain- insert*
*Gly-- Ile- PDZ domain insert*

2) **GST-BamHI**/*BglII-PDZ domain insert*
**Gly-Ile**-*PDZ domain insert*

3) ***GST- EcoRI***/ *EcoI- PDZ domain insert*
**Gly- Ile- Pro- Gly-- Asn-** *PDZ domain insert*

4) **GST-- SmaI**/*SmaI- PDZ domain insert*
**Gly- Ile- Pro-** *PDZ domain insert*

The PDZ-encoding PCR fragment and linearized pGEX-3X vector were ethanol precipitated and resuspended in 10 ul standard ligation buffer. Ligation was performed for 4-10 hours at 7°C using T4 DNA ligase. It will be understood that some of the resulting constructs include very short linker sequences and that, when multiple PDZ domains were cloned, the constructs included some DNA located between individual PDZ domains.

The ligation products were transformed in DH5α or BL-21 *E.coli* bacteria strains. Colonies were screened for presence and identity of the cloned PDZ domain containing DNA as well as for correct fusion with the glutathione S-transferase encoding DNA portion by PCR and by sequence analysis. Positive clones were tested in a small scale assay for expression of the GST/PDZ domain fusion protein and, if expressing, these clones were subsequently grown up for large scale preparations of GST/PDZ fusion protein.

GST- PDZ domain fusion protein was overexpressed following addition of IPTG to the culture medium and purified. Detailed procedure of small scale and large scale fusion protein expression and purification are described in "GST Gene Fusion System" (second edition, revision 2; published by Pharmacia) . In brief, a small culture (3- 5mls) containing a bacterial strain (DH5α, BL21 or JM109) with the fusion protein construct was grown overnight in LB- media at 37°C with the appropriate antibiotic selection (100ug/ml ampicillin; a.k.a. LB- amp) . The overnight culture was poured into a fresh preparation of LB- amp (typically 250- 500mls) and grown until the optical density (OD) of the culture was between 0.5 and 0.9 (approximately 2.5 hours) . IPTG (isopropyl (β- D- thiogalactopyranoside) was added to a final concentration of 1.0mM to induce production of GST fusion protein, and culture was grown an additional 1.5- 2.5 hours. Bacteria were collect by centrifugation (4500 g) and resuspended in Buffer A- (50mM Tris, pH 8.0, 50mM dextrose, 1mM EDTA, 200uM phenylmethylsulfonylfluoride) . An equal volume of Buffer A+ (Buffer A-, 4mg/ml lysozyme) was added and incubated on ice for 3 min to lyse bacteria. An equal volume of Buffer B (10mM Tris, pH 8.0, 50mM KCl, 1mM EDTA. 0.5% Tween- 20, 0.5% NP40 (a.k.a. IGEPAL CA- 630) , 200uM phenylmethylsulfonylfluoride) was added and incubated for an additional 20 min. The bacterial cell lysate was centrifuged (x20, 000g) , and supernatant was added to glutathione sepharose 4B (Pharmacia, cat no. 17- 0765- 01) previous swelled (rehydrated) in 1X phosphate- buffered saline (PBS) . The supernatant- sepharose slurry was poured into a column and washed with at least 20 bed volumes of 1X PBS. GST fusion protein was eluted off the glutathione sepharose by applying 0.5- 1.0 ml aliquots of 5mM glutathione and collected as separate fractions. Concentrations of fractions were determined using BioRadProtein Assay (cat no. 500-0006) according to manufacturer's specifications. Those fractions containing the highest concentration of fusion protein were pooled and dialyzed against 1X PBS/ 35% glycerol. Fusion proteins were assayed for size and quality by SDS gel electrophoresis (PAGE) as described in "Sambrook." Fusion protein aliquots were stored at minus 80°C and at minus 20°C.

6.2.2 <u>Identification of Candidate PL Proteins and Synthesis of Peptides</u>

In some non- hematopoietic cells (e.g., neurons, epithelial cells) , certain PDZ domains are known to be bound by the C- terminal residues of PDZ- binding proteins. To identify PL proteins that function in hematopoietic and endothelial cells, cell surface receptor proteins were identified and peptides having the sequence corresponding to the c- terminus of each protein were synthesized. **TABLE 4** lists these proteins, and provides corresponding C- terminal sequences and GenBank accession numbers. "Clasp 1" is described in WO 00/20434 (published 13 April 2000) . "Clasp 2" and "Clasp 4" are described in copending applications USSN 09/547276 (attorney docket No. 20054- 000200) and 60/196527 (attorney docket No. 20054- 000400) , both filed April 11, 2000.

Synthetic peptides of defined sequence (e.g., corresponding to the carboxyl-termini of the indicated proteins) can be synthesized by any standard resin-based method (see, e.g., U. S. Pat. No. 4,108,846; see also, Caruthers et al., 1980, Nucleic Acids Res. Symp. Ser., 215-223; Horn et al., 1980, Nucleic Acids Res. Symp. Ser., 225-232; Roberge, et al., 1995, Science 269:202). The peptides used in the assays described herein were prepared by the FMOC (see, e.g., Guy and Fields, 1997, Meth. Enz. 289:67-83; Wellings and Atherton, 1997, Meth. Enz.289:44-67). In some cases (e.g., for use in the A and G assays of the invention), peptides were labeled with biotin at the amino-terminus by reaction with a four-fold excess of biotin methyl ester in dimethylsulfoxide with a catalytic amount of base. The peptides were cleaved from the resin using a halide containing acid (e.g. trifluoroacetic acid) in the presence of appropriate antioxidants (e.g. ethanedithiol) and excess solvent lyophilized.

Following lyophilization, peptides can be redissolved and purified by reverse phase high performance liquid chromatography (HPLC). One appropriate HPLC solvent system involves a Vydac C-18 semi-preparative column running at 5 mL per minute with increasing quantities of acetonitrile plus 0.1 % trifluoroacetic acid in a base solvent of water plus 0.1% trifluoroacetic acid. After HPLC purification, the identities of the peptides are confirmed by MALDI cation-mode mass spectrometry. As noted, exemplary biotinylated peptides are provided in **TABLE 4**.

6.2.3 <u>Detecting PDZ-PL Interactions</u>

Based on the determination that immune system cells contain both many PDZ proteins and similarly many candidate PL proteins, it was apparent to the inventors that characterization of the specific PDZ-PL interactions among these proteins would require reliable and rapid assays for such interactions. A variety of assay formats known in the art can be used to select ligands that are specifically reactive with a particular protein. For example, solid-phase ELISA immunoassays, immunoprecipitation, Biacore, and Western blot assays can be used to identify peptides that specifically bind PDZ-domain polypeptides. As discussed *supra,* two different, complementary assays were developed to detect PDZ-PL interactions. In each, one binding partner of a PDZ-PL pair is immobilized, and the ability of the second binding partner to bind is determined. These assays, which are described *infra,* can be readily used to screen for hundreds to thousand of potential PDZ-ligand interactions in a few hours. Thus these assays can be used to identify yet more novel PDZ-PL interactions in hematopoietic cells. In addition, they can be used to identify antagonists of PDZ-PL interactions (see *infra*).

6.2.3.1 <u>"A Assay"Detection of PDZ-Ligand Binding Using Immobilized PL Peptide.</u>

In one aspect, the invention provides an assay in which biotinylated candidate PL peptides are immobilized on an avidin coated surface. The binding of PDZ-domain fusion protein to this surface is then measured. In a preferred embodiment, the PDZ-domain fusion protein is a GST/PDZ fusion protein and the assay is carried out as follows:

(1) Avidin is bound to a surface, e.g. a protein binding surface. In one embodiment, avidin is bound to a polystyrene 96 well plate (e.g., Nunc Polysorb (cat #475094) by addition of 100 uL per well of 20 ug/mL of avidin (Pierce) in phosphate buffered saline without calcium and magnesium, pH 7.4 ("PBS", GibcoBRL) at 4°C for 12 hours. The plate is then treated to block nonspecific interactions by addition of 200 uL per well of PBS containing 2 g per 100 mL protease-free bovine serum albumin ("PBS/BSA") for 2 hours at 4°C. The plate is then washed 3 times with PBS by repeatedly adding 200 uL per well of PBS to each well of the, plate and then dumping the contents of the plate into a waste container and tapping the plate gently on a dry surface.

(2) Biotinylated PL peptides (or candidate PL peptides, e.g. see **TABLE 4**) are immobilized on the surface of wells of the plate by addition of 50 uL per well of 0.4 uM peptide in PBS/BSA for 30 minutes at 4°C. Usually, each different peptide is added to at least eight different wells so that multiple measurements (e.g. duplicates and also measurements using different (3ST/PDZ-domain fusion proteins and a GST alone negative control) can be made, and also additional negative control wells are prepared in which no peptide is immobilized. Following immobilization of the PL peptide on the surface, the plate is washed 3 times with PBS.

(3) GST/PDZ-domain fusion protein (prepared as described *supra*) is allowed to react with the surface by addition of 50 uL per well of a solution containing 5 ug/mL GST/PDZ-domain fusion protein in PBS/BSA for 2 hours at 4°C. As a negative control, GST alone (i.e. not a fusion protein) is added to specified wells, generally at least 2 wells (i.e. duplicate measurements) for each immobilized peptide. After the 2 hour reaction, the plate is washed 3 times with PBS to remove unbound fusion protein.

(4) The binding of the GST/PDZ- domain fusion protein to the avidin- biotinylated peptide surface can be detected using a variety of methods, and detectors known in the art. In one embodiment, 50 uL per well of an anti- GST antibody in PBS/BSA (e.g. 2.5 μg/mL of polyclonal goat- anti- GST antibody, Pierce) is added to the plate and allowed to react for 20 minutes at 4°C. The plate is washed 3 times with PBS and a second, delectably labeled antibody is added. In one embodiment, 50 uL per well of 2.5 ug/mL of horseradish peroxidase (HRP)- conjugated polyclonal rabbit anti- goat inununoglobulin antibody is added to the plate and allowed to react for 20 minutes at 4°C. The plate is washed 5 times with 50 mM Tris pH 8.0 containing 0.2% Tween 20, and developed by addition of 100 uL per well of HRP- substrate solution (TMB, Dako) for 20 minutes at room temperature (RT) . The reaction of the HRP and its substrate is terminated by the addition of 100 uL per well of 1M sulfuric acid and the optical density (O.D.) of each well of the plate is read at 450 nm.

(5) Specific binding of a PL peptide and a PDZ-domain. polypeptide is detected by comparing the signal from the well(s) in which the PL peptide and PDZ domain polypeptide are combined with the background signal(s). The background signal is the signal found in the negative controls. Typically a specific or selective reaction will be at least twice background signal, more typically more than 5 times background, and most typically 10 or more times the background signal. In addition, a statistically significant reaction will involve multiple measurements of the reaction with the signal and the background differing by at least two standard errors, more typically four standard errors, and most typically six or more standard errors. Correspondingly, a statistical test (e.g. a T-test) comparing repeated measurements of the signal with repeated measurements of the background will result in a p-value < 0.05, more typically a p-value < 0.01, and most typically a p-value < 0.001 or less.

As noted, in an embodiment of the "A" assay, the signal from binding of a GST/PDZ-domain fusion protein to an avidin surface not exposed to (i.e. not covered with) the PL peptide is one suitable negative control (sometimes referred to as "B"). The signal from binding of GST polypeptide alone (i.e. not a fusion protein) to an avidin-coated surface that has been exposed to (i.e. covered with) the PL peptide is a second suitable negative control (sometimes referred to as "B2"). Because all measurements are done in multiples (i.e. at least **duplicate)** the arithmetic mean (or, equivalently, average) of several measurements is used in determining the binding, and the standard error of the mean is used in determining the probable error in the measurement of the binding. The standard error of the mean of N measurements equals the square root of the following: the sum of the squares of the difference between each measurement and the mean, divided by the product of (N) and (N-1). Thus, in one embodiment, specific binding of the PDZ protein to the plate-bound PL peptide is determined by comparing the mean signal ("mean S") and standard error of the signal ("SE") for a particular PL-PDZ combination with the mean B1 and/or mean B2. In **TABLE 2,** binding was detected to be specific (denoted by an "A" in the matrix) when (1) the, mean S was at least twice the mean B1 and at least twice the mean B2 and (2) the mean S was at least six standard errors (six SE) greater than both the mean B1 and the mean B2. In addition, in the experiments summarized in **TABLE 2**, an additional criterion was used to ensure that none of the interactions defined as specific arose from a combined tendency of both the particular PDZ fusion protein and PL peptide tested to each give a higher than usual background. This criteria was that (3) the mean S was at least twenty times the product of the mean B1 and the mean B2. The factor twenty times reflects that at least one of B 1 and B2 is generally less than 0.1 O.D. units, and therefore twenty times the product of the mean B1 and the mean B2 is generally less than twice the mean B1 and twice the mean B2, making criteria (3) less stringent than criteria (1). Only in a few cases where the mean B 1 and the mean B2 are both greater than 0.1 O.D. units (i.e. both the particular PDZ fusion protein and PL peptide tested tend to give a higher than usual background) is criteria (3) more stringent than criteria (1).

| | | | |
|---|---|---|---|
| **Table 4: PL Peptides** | | | |
| | | | |
| **CODE** | **PROTEIN NAME** | **GENBANK ACCESS** | **SEQUENCE** |
| | | | |
| AA1L | Clasp-1 | | ISKATPALPTVSISSSAEV |
| AA2L | Clasp-2 | | ISGTPTSTMVHGMTSSSSVV |
| AA3L | Clasp-4 | | CAISGTSSDRGYGSPRYAEV |
| AA4L | CD3n | M33158 | SVFSIPTLWSPWPPSSSSQL |
| AASL-M* | CD4 | M12807 | SEKKTSQSPHRFQKTCSPI |

(continued)

| CODE | PROTEIN NAME | GENBANK ACCESS | SEQUENCE |
|---|---|---|---|
| AA6L | CD6 | X60992 | SPQPDSTDNDDYDDISAA |
| AA7L | CD34 | M81104 | QATSRNGHSARQHVVADTEL |
| AA9L | CD44 | M69215 | QFMTADETRNLQNVDMKIGV |
| AA10L | CD46 (Form 1) | M58050 | KKGTYLTDETHREVKFTSL |
| AA11L | CD49E ( 4) | X06256 | PYGTAMEKAQLKPPATSDA |
| AA12L | CD49F | X53586 | HKAEIHAQPSDKERLTSDA |
| AA13L | CD95 | M67454 | KDITSDSENSNFRNEIQSLV |
| AA14L | CD97 | X84700 | TSGTGHNQTRALRASESGI |
| AA15L | CD98 | J02939 | ERLKLEPHEGLLLRFPYAA |
| AA16L | CD105 | X72012 | STNHSIGSTQSTPCSTSSMA |
| AA17L | VCAM1 | M73255 | ARKANMKGSYSLVEAQKSKV |
| AA18L | CD138 | J05392 | PKQANGGAYQKPTKQEEFYA |
| AA19L | CD148 | D37781 | ENLAPVTTFGKTNGYIA |
| AA20L | CD166 | L38608 | DLGNMEENKKLEENNHKTEA |
| AA22L | DNAM-1 | U56102 | TREDIYVNYPTFSRRPKTRV |
| AA23L-M* | FasL | U11821 | SSKSKSSEESQTFFGLYKL |
| AA25L | FceRIb | D10583 | YSATYSELEDPGEMSPPIDL |
| AA28L | CDW125 (IL5R) | X62156 | EVICYIEKPGVETLEDSVF |
| AA29.1L | CDW128A (ILBRA) | M68932 | ARHRVTSYTSSSVNVSSNL |
| AA29.2L | CDW128B (IL8RB) | M73969 | KDSRPSFVGSSSGHTSTTL |
| AA30L | LPAP | X81422 | AWDDSARAAGGQGLHVTAL |
| AA33L | KV1.3 | AAC31761 | TTNNNPNSAVNIKKIFTDV |
| AA34.2L | NMDA | NP000824 | LNSCSNRRVYKKMPSIESDV |
| AA37L | Glycophorin C | AAA52574 | QGDPALQDAGDSSRKEYFI |
| AA38L | Neurexin | AB011150 | SSAKSSNKNKKNKDKEYYV |
| AA39L | Syndecan-2 | A33880 | GERKPSSAAYQKAPTKEFYA |
| AA40L | DOCK2 | BAA13200 | LASKSAEEGKQIPDSLSTDL |
| AA41L | CC CKR-1R | L09230 | LERVSSTSPSTGEHELSAGF |
| AA42L | CC CKR-2 | U03882 | GKGKSIGRAPEASLQDKEGA |
| AA43L | CC CKR-3 | HSU28694 | LERTSSVSPSTAEPELSIVF |
| AA44L | CC CKR-4 | X85740 | DTPSSSYTQSTMDHDLHDAL |
| AA45L | BLR-1 | S56162 | PSWRRSSLSESENATSLTTF |
| AA47L | CD83 | Z11697 | VTSPNKHLGLVTPHKTELV |
| AA48L | CD62E | M30640 | SSSQSLESDGSYQKPSYIL |
| AA49L | CD5 | X04391 | SMQPDNSSDSDYDLHGAQRL |
| AA55L | CD148 | D37781 | TIYENLAPVTTFGKTIA |
| | | | |
| | | | |

(continued)

| CODE | PROTEIN NAME | GENBANK ACCESS | SEQUENCE |
|---|---|---|---|
| | *The Sequence studied is mutated at positions >10 amino acids | | |
| | from C-terminus to increase water solubility and/or | | |
| | eliminate intramolecular disulfides. | | |

6.2.3.2 "G Assay" - Detection of PDZ-Ligand Binding Using Immobilized PDZ-Domain Fusion Polypeptide

In one aspect, the invention provides an assay in which a GST/PDZ fusion protein is immobilized on a surface ("G" assay). The binding of labeled PL peptide (as listed in **TABLE 4**) to this surface is then measured. In a preferred embodiment, the assay is carried out as follows:

(1) A PDZ-domain polypeptide is bound to a surface, e.g. a protein binding surface. In a preferred embodiment, a GST/PDZ fusion protein containing one or more PDZ domains is bound to a polystyrene 96-well plate. The GST/PDZ fusion protein can be bound to the plate by any of a variety of standard methods known to one of skill in the art, although some care must be taken that the process of binding the fusion protein to the plate does not alter the ligand-binding properties of the PDZ domain. In one embodiment, the GST/PDZ fusion protein is bound via an anti-GST antibody that is coated onto the 96-well plate. Adequate binding to the plate can be achieved when:

a. 100 uL per well of 5 ug/mL goat anti-GST polyclonal antibody (Pierce) in PBS is added to a polystyrene 96-well plate (e.g., Nunc Polysorb) at 4°C for 12 hours.
b. The plate is blocked by addition of 200 uL per well of PBS/BSA for 2 hours at 4°C.
c. The plate is washed 3 times with PBS.
d. 50 uL per well of 5 ug/mL GST/PDZ fusion protein) or, as a negative control, GST polypeptide alone (i.e. not a fusion protein) in PBS/BSA is added to the plate for 2 hours at 4°C.
e. the plate is again washed 3 times with PBS.

(2) Biotinylated PL peptides (or candidate PL peptides, e.g. as shown in **TABLE 4**) are allowed to react with the surface by addition of 50 uL per well of 20 uM solution of the biotinylated peptide in PBS/BSA for 10 minutes at 4°C, followed by an additional 20 minute incubation at 25°C. The plate is washed 3 times with ice cold PBS.

(3) The binding of the biotinylated peptide to the GST/PDZ fusion protein surface can be detected using a variety of methods and detectors known to one of skill in the art. In one embodiment, 100 uL per well of 0.5 ug/mL streptavidin-horse radish peroxidase (HRP) conjugate dissolved in BSA/PBS is added and allowed to react for 20 minutes at 4°C. The plate is then washed 5 times with 50 mM Tris pH 8.0 containing 0.2% Tween 20, and developed by addition of 100 uL per well of HRP-substrate solution (TMB, Dako) for 20 minutes at room temperature (RT). The reaction of the HRP and its substrate is terminated by addition of 100 uL per well of I M sulfuric acid, and the optical density (O.D.) of each well of the plate is read at 450 um.

(4) Specific binding of a PL peptide and a PDZ domain polypeptide is determined by comparing the signal from the well(s) in which the PL peptide and PDZ domain polypeptide are combined, with the background signal(s). The background signal is the signal found in the negative control(s). Typically a specific or selective reaction will be at least twice background signal, more typically more than 5 times background, and most typically 10 or more times the background signal. In addition, a statistically significant reaction will involve multiple measurements of the reaction with the signal and the background differing by at least two standard errors, more typically four standard errors, and most typically six or more standard errors. Correspondingly, a statistical test (e.g. a T-test) comparing repeated measurements of the signal with -repeated measurements of the background will result in a p-value < 0.05, more typically a p-value < 0.01, and most typically a p-value < 0.001 or less. As noted, in an embodiment of the "G" assay, the signal from binding of a given PL peptide tc immobilized (surface bound) GST polypeptide alone is one suitable negative control (sometimes referred to as "B 1 "). Because all measurement are done in multiples (i.e. at least duplicate) the arithmetic mean (or, equivalently, average.) of several measurements is used in determining the binding, and the standard error of the mean is used in determining the probable error in the measurement of the binding. The standard error of the mean of N measurements equals the square root of the following: the sum of the squares of the difference between each measurement and the mean, divided by the product of (N) and (N-1). Thus, in one embodiment, specific binding of the PDZ protein to the platebound peptide is determined by comparing the mean signal ("mean S") and standard error of the signal ("SE") for a particular PL-PDZ combination with the mean B1. In experiments summarized in **TABLE 2**, binding was determined to be specific (denoted by a "G" in the matrix) when (1) the mean S was at least twice the mean B1 and (2) the mean S was at least six standard errors (six SE) greater than the mean B1. Results of exemplary "G" assays are shown in **Figures 1A-1D**.

6.2.4 Assay Variations

As discussed *supra,* it will be appreciated that many of the steps in the above-described assays can be varied, for example, various substrates can be used for binding the PL and PDZ-containing proteins; different types of PDZ containing fusion proteins can be used; different labels for detecting PDZ/PL interactions can be employed; and different ways of detection can be used.

The PDZ-PL detection assays can employ a variety of surfaces to bind the PL and PDZ-containing proteins. For example, a surface can be an "assay plate" which is formed from a material (e.g. polystyrene) which optimizes adherence of either the PL protein or PDZ-containing protein thereto. Generally, the individual wells of the assay plate will have a high surface area to volume ratio and therefore a suitable shape is a flat bottom well (where the proteins of the assays are adherent). Other surfaces include, but are not limited to, polystyrene or glass beads, polystyrene or glass slides, and alike.

For example, the assay plate can be a "microtiter" plate. The term "microtiter" plate when used herein refers to a multiwell assay plate, e.g., having between about 30 to 200 individual wells, usually 96 wells. Alternatively, high density arrays can be used Often, the individual wells of the microtiter plate will hold a maximum volume of about 250 ul. Conveniently, the assay plate is a 96 well polystyrene plate (such as that sold by Becton Dickinson Labware, Lincoln Park, N.J.), which allows for automation and high throughput screening. Other surfaces include polystyrene microtiter ELISA plates such as that sold by Nunc Maxisorp, Inter Med, Denmark. Often, about 50 ul to 300 ul, more preferably 100 ul to 200 ul, of an aqueous sample comprising buffers suspended therein will be added to each well of the assay plate.

The detectable labels can be any detectable compound or composition which is conjugated directly or indirectly with a molecule (such as described above). The label can be detectable by itself (e.g., radioisotope labels or fluorescent labels) or, in the case of an enzymatic label, can catalyze a chemical alteration of a substrate compound or composition which is detectable. The preferred label is an enzymatic one which catalyzes a color change of a non-radioactive color reagent.

Sometimes, the label is indirectly conjugated with the antibody. One of skill is aware of various techniques for indirect conjugation. For example, the antibody can be conjugated with biotin and any of the categories of labels mentioned above can be conjugated with avidin, or vice versa (see also "A" and "G" assay above) . Biotin binds selectively to avidin and thus, the label can be conjugated with the antibody in this indirect manner. See, Ausubel, *supra,* for a review of techniques involving biotin- avidin conjugation and similar assays. Alternatively, to achieve indirect conjugation of the label with the antibody, the antibody is conjugated with a small hapten (e.g. digoxin) and one of the different types of labels mentioned above is conjugated with an anti- hapten antibody (e.g. anti- digoxin antibody) . Thus, indirect conjugation of the label with the antibody can be achieved.

Assay variations can include different washing steps. By "washing" is meant exposing the solid phase to an aqueous solution (usually a buffer or cell culture media) in such a way that unbound material (e.g., non-adhering cells, non-adhering capture agent, unbound ligand, receptor, receptor construct, cell lysate, or HRP antibody) is removed therefrom. To reduce background noise, it is convenient to include a detergent (e.g., Triton X) in the washing solution. Usually, the aqueous washing solution is decanted from the wells of the assay plate following washing. Conveniently, washing can be achieved using an automated washing device. Sometimes, several washing steps (e.g., between about 1 to 10 washing steps) can be required.

Various buffers can also be used in PDZ-PL detection assays. For example, various blocking buffers can be used to reduce assay background. The term "blocking buffer" refers to an aqueous, pH buffered solution containing at least one blocking compound which is able to bind to exposed surfaces of the substrate which are not coated with a PL or PDZ-containing protein. The blocking compound is normally a protein such as bovine serum albumin (BSA), gelatin, casein or milk powder and does not cross-react with any of the reagents in the assay. The block buffer is generally provided at a pH between about 7 to 7.5 and suitable buffering agents include phosphate and TRIS.

Various enzyme-substrate combinations can also be utilized in detecting PDZ-PL interactions. Examples of enzyme-substrate combinations include, for example:

(i) Horseradish peroxidase (HRPO) with hydrogen peroxidase as a substrate, wherein the hydrogen peroxidase oxidizes a dye precursor (e.g. orthophenylene diamine [OPD] or 3, 3', 5, 5'- tetramethyl benzidine hydrochloride [TMB] ) (as described above) .

(ii) alkaline phosphatase (AP) with para-Nitrophenyl phosphate as chromogenic substrate.

(iii) β- D- galactosidase (β D- Gal) with a chromogenic substrate (e.g. p- nitrophenyl- β- D- galactosidase) or fluorogenic substrate 4- methylumbelliferyl- β- D- galactosidase.

Numerous other enzyme-substrate combinations are available to those skilled in the art. For a general review of these, see U.S. Pat. Nos. 4,275,149 and 980.

Further, it will be appreciated that, although, for convenience, the present discussion primarily refers antagonists of PDZ- PL interactions, agonists of PDZ- PL interactions can be identified using the methods disclosed herein or readily apparent variations thereof.

6.2.5 Results of PDL-PL Interaction Assays

**TABLE 2**, *supra,* shows the results of assays in which specific binding was detected using the "A" and "G" assays described herein. The top row of the table specifies the source of the PDZ domain used in the GST-PDZ fusion proteins (see **TABLE 3**). The first column lists the cell surface proteins from which C-terminal peptide sequences were derived and the second column ("code") identifies the peptide used in the assay (see **TABLE 4**). The third column, "Seq" provides the sequence of the four (4) C-terminal residues of the cell surface protein and peptide. In the matrix, "A" indicates specific binding as detected in the "A" assay. "G" indicates specific binding as shown in the "G" assay. A blank indicates that no specific binding was detected using the "A" or "G" assays. An asterisk (*) indicates that a pairwise interaction between the PDZ protein and the cell surface protein (or subdomains of either) has been described by others.

6.2.5.1 New PL Motifs

As noted supra, **TABLE 2** shows the results of assays (referred to as "PRISM MATRIX") to detect binding between PDZ proteins and candidate PL peptides. A number of specific PDZ-PL interactions are identified by the MATRIX and key amino acids and positions important in PDZ binding ("PL motifs") are deduced from these results. Not only is the MATRIX useful to catalog comprehensively PDZ-PL binding combinations, the assay can further aid in the rapid discovery and characterization of novel PL proteins and PL motifs to help in rational drug design and synthesis of PL-PDZ interaction inhibitors.

Other investigators have reported certain PL motifs important in PDZ binding, e.g., the C-terminal motifs S/T-X-V/I/L (for DLG1) and Y/F-Y/F-I/L/F for MPP1 (see, Doyle et al., 1996, Cell 85, 1067; Songyang et al., 1997, Science 275, 73). However, the reported motifs are not sufficiently specific (i.e. a large number of proteins meet these criteria yet are not necessarily actual PDZ ligands) and cover only a small number of PDZ proteins (approximately 10). The PRISM MATRIX can be used to determine ligand specificity and to deduce ligand binding motifs for any PDZ protein because it can precisely determine sequences of amino acids that do or do not result in specific PDZ binding. In addition, the assay has revealed a significant of new PDZ domain binding motifs (i.e. PL motifs): C-terminal sequence of CD6, ISAA; C- terminal sequence of CD49E, TSDA; C- terminal sequence of CD49F, TSDA; C-terminal sequence of Clap-1, SAEV; C- terminal sequence of CLASP-4, YAEV; C- terminal sequence of CD44, KIGV; C-terminal sequence of IL5R, DSVF; C-terminal sequence of BLR-1, LTTF. Identification of these novel PL sequences allows the definition of novel PL motifs (See **TABLE 5A**, *infra).* The specificity with which these novel motifs are defined is enhanced by the fact that the MARTRIX reports both positive results (i.e. PDZ-PL) combinations that result in specific binding interactions) and negative results (i.e. PDZ-PL combinations that do not result in specific binding). For example, the C-terminal sequence of CD6, SAA and the C-terminal sequence of CD49E, SDA bind to the PDZ-domain polypeptide 41.8 while the related C-terminal sequence of CD166, TEA and C- terminal sequence of CD148, YIA do not. This identifies the novel PL motif (Motif 1, *infra)* of polypeptides terminating in alanine with serine at the -2 position and excludes polypeptides with threonine and tyrosine at the -2 position. This motif is therefore more specific than most previously identified motifs. Other novel motifs are described in **TABLE 5A**.

**TABLE 5A**

| Position: | -3 | -2 | -1 | C- terminal |
|---|---|---|---|---|
| Motif 1 | X | S | X | A |
| Motif 2 | X | A | D/E | V |
| Motif 3 | X | V/I/L | X* | V |
| Motif 4 | X | S/T | X | F |

X* is any non-aromatic amino acid (any residue other than T, F or W).

6.2.5.2 Binding Clusters

**TABLE 2** is arranged so that both the PDZ/GST fusion proteins and the PL peptide ligands are ordered with structurally similar molecules close to each other. In preparing TABLE 2 and carrying out the experiments used to create this table, the PDZ domains of each of the GST-PDZ fusion proteins were ranked for amino acid sequence similarity using the CLUSTAL multiple sequence alignment software package. Proteins with greatest sequence similarity are closest to each other in the matrix. The PL peptide ligands were also ordered on the basis of amino acid similarity, but with weight given to residues reported to be important in PDZ binding (Doyle et al., 1996, Cell 85, 1067). In particular, peptides were first ordered based on the most C-terminal residue (zero position) in the following amino acid order: G, A, C, S, T, N, Q, D, E, H, K, R, V, I, L, M, P, F, Y, W. Among peptides with identical C-termini, the same ranking scheme was then applied to the next most important residue for peptide binding, the -2 position, followed by the -1 position and the -3 position. (In an alternative approach, the GST-PDZ fusions can also be arranged to give additional weight to residues known to be important for ligand binding when aligning the proteins.)

Regions of the TABLE 2 matrix that are densely filled with significant binding interactions indicate that a particular ligand structure tends to bind to a particular family of PDZ domains. The results indicate that PDZ domains with similar structures bind ligands with similar structures, as indicated by the presence in the matrix of some clusters of many significant binding interactions and other areas of few significant binding interactions. These results reveal a number of structural relationships between different PDZ proteins and their ligands. The understanding of these structural relationships can be used to scan databases for probable ligands of specific PDZ proteins, facilitating the design of inhibitors of such novel interactions and the prediction of the side-effect profile of such inhibitors.

The most striking example of a cluster occurs in columns 1- 5 of the matrix (PDZ domains from CASK, MPP 1, DLG1, PSD95, and NeDLG) . In our assays, CASK bound significantly to only one of the tested ligands, neurexin (this interaction was previously identified in neuronal cells) . MPP1, the closest relative to CASK in the ordering system used, bound a set of five different ligands, including neurexin. All of the MPP1 ligands identified arc structurally similar in that they terminate in valine (V) and have a charged amino acid (E or K) at the- 3 position from the C- terminus ((C-terminal motif: E/K- X- X- V)

Out of the five MPP1 ligands identified, four of these also bind to DLG1, the next most similar protein to CASK and MPP1. DLG is closely related to two other proteins, PSD95 and NeDLG. Each of these three proteins binds ligands that terminate in the motif S/T/Y - X- V/I/L, as previously described (Songyang et al., 1997, Science 275, 73). Interestingly, if the terminal ligand residue is valine, other previously unidentified residues at the -2 position are compatibile with binding, with the C-terminal sequences AEV and ELV resulting in significant binding events. While this reflects a previously unrecognized flexibility in PDZ-ligand binding, the specificity of the interactions detected in our assays is reflected in the large areas at the top and bottom of columns 1 - 5 that contain no significant binding event. These areas reflect that none of the potential ligands terminating in residues other than V/I/L bound significantly to CASK, MPP1, DLG1, PSD95, or NeDLG.

(With respect to MPP1, it worth noting that Glycophorin C, a known ligand of MPP1, Marfatia et al., 1997, J. Biol. Chem. 272, 24191, did not bind significantly to MPP1 in our tests, but does terminate in isoleucine, an amino acids very similar to valine, and does have a charged residue at the- 3 position of the C- terminus.)

Other smaller clusters on the matrix also provide valuable information about the ligand specificity of certain PDZ domains. For example, five ligands bind significantly in both the "G" and the "A" assays to 41.8 kD protein, a previously unstudied PDZ domain-containing protein.

All of these ligands terminate in A, V, I, or L, and four of five have serine (S) at the- 2 position, defining the C-terminal ligand motif S- X- A/V/I/L for this PDZ domain. Similarly, two of three ligands that bind specifically to KIAA0561 fit the C- terminal motif X*- S/T- D/E- V/I/L where X* is any non- aromatic amino acid. Interestingly, one of these ligands also binds to KIAA 0561's closest relative, TAX2. Likewise, the two ligands that bind to prIL16 both have a charged amino acid at the- 3 position, a hydrophobic amino acid at the- 2 position, and valine at the terminal position. A final interesting example of defining the ligand specificity of a PDZ domain involves PTN- 4. Although the two ligands that bind significantly to PTN- 4 are not closely related at their C- terminal 2 residues, both fit the C- terminal motif D/E- S- X- V/I/L/F/Y. These motifs characterizing the ligand specificity of particular PDZ proteins are summarized in **TABLE 5B**. Such motifs allow searches of databases for novel ligands (PL proteins) binding to these particular PDZ proteins. Knowledge of the PL proteins that bind to these PDZ proteins allows the design of inhibitors of these interactions, using the methods described herein, infra. In addition, knowledge of the set of PL proteins that bind to a particular PDZ protein can be used to predict the utility and the side effects of compounds that target this PDZ protein.

**TABLE 5B**

| PDZ | -3 | -2 | -1 | 0 |
|---|---|---|---|---|
| 41.8 kD | X | X | X | A/V/I/L |

(continued)

| | | | | |
|---|---|---|---|---|
| KIAA 0561 | X* | S/T | D/E | V/I/L |
| TAX 2 | X | S | D/E | V |
| PRIL16 | D/E/K/R | V/I/L/F/Y | X | V |
| PTN4 | D/E | S | X | V/I/L/F/Y |

X* is any non-aromatic amino acid.

6.3 Measurement of PDZ-Ligand Binding Affinity

The "A" and "G" assays of the invention can be used to determine the "apparent affinity" of binding of a PDZ ligand peptide to a PDZ-domain polypeptide. Apparent affinity is determined based on the concentration of one molecule required to saturate the binding of a second molecule (e.g., the binding of a ligand to a receptor). Two particularly useful approaches for quantitation of apparent affinity of PDZ-ligand binding are provided *infra.*

(1) A GST/PDZ fusion protein, as well as GST alone as a negative control, are bound to a surface (e.g., a 96-well plate) and the surface blocked and washed as described supra for the "G" assay.

(2) 50 uL per well of a solution of biotinylated PL peptide (e.g. as shown in **TABLE 4**) is added to the surface in increasing concentrations in PBS/BSA (e.g. at 0.1 $\mu$M, 0.33 uM, 1 uM, 3.3 uM, 10 uM, 33 uM, and 100 uM). In one embodiment, the PL peptide is allowed to react with the bound GST/PDZ fusion protein (as well as the GST alone negative control) for 10 minutes at 4 C followed by 20 minutes at 25 C. The plate is washed 3 times with ice cold PBS to remove unbound labeled peptide.

(3) The binding of the PL peptide to the immobilized PDZ-domain polypeptide is detected as described supra for the "G" assay.

(4) For each concentration of peptide, the net binding signal is determined by subtracting the binding of the peptide to GST alone from the binding of the peptide to the GST/PDZ fusion protein. The net binding signal is then plotted as a function of ligand concentration and the plot is fit (e.g. by using the Kaleidagraph software package curve fitting algorithm) to the following equation, where "$Signal_{[ligand]}$" is the net binding signal at PL peptide concentration "[ligand] , " "Kd" is the apparent affinity of the binding event, and "Saturation

Binding" is a constant determined by the curve fitting algorithm to optimize the fit to the experimental data:

$$Signal_{[ligand]} = \text{Saturation Binding x } ([ligand] / ([ligand] + Kd))$$

For reliable application of the above equation it is necessary that the highest peptide ligand concentration successfully tested experimentally be greater than, or at least similar to, the calculated Kd (equivalently, the maximum observed binding should be similar to the calculated saturation binding). In cases where satisfying the above criteria proves difficult, an alternative approach (infra) can be used.

The results obtained when using approach 1 are demonstrated in **FIGURES 2A and 2B. FIGURE 2** shows varying concentrations of biotinylated CLASP-2 (**FIG. 2A**) or Fas (**FIG. 2B**). C-terminal peptides reacted with immobilized (plate bound) GST polypeptide or GST/PDZ fusion proteins (GST/DLG1, GST/NeDLG, and GDT/PSD95) in duplicate. The signals were normalized, plotted and fit to a saturation binding curve, yielding an apparent affinity of 21 uM for DLG1-CLASP-2 interaction, 7.5 uM for NeDLG-CLASP-2 interaction, 45 uM for PSD95-CLASP-2 interaction, and 54 uM for DLG1-Fas interaction, 54 uM for NeDLG-Fas interaction, and 85 uM for PSD95-Fas interaction.

Approach 2:

(1) A fixed concentration of a PDZ-domain polypeptide and increasing concentrations of a labeled PL peptide (labeled with, for example, biotin or fluorescein, see TABLE 4 for representative peptide amino acid sequences) are mixed together in solution and allowed to react. In one embodiment, preferred peptide concentrations are 0.1 $\mu$M, 1 uM, 10 uM, 100 uM, 1 mM. In various embodiments, appropriate reaction times can range from 10 minutes to 2 days at temperatures ranging from 4 C to 37 C. In some embodiments, the identical reaction can also be carried out using a non-PDZ domain-containing protein as a control (e.g., if the PDZ-domain polypeptide is fusion protein, the fusion partner can be used).

(2) PDZ-ligand complexes can be separated from unbound labeled peptide using a variety of methods known in the art. For example, the complexes can be separated using higb performance size-exclusion chromatography (HPSEC, gel filtration) (Rabinowitz et al., 1998, Immunity 9:699), affmity chromatography(e.g. using glutathione sepharose beads), and affinity absorption (e.g., by binding to an anti-GST-coated plate as described *supra).*

(3) The PDZ-ligand complex is detected based on presence of the label on the peptide ligand using a variety of methods and detectors known to one of skill in the art. For example, if the label is fluorescein and the separation is achieved using HPSEC, an in-line fluorescence detector can be used. The binding can also be detected as described supra for the G assay.

(4) The PDZ- ligand binding signal is plotted as a function of ligand concentration and the plot is fit. (e.g., by using the Kaleidagraph software package curve fitting algorithm) to the following equation, where "$\text{Signal}_{[\text{ligand}]}$" is the binding signal at PL peptide concentration "[ligand] , " "Kd" is the apparent affinity of the binding event, and "Saturation Binding" is a constant determined by the curve fitting algorithm to optimize the fit to the experimental data:

$$\text{Signal}_{[\text{Ligand}]} = \text{Saturation Binding x ([ligand] / ([ligand + Kd])}$$

Measurement of the affinity of a labeled peptide ligand binding to a PDZ-domain polypeptide n is useful because knowledge of the affinity (or apparent affinity) of this interaction allows rational design of inhibitors of the interaction with known potency (See **EXAMPLE 2**). The potency of inhibitors in inhibition would be similar to (i.e. within one- order of magnitude of) the apparent affinity of the labeled peptide ligand binding to the PDZ-domain.

6.4 <u>Assays to Identify Novel PDZ Domain Binding Moieties and to Identify Inhibitors of PDZ Protein-PL Protein Binding</u>

Although described *supra* primarily in terms of identifying interactions between PDZ-domain polypeptides and PL proteins, the assays described *supra* and other assays can also be used to identify the binding of other molecules (e.g., peptide mimetics, small molecules, and the like) to PDZ domain sequences. For example, using the assays disclosed herein, combinatorial and other libraries of compounds can be screened. Screening of libraries can be accomplished by any of a variety of commonly known methods. See, e.g., the following references, which disclose screening of peptide libraries: Parmley and Smith, 1989, Adv. Exp. Med. Biol. 251:215-218; Scott and Smith, 1990, Science 249: 386-390; Fowlkes et al., 1992; BioTechniques 13:422-427; Oldenburg et al., 1992, Proc. Natl. Acad. Sci. USA 89: 5393-5397; Yu et al., 1994, Cell 76:933-945; Staudt et al., 1988, Science 241:577-580; Bock et al., 1992, Nature 355: 564-566; Tuerk et al., 1992, Proc. Natl. Acad. Sci. USA 89:6988-6992; Ellington et al., 1992, Nature 355:850-852; U.S. Patent No. 5,096,815, U.S. Patent No. 5,223,409, and U.S. Patent No. 5,198,346, all to Ladner et al.; Rebar and Pabo, 1993, Science 263:671-673; and PCT Publication No. WO 94/18318.

In a specific aspect, screening can be carried out by contacting the library members with a hematopoietic cell PDZ-domain polypeptide immobilized on a solid support (e.g. as described *supra* in the "G" assay) and harvesting those library members that bind to the protein. Examples of such screening methods, termed "panning" techniques are described by way of example in Parmley and Smith, 1988, Gene 73:305-318; Fowlkes et al., 1992, BioTechniques 13:422-427; PCT Publication No. WO 94/18318; and in references cited hereinabove.

In another aspect, the two-hybrid system for selecting interacting proteins in yeast (Fields and Song, 1989, Nature 340:245-246; Chien et al., 1991, Proc. Natl. Acad. Sci. USA 88:9578-9582) can be used to identify molecules that specifically bind to a PDZ domain-containing protein. Furthermore, the identified molecules are further tested for their ability to inhibit transmembrane receptor interactions with a PDZ domain.

In one aspect, antagonists of an interaction between a PDZ protein and a PL protein are identified. In one embodiment, a modification of the "A" assay described *supra* is used to identify antagonists. In one embodiment, a modification of the "G" assay described *supra* is used to identify antagonists.

In one aspect, screening assays are used to detect molecules that specifically bind to PDZ domains in hematopoietic cells. Such molecules are useful as gonists or antagonists of PDZ-protein-mediated cell function (e.g., cell activation, e.g., T cell activation, vesicle transport, cytokine release, growth factors, transcriptional changes, cytoskeletin rearrangement, cell movement, chemotaxis, and the like). In one aspect, such assays are performed to screen for leukocyte activation inhibitors for drug development. The disclosure thus provides assays to detect molecules that specifically bind to PDZ domain containing proteins in hematopoietic cells. For example, recombinant cells expressing PDZ domain-encoding nucleic acids can be used to produce PDZ domains in these assays and to screen for molecules that bind to the domains. Molecules are contacted with the PDZ domain (or fragment thereof) under conditions conducive to binding, and then molecules that specifically bind to such domains are identified. Methods that can be used to carry out the foregoing are commonly known in the art.

By way of example, diversity libraries, such as random or combinatorial peptide or non-peptide libraries can be screened for molecules that specifically bind to PDZ domains in hematopoietic cells. Many libraries are known in the art that can be used, *e.g.*, chemically synthesized libraries, recombinant (*e.g.*, phage display libraries), and *in vitro* translation-based libraries.

Examples of chemically synthesized libraries are described in Fodor et al., 1991, Science 251: 767- 773; Houghten et al., 1991, Nature 354: 84- 86; Lam et al., 1991, Nature 354: 82- 84; Medynski, 1994, Bio/ Technology 12: 709- 710; Gallop et al., 1994, J. Medicinal Chemistry 37 (9) : 1233- 1251; Ohlmeyer et al., 1993, Proc. Natl. Acad. Sci. USA 90: 10922- 10926; Erb et al., 1994, Proc. Natl. Acad. Sci. USA 91: 11422- 11426; Houghten et al., 1992, Biotechniques 13: 412; Jayawickreme et al., 1994, Proc. Natl. Acad. Sci. USA 91: 1614- 1618; Salmon et al., 1993, Proc. Natl. Acad. Sci. USA 90: 11708- 11712; PCT Publication No. WO 93/20242; and Brenner and Lerner, 1992, Proc. Natl. Acad. Sci. USA 89: 5381- 5383.

Examples of phage display libraries are described in Scott and Smith, 1990, Science 249:386-390; Devlin et al., 1990, Science, 249:404-406; Christian, R.B., et al., 1992, J. Mol. Biol. 227:711-718); Lenstra, 1992, J. Immunol. Meth. 152:149-157; Kay et al., 1993, Gene 128:59-65; and PCT Publication No. WO 94/18318 dated August 18, 1994.

*In vitro* translation-based libraries include but are not limited to those described in PCT Publication No. WO 91/05058 dated April 18, 1991; and Mattheakis et al., 1994, Proc. Natl. Acad. Sci. USA 91:9022-9026.

By way of examples of nonpeptide libraries, a benzodiazepine library (*see e.g.,* Bunin et al., 1994, Proc. Natl. Acad. Sci. USA 91:4708-4712) can be adapted for use. Peptoid libraries (Simon et al., 1992, Proc. Natl. Acad. Sci. USA 89:9367-9371) can also be used. Another example of a library that can be used, in which the amide functionalities in peptides have been permethylated to generate a chemically transformed combinatorial library, is described by Ostresh et al. (1994, Proc. Natl. Acad. Sci. USA 91:11138-11142).

It will be appreciated by the ordinarily skilled practitioner that, in one aspect, antagonists are identified by conducting the A or G assays in the presence and absence of a known or candidate antagonist. When decreased binding is observed in the presence of a compound, that compound is identified as an antagonist. Increased binding in the presence of a compound signifies that the compound is an agonist.

For example, in one assay, a test compound can be identified as an inhibitor (antagonist) of binding between a PDZ protein and a PL protein by contacting a PDZ domain polypeptide and a PL peptide in the presence and absence of the test compound, under conditions in which they would (but for the presence of the test compound) form a complex, and detecting the formation of the complex in the presence and absence of the test compound. It will be appreciated that less complex formation in the presence of the test compound than in the absence of the compound indicates that the test compound is an inhibitor of a PDZ protein -PL protein binding. In various embodiments, the PL peptide comprises an amino acid sequence substantially identical to the C-terminal sequence of a PL protein (e.g., CD6, CD49E, CD49F, CD138, Clasp-1, Clasp-4, VCAM1, Clasp-2, CD95, DNAM-1, CD83, CD44, CD4, CD97, Neurexin, CD3n, DOCK2, CD34, FceRIb, or FasLigand).

In one aspect, the "G" assay is used in the presence or absence of an candidate inhibitor. In one embodiment, the "A" assay is used in the presense or absence of a candiate inhibitor.

In one aspect (in which a G assay is used), one or more PDZ domain containing GST-fusion proteins are bound to the surface of wells of a 96-well plate as described *supra* (with appropriate controls including nonfusion GST protein). All fusion proteins are bound in multiple wells so that appropriate controls and statistical analysis can be done. A test compound in BSA/PBS (typically at multiple different concentrations) is added to wells. Immediately thereafter, 30 uL of a detectably labeled (e.g., biotinylated) peptide known to bind to the relevant PDZ domain (see, e.g., **TABLE 2**) is added in each of the wells at a final concentration of, e.g., between about 2 uM and about 40 uM, typically 5 uM, 15 uM, or 25 uM. This mixture is then allowed to react with the PDZ fusion protein bound to the surface for 10 minutes at 4°C followed by 20 minutes at 25°C. The surface is washed free of unbound peptide three times with ice cold PBS and the amount of binding of the peptide in the presence and absence of the test compound is determined. Usually, the level of binding is measured for each set of replica wells (e.g. duplicates) by subtracting the mean GST alone background from the mean of the raw measurement of peptide binding in these wells.

In an alternative aspect, the A assay is carried out in the presence or absence of an test candidate to identify inhibitors of PL-PDZ interactions.

In one aspect, a test compound is determined to be a specific inhibitor of the binding of the PDZ domain (P) and a PL (L) sequence when, at a test compound concentration of less than or equal to 1 mM (e.g., less than or equal to: 500 uM, 100 uM, 10 uM, 1 uM, 100 nM or 1 nM) the binding of P to L in the presence of the test compound less than about 50% of the binding in the absence of the test compound. (in various embodiments, less than about 25%, less than about 10%, or less than about 1%). Preferably, the net signal of binding of P to L in the presence of the test compound plus six (6) times the standard error of the signal in the presence of the test compound is less than the binding signal in the absence of the test compound.

In one aspect, assays for an inhibitor are carried out using a single PDZ protein-PL protein pair (e.g., a PDZ domain fusion protein and a PL peptide). In a related embodiment, the assays are carried out using a plurality of pairs, such as a plurality of different pairs listed in **TABLE 2.** pairs listed in TABLE 2 In some aspects, it is desirable to identify compounds that, at a given concentration, inhibit the binding of one PL-PDZ pair, but do not inhibit (or inhibit to a lesser degree) the binding of a specified second PL-PDZ pair. These antagonists can be identified by carrying out a series of assays using a candidate inhibitor and different PL-PDZ pairs (e.g., as shown in the matrix of **TABLE 2**) and comparing the results of the assays. All such pairwise combinations are contemplated (e.g., test compound inhibits binding of $PL_1$ to PDZ, to a greater degree than it inhibits binding of $PL_1$ to $PDZ_2$ or $PL_2$ to $PDZ_2$). Importantly, it will be appreciated that, based on the data provided in **TABLE 2** and disclosed herein (and additional data that can be generated using the methods described herein) inhibitors with different specificities can readily be designed.

The PDZ binding moieties and PDZ protein -PL protein binding antagonists of the invention are used to modulate biological activities or fuctions of cells (e.g., hematopoietic cells, such as T cells and B cells and the like), endothelial cells, and other immune system cells, as described herein, and for treatment of diseases and conditions in human and nonhuman animals (e.g., experimental models). Exemplary biological acitivities are listed *supra.*

When administered to patients, the compounds (e.g., PL- PDZ interaction inhibitors) are useful for treating (ameliorating symptoms of) a variety of diseases and conditions, including diseases characterized by inflammatory and humoral immune responses, e.g., inflammation, allergy (e.g., systemic anaphylaxis, hypersensitivity responses, drug allergies, insect sting allergies; inflammatory bowel diseases, ulcerative colitis, ileitis and enteritis; psoriasis and inflammatory dermatoses, scleroderma; respiratory allergic diseases such as asthma, allergic rhinitis, hypersensitivity lung diseases, and the like vasculitis, rh incompatibility, transfusion reactions, drug sensitivities, PIH, atopic dermatitis, eczema, rhinnitis; autoimmune diseases, such as arthritis (rheumatoid and psoriatic) , multiple sclerosis, systemic lupus erythematosus, insulin- dependent diabetes, glomerulonephritis, scleroderma, MCTD, IDDM, Hashimoto thyroiditis, Goodpasture syndrome, psoriasis and the like, osteoarthritis, polyarthritis, graft rejection (e.g., allograft rejection, e.g., renal allograft rejection, graft- vs- host disease, transplantation rejection (cardiac, kidney, lung, liver, small bowel, cornea, pancreas, cadaver, autologous, bone marrow, xenotransplantation) ) , atherosclerosis, angiogenesis- dependent disorders, cancers (e.g., melanomas and breast cancer, prostrate cancer, leukemias, lymphomas, metastatic disease) , infectious diseases (e.g., viral infection, such as HIV, measles, parainfluenza, virus- mediated cell fusion, ) , ischemia (e.g., post- myocardial infarction complications, joint injury, kidney, scleroderma) .

The PL proteins and PDZ proteins listed in **TABLE 2** are well characterized, and one of skill, guided by this disclosure (including the discovery.of the interactions between PL proteins and PDZ proteins described herein), will recognize many uses for modulators (e.g., enhancers or inhibitors) of PDZ-PL interactions such as those described in **TABLE 2**. To further assist the reader, a discussion of the characteristics of selected PL proteins (and their function) is provided *infra.* It will be recognized that this discussion is not comprehensive and is not intended to limit the invention in any way. Moreover, nothing in this section should be construed as an intention by the inventors to be limited to a particular mechanism of action.

### A. CD6

As shown *supra,* CD6 binds PDZ protein 41.8. CD6 is expressed on thymocytes, T cells, and B cell chronic lymphocytic leukemias. CD6 plays a role in T cell co-stimulation and CD6 negative T cells are less autoreactive than CD6 positive T cells. Inhibition of CD6 and CD6/41.8 interactions is predicted to reduce the symptoms of graft-versus-host disease (GVHD) or psoriasis. Thus, in one aspect of the disclosure, GVHD is reduced in a patient receiving donor bone marrow cells by pre-treating the cells with an effective amount of an antagonist. In combination with post-transplantation immunosuppressive therapy such as FK506, Cellcept, or cyclosporin, CD6-PDZ interaction inhibitors will improve overall survival of transplantation patients (e.g., leukemia patients).

### B. CD49e (ALPHA-4)

As shown by the experiments reported herein, the C-terminal end of CD49e binds to the PDZ-domain-containing protein41.8kD. CD49e is a 110 kD transmembrane membrane protein of the integrin alpha family (integrin alpha 5). Paired with the integrin beta-1 subunit it forms VLA-5. VLA-5 is expressed predominantly on hematopoietic and lymphoid lineage cells including monocytes, basophils, T cells, and activated B cells. VLA-5 is the receptor for the ubiquitously-expressed adhesion molecule fibronectin. Tissue injury such as myocardial infarction releases soluble fragments of fibronectin. Binding of these soluble fragments to VLA-5 results in chemotaxis of immune cells including monocytes to the source of fibronectin, as well as down-modulation of VLA-5 expression on these cells. Such ligand-induced down-modulation is a common and required feature of chemotaxic receptors. Once immune cells migrate fully to the source of fibronectin, adhesion to the fibronectin surface is enhanced by fibronectin-VLA-5 interaction. Without intending to be bound by a particular mechanism, the 41.8/CD49e interaction is believed to be necessary for proper membrane distribution of CD49e and/or recycling of CD49e such that when it is disrupted, the migration and adherence to fibronectin -containing surfaces is similarly disrupted, resulting in an inability of immune system cells to effectively migrate toward a fibronectin source and adhere to fibronectin-containing surfaces. Such disruption would therefore result in desirable reduced inflammatory processes, including reduced post-myocardial infarction inflammation. Other diseases to be treated include but are not limited to joint inflamation, psoriasis, contact allergy, Crohn's Disease, inflammatory bowel disease, eczema, atopic dermatitis.

**C. CD49F (VLA-6 $\alpha$ subunit)**

As shown *supra,* CD49F binds PDZ protein 41.8. CD49f is known as an integrin subunit that pairs either with the $\beta$1 integrin subunit (CD29), forming VLA-6, or with CD104 ($\beta$4 integrin subunit). The integrin supergene family consists of a number of cell surface $\alpha\beta$ heterodimers important for many different physiologic processes, including embryogenesis, thrombosis, wound healing, tumorigenesis and immune responses. Each $\beta$ chain can pair with various $\alpha$ chains. Both VLA-6 and CD49f/CD104 are widely expressed on epithelia in non-lymphoid tissues. VLA-6 is also expressed on platelets, monocytes, thymocytes and T lymphocytes, with an increased expression on activated and resting memory T cells.

Inhibition of interactions between VLA-6 and 41.8 has a number of therapeutic functions such as the prevention and treatment of metastatic cancers, and treatment of overactive immunity. For example, VLA-6 is associated with invasivion of prostrate carcinoma and plays a role in the metastasis of breast cancer. Blockage of VLA-6 function combined with conventional treatment for prostrate cancer, would be a more effective treatment by preventing metastatic disease (see, Cress et al., 1995, Cancer Metastasis R). Blockage of CD49f through PDZ interaction may also treat Rh incompatibility by blunting memory response or in the treatment of keloids.

**D. CD138** (syndecan-1)

CD138 is a transmembrane proteoglycan receptor with the extracellular domain functioning as a ligand binding domain for various extracellular matrix components and the intracellular portion functioning to alter cytoskeleton and transduce intracellular signals. CD138 also binds FGF2 and may be a co-receptor for FGF receptor (Yayon et al., 1991).

As shown *supra,* CD138 interacts with 41.8kD protein and TIAM1. The c-terminus of CD138 has also been reported to bind the PDZ domains of syntenin and human CASK (Cohen et al., 1998, J. Cell. Biol. 142:129-138; Grootjans et al., 1997, PNAS 94:13683-13688; Hsueh et al., 1998, J. Cell. Biol. 142:139-151).CD138 is expressed in pre-B cells immature B cells, plasma cells, neural cells, the basolateral surface of epithelial cells, embryonic mesenchymal cells, vascular smooth muscle cells, endothelial cells and neural cells but not mature circulating B cells. The interaction between CD138 and the PDZ domains of the 41.8kD protein and TIAM1 proteins is believed to be necessary for the proper distribution of CD138 on the cell surface. Disruption of the interaction by administration of an effective amount of an antagonist is expected to interfere with the migration and adherence of cells to the extracellular matrix, resulting in reduced inflammatory and humoral immune responses. Inhibition of CD138 may be used to treat without limitation diseases such as post-myocardial infarction inflamatory damage, joint injury, rheumatoid arthritis, vasculitis, drug reaction, scleraderma, SLE, Hashimoto thyroiditis, Goodpasture's syndrome, juvenile insulin-dependent diabetes, psoriasis.

**E. CD98**

As shown *supra,* CD98 interacts with MPP2. CD98 is expressed at high levels on monocytes and at low levels on T cells, B cells, splenocytes, NK cells, and granulocytes. CD98 plays roles in adhesion, fusion and is a L-type amino acid transporter. CD98 is also involved in virus-mediated cell fusion (e.g. paramyoviruses: parainfluenza virus type 2, Newcastle disease virus, and rubulaviruses) and antagonism of CD98 function is expected to treat )viral infections and limit viral spread. CD98 inhibitors can be an antiviral agent for, but not limited to paramyovirus, parainfluenza, Newcastle disease and rubula. Other roles include treatment for acute leukemias.

**F. CLASP-1**

As shown supra, CLASP-1 interacts with DLG1, PSD95, and NeDLG. CLASP-1 is a member of a superfamily of immune-cell associated proteins with similar motifs (see PCT/US99/22996 published as WO 00/20434). CLAP-1 functions in the maintenance of the immune synapse. The CLASP-1 transcript is present in lymphoid organs and neural tissue, and the protein is expressed by T and B lymphocytes and macrophages in the MOMA-1 subregion of the marginal zone of the spleen, an area known to be important in T:B cell interaction. CLASP-1 staining of individual T and B cells exhibits a preactivation structural polarity, being organized as a "ball" or "cap" structure in B cells, and forming a "ring", "ball" or "cap" structure in T cells. The placement of these structures is adjacent to the microtubule-organizing center ("MTOC"). CLASP-1 antibody staining indicates that CLASP-1 is at the interface of T-B cell conjugates that are fully committed to differentiation. Antibodies to the extracellular domain of CLASP-1 also block T-B cell conjugate formation and T cell activation.

Antagonism of CLASP-1 function is expected to interfere with immune responses (e.g., T and B cell activation), signal transduction, cell-cell interactions, and membrane organization. Diseases to be treatment by CLASP-1 agonists/ antagonists include, but is not limited to, rheumaotoid arthritis, juvenile diabetes, organ rejection, graft-versus-host disease, scleroderma, multiple sclerosis.

**G. CLASP-4**

As shown supra, CLASP-4 interacts with DLG1, PSD95, NeDLG, LDP, AF6, 41.8, and MINT1. CLASP-4 is a member of a superfamily of immune-cell associated proteins with similar motifs (see copending U.S.Pat. Application 60/196,527 filed April 11, 2000). The CLASP-4 protein is expressed primarily in peripheral blood lymphocytes. Inhibition of the interaction of CLASP-4 and PDZ domains will interfere with immune responses (e.g., T and B cell activation), signal transduction, cell-cell interactions, and membrane organization. Diesease to be treated bu CLASP-4 agonists/ antagonists include, but is not limited to, rheumaotoid arthritis, juvenile diabetes, organ rejection, graft-versus-host disease, scleroderma, multiple sclerosis, acute leukemias, leukemic blast crisis, post-infarction inflamation (cardiac, etc.), atherosclerosis.

**H. VCAM1**

The vascular cell adhesion molecule-1 (VCAM-1, CD106) is predominantly expressed by vascular endothelium (i.e.,, endothelial cells) but has been detected in macrophages, dendritic cells, bone marrow-derived cells, fibroblasts, cortical thymic epithelial cells, vascular smooth muscle cells, myoblasts and myotubes. VCAM-1 mediates adhesion through interacting with an integrin ligand, VLA-4, which is expressed by lymphocytes, monocytes and eosinophils. The interaction between VCAM-1 and VLA-4 is important for activation, flattening and extravasation of VLA-4 expressing cells when the endothelium itself has become activated due to inflammation or injury (Salomon et al., 1997, Blood 89: 2461-2471; St-Pierre et al., 1996, Eur. J. Immunol. 26:2050-2055; Bell et al., 1995, Int. Immunol. 7:1861-1871).

As discussed *supra,* the C-terminal region of VCAM-1 is a ligand for the PDZ domains of MPP1, DLG1, NeDLG1, LDP, 41.8 protein, TIAM1 and K303. These interactions are believed to mediate the function of endothelial cell interactions with integrin expressing leukocytes. When the PDZ-PL interactions are disrupted, the adherence of leukocytes to the endothelium will similarly be disrupted, resulting in, e.g., reduction of inflammation. Thus, inhibition of VCAM-1 binding to PDZ proteins is useful for reducing abnormal VCAM-1 inflammatory responses and associated pathologies such as (but not limited to) renal allograft rejection, insulin-dependent diabetes, rheumatoid arthritis, post-myocardial infarction complications and systemic lupus erythematosus (Pasloske et al., 1994, Ann Rev Med 45:283; Ockenhouse et al., 1992, J. Exp. Med. 176:1183; Solezk et al., 1997, Kidney Int. 51:1476; Tedla, et al., 1999, Clin. Exp. Immunol. 117:92-99; Kusterer et al., 1999, Exp Clin Endocrinol Diabetes 107:S102-107; Bonomini et al., 1998, Nephron 79:399; Suassuna et al., 1994, Kidney Int. 46:443; Ferri et al., 1999, Hypertension 34:568).

**I. CLASP-2**

As shown *supra,* CLASP-2 interacts with PSD-95, NeDLG, and DLG1. CLASP-2 is a member of a superfamily of immune-cell associated proteins with similar motifs (see copending U.S. Pat. 09/547,276 filed April 11, 2000; WO 00/10158 filed April 11, 2000; WO 00/10156 filed April 11, 2000). The CLASP-2 transcript is present most strongly in placenta followed by lung, kidney and heart and the protein is expressed in T and B cells, and kidney epithelial cells.

Inhibition of the interaction of CLASP-2 and PDZ domains will interfere with CLASP-2 function resulting in interference with T and B cell function (e.g., T and B cell activation), signal transduction, cell-cell interactions, and membrane organization. In addition, since CLASP-2 is present in heart, blocking CLASP-2 function or expression can selectively block immune responses in the heart (for example, to selectively stop immune response in the heart compartment, *e.g*., following cardiac transplant rejection or post-MI inflammation, without compromising immunity elsewhere). Other diseases to be treatment by CLASP-1 agonists/antagonists include, but is not limited to, rheumaotoid arthritis, juvenile diabetes, organ rejection, graft-versus-host disease, scleroderma, multiple sclerosis.

**J. CD95 (Apo-1/Fas)**

CD95 (Fas/Apo- 1) and Fas ligand (FasL) are a receptor- ligand pair involved in lymphocyte homeostasis and peripheral tolerance. Binding of Fas by its ligand results in apoptotic cell death, an important major mechanism for safe clearance of unwanted cell during resolution of the acute inflammatory response. As is shown *supra,* CD95 binds the PDZ domains DLG1, PSD95, NeDLG, and 41.8. Inhibition of the interaction of CD95 and PDZ domains.

**K. KV1.3 (Shaker Type Kv1.3 Potassium Channel)**

As shown supra, Kv1.3 binds DLG1, PSD95, NeDLG, LIMK, 41.8, RGS12, DVL1, and MINT1. Kv1.3 is a Shaker-related channel protein that is involved in modulating the membrane potential of T lymphocytes (Lewis and Cahalan, 1995, Ann. Rev. Immunol. 13:623). Inhibition of the Kv1.3 channel chronically depolarizes the T cell membrane, reduces calcium entry via calcium-activated release calcium channels in the plasma membrane, and consequently inhibits the calcium-signaling pathway essential for lymphocyte activation. Hanada et al., reported that Kv1.3 is associated with DLG1 and PSD95 in Jurkat T cells (J. Biol. Chem. 1997, 272:26899). Administration of Kv1.3-PDZ protein agonist/antagonists will disrupt T cell signaling and can be a useful therapeutic drug to treat, but not limited to, organ transplantation, graft-versus-host disease, Crohn's Disease, Ulcerative colitis, inflammatory bowel disease, rheumatoid arthritis, osteoarthritis, multiple sclerosis, scleroderma, mixed connective tissue disease.

**L. DNAM-1**

As shown *supra,* DNAM-1 binds several PDZ proteins, including MPP2, DLG1, PSD95, NeDLG, LIM, AF6, 41.8 and RGS12. DNAM-1 is associated with Fyn constitutively but required the presence of pervanadate (a tyrosine phosphatase inhibitor) (see Shibuya, et al. 1999, Immunity. 11:615-623). Upon stimulation with anti-CD3 (or DNAM-1), DNAM-1 is phosphorylated at Serine 329 (Shibuya, et al. 1998, J. Immun. 161:1671-1676.) and associates with LFA-1. Furthermore, Fyn becomes associated with DNAM-1 independent ofpervanadate. Fyn phosphorylates DNAM-1 at Y 322, but does not require Y322 to continue binding to DNAM-1.

Since DNAM-1 itself does not have a SH3 binding domain but only has the Src phosphorylation site at Y322, an adaptor molecule must be present to bridge DNAM-1 and Fyn. DLG1 has been described in the literature to be present in T cells (Hanada, et al. 1997. J Biol Chem 272:26899), but does not bind Fyn. PSD95 does not have a SH3 binding site. However, several of the other PDZ proteins do have SH3 binding domains including but not limited to NeDLG, RGS12, MPP2 and can fulfill this function. These adaptor PDZ listed *supra* binds to DNAM-1 through its PDZ domain and simultaneously binds to the SH3 domain of Fyn through its proline-rich sequences just N-terminal to the PDZ domain. The Y139 is a candidate phosphorylation site to control association of Fyn to DNAM-1 and the adaptor PDZ.

Based on this analysis, inhibition of PDZ association with DNAM- 1 using the reagents described herein will inhibit Fyn association with DNAM- 1 and the subsequent Y322 phosphorylation and activation of cytotoxic T cells. Diseases that can be treated include but are not limited to Crohn's Disease, multiple sclerosis, ulcerative colitis, inflammatory bowel disease, graft- versus- host, juvenile diabetes, Hashimoto's disease.

**M. CD83 (HB15)**

CD83 is a transmembrane glycoprotein, expressed predominantly on activated dendritic cells (DCs), Langerhans cells in the skin, with some weak expression detected on activated peripheral lymphocytes, and interdigitating reticulum cells within the T cell zones of lymphoid organs (Zhou and Tedder, 1995, J. Immunol. 154:3821-3835; Zhou et al., 1992, J. Immunol. 149:735-742). CD83 is up-regulated de novo upon activation of an immature DCs, and is the major discriminating marker and characteristic for activated, mature DCs (Czerniecki et al., 1997, J. Immunol. 159: 3823-3837). DCs function as antigen presenting cells (APCs). Upregulation and expression of CD83 thus appears to be required for DCs to mature and function as APCs.

As shown by experiments described *supra,* the CD83 binds to the PDZ domains of DLG1, PSD95, and NeDLG. These interactions between CD83 and PDZ domains, and between CD83 and DLG1, PSD95, and NeDLG are believed to be important for proper distribution and recycling of CD83. Disruption of CD83 and PDZ proteins with agonists and antagonist can be used to treat, but not limited to, psoriasis, cancers, allergies, autoimmune diseases such as multiple sclerosis, system lupus erythematosis.

**N. CD44** (phagocytic glycoprotein 1, lymphocyte homing receptor, p85 and HCAM)

CD44 is single pass transmembrane protein that has several different isoforms due to alternative splicing. It has a broad pattern of expression being detected on both hematopoietic and non-hematopoietic cell types including epithelial, endothelial, mesenchymal and neuronal cells. CD44H is a major isoform that is expressed in lymphoid, myeloid and erythroid cells (reviewed in Barclay et al., 1997, The Leukocyte Antigen Facts Book, 2ed, Academic Press). CD44 is a receptor for hyaluronate (HA), which is a constituent of the extracellular matrix (ECM). In the immune system, CD44 functions as an adhesion molecule on the surface of leukocytes and erythrocytes that binds HA polymers in the ECM, and it can also act as a signaling receptor when HA becomes soluble during inflammatory reactions or tissue damage. The cytoplasmic region of CD44 has been shown to bind or be associated with the actin cytoskeleton through interactions with spectrin and members of the ERM (ezrin, radixin, and meosin) family (reviewed in Lesley et al., 1993; Bajorath, 2000, Proteins 39:103-111). Additionally, CD44 is associated with the non-receptor tyrosine kinase p56Lck (Taher et al., 1996, J. Biol. Chem. 271:2863-2867. CD44 has been shown to be a co-stimulatory molecule with CD3/TCR engagement to activate T cells (reviewed in Aruffo, 1996, J. Clin. Invest. 98:2191-2192).

As described *supra* by experiments reported herein, the C-terminus of CD44 is a ligand for the PDZ domain contained in MPP1, prIL-16 and MINT1. It is believed that the interactions of CD44 with PDZ domains, and between CD44 with MPP1, prIL-16 and MINT1 function in maintenance of leukocyte structure and in leukocyte signaling. Thus, when a CD44-PDZ interaction is disrupted, CD44 will fail to transduce proper intracellular signals, and maintain proper distribution of CD44 on the surface, which will prevent adhesion of leukocytes to the endothelium during inflammation and tissue damage. Administration of agonists/antagonists of this interaction will thus result in, but not limited to, reduced inflammatory responses during tissue ischemia and cell lysis (e.g., rhabdomyosis), vascular insufficiencies (e.g. frostbite), psoriasis, eczema, graft-versus-host disease, granuloma annulare, scleroderma.

**O. CD97 (CD55)**

As discussed *supra,* CD97 binds the PDZ domains of DLG1 and 41.8. CD97 is a 79.7 kD seven-span transmembrane protein expressed on granulocytes and monocytes and at low levels on resting T cells and B cells. Upon T or B cell activation expression levels of CD97 in T cells and B cells increases rapidly (Eichler et al., 1994, Scand. J Immunol. 39, 111-115; Pickl et al., 1995, Leukocyte Typing V:1151-1153). When expressed on COS cells, CD97 confers adhesion to lymphocytes and to erythrocytes.

According to the present disclosure, the interaction of CD97 with DLG1 and the 41.8 kd protein can be altered to interfere with proper membrane distribution of CD97 and/or recycling of CD97. Such modulation will affect CD97 dependent adherence of cells with therapeutic benefit. Without being limited, agonists and antagonists of CD97-PDZ protein interaction can be used to treat rheumatoid arthritis, osteoarthritis, Crohn's Disease, Ulcerative colitis, psoriasis.

**P. Glycophorin C (GC)**

As is shown *supra,* the c-terminus of Glycophorin C (GC) interacts with the PDZ domains of human DLG, PSD95, NeDLG, MMP2, AF6, 41.8, and MINT 1 (with Mint-1 described previously). Glycophorin C is an integral membrane protein expressed in erythroid cells, thymus, stomach, breast, adult and fetal liver, monocytes, T and B cells (Le Van Kim et al., 1989, J. Biol. Chem. 264:20407-20414) and is known for its role in human erythrocytes where it interacts with MPP1 and protein 4.1 to regulate the shape, integrity and mechanical stability of red cells (Marfatia et al., 1997, J. Biol. Chem. 272:24191-24197; Reid et al., 1987, Blood 69:1068-1072.).

Interactions between Glycophorin C and PDZ proteins DLG, PSD95, NeDLG, MMP2, AF6, 41.8, and MINT1 are believed necessary for maintenance of the physical integrity of cells in which they are expressed. Modulation of GC-PDZ interactions will alter with the function of these and can be utilized to treat, but not limited to, polycythemia vera, spherocytosis.

Q. CDw128A **(IL8RA)**

As is described *supra,* CDW128A binds to the PDZ domains of DLG1 and NeDLG. There are two forms for the IL- 8 receptor, IL- 8RA (CDw128A) and IL- 8RB (CDw128B) both of which are members of the G protein- coupled receptor superfamily and chemokine receptor branch of rhodopsin family. CDw128A and CDw128B both bind IL- 8 with the same affinity but only CDw128B, binds three other IL- 8- related CXC chemokines: melanoma growth- stimulating activity (GRO/ MGSA) , neutrophil- activating peptide 2 (NAP- 2) and ENA- 78. See, e.g., Ahuja, SK. And Murphy, PM. 1996. J Biol Chem 271: 20545- 50.

CDw128A is expressed on all granulocytes, a subset of T cells, monocytes, endothelial cells, keratinocytes, erythrocytes, and melanoma cells. IL-8 induces chemotaxis of neutrophils, basophils, and T lymphocytes and increases neutrophil and monocyte adhesion to endothelial cells. The binding of IL-8 to IL8RA induces a transient increase in intracellular calcium levels, activation of phospholipase D, a respiratory burst of neutrophils and chemotaxis. This pro-inflammatory response is effective in normal immune responses. Inhibitors of CDw128A are useful for treatment of psoriasis, rheumatoid arthritis, polyarthritis, and for control of angiogenesis-dependent disorders such as melanomas and breast cancer.

**(R) CD3-eta($\eta$)**

CD3- $\eta$ is a splice variant of CD3 zeta and a component of the CD3/TCR complex, which is required for antigen recognition, signal transduction and activation of T cells (Weiss and Littman, 1994, Cell 76: 263- 274.) . See, Barclay et al., 1997, The Leucocyte antigen facts book, 2nd Ed, Academic Press. As shown by experiments reported herein, the C- terminal region of CD3- η is a ligand for the PDZ domains of MINT1, 41.8 protein, DLG1, and PSD95. The interactions of CD3- η with PDZ domains, and of CD3- η with MINT1, 41.8 protein, DLG1, and PSD95 are believed to be important activation of T cells, which is required for all cellular immune responses. Modulation of this interaction by agonists and antogonists can be used to treat, but is not limited to, acute and chronic allograft rejection, multiple sclerosis, graft-versus- host disease, rheumatoid arthritis.

**(S) LPAP (CD45-AP, LSM-1)**

LPAP is a transmembrane protein expressed on resting and activated T- and B-cells. LPAP has been shown to bind to CD45, a protein that is part of the T-cell receptor complex and has been found to co-localize with CD4, CD2 and Thy-1. LPAP has also been co-immune precipitated with p56(lck) and ZAP-70. The actual function of LPAP is unknown, but it has been suggested that it is an assembly molecule for the CD45 complex.

As shown *supra,* LPAP binds to DLG-1 and MINT-1. Notably, DLG-1 and MINT-1 are both expressed in T-cells. It has also been shown that DLG-1 co-precipitates with p56(lck) in T-cells. The assays described herein also demonstrated that DLG-1 binds to CD95 and KV1.3, and binding of MINT-1 to KV1.3. All of these molecules are involved in signaling by the TCR. LPAP is believed to function in organizing the signaling by CD45 in T-cell activation, possibly by recruiting p56(lck) as a substrate for CD45. Blocking the function of CD45 has been shown to severely impair the T-cell response. Inhibiting the interaction between LPAP and PDZ proteins is expected to alter the CD45-mediated path from the rest of the immune response. Agonists and antagonists of PL - PDZ binding can be used to treat, but is not limited to, rheumatoid arthritis, transplant rejection, multiple sclerosis, scleroderma, graft-versus-host disease.

**(T) CD46** (Complement membrane cofactor protein (MCP))

CD46 is a membrane protein expressed on all nucleated cells, but not on erythrocytes. CD46 is a member of the regulator of complement activation protein family. Its primary function is the protection of cells from complement attack by inactivating membrane deposited C3b/C4b complement (Liszewski, et al., 1999, Adv. Immunol. 61:201-283). CD46 exists in more than 8 isoforms that are generated by differential splicing, with molecular weights ranging from 45 to 70 kD. In addition to the above function, CD46 also serves as the receptor for the measles virus and for other pathogenic microorganisms (e.g. Streptococcus pyogenes) (Manchester et al, 1994, Proc. Natl Acad. Sci. USA 91:2161; Okada et al., 1995, Proc. Natl Acad. Sci. USA 92:2489-2493.). CD46 also appears to be over-expressed on certain tumors (Jurianz et al., 1999, Mol Immunol 36:929-939) thus rendering tumor cells insensitive to the action of complement. See, Barclay et al., (1997) The Leucocyte antigen facts book, 2ed, Academic Press.

As shown *supra,* CD46 binds DLG1, PSD95 and Ne-DLG. This interaction is believed to be necessary for proper membrane distribution of CD46 and/or recycling of CD46. Alteration of the CD46-PDZ interaction can reduce the ability of measles virus and other pathogens to enter cells, renders CD46-expressing tumors susceptible to attack by complement. The administration of CD46-PDZ interaction agonists and antagonists is useful for the treatment of , but not limited to, cancers and viral infectious diseases.

**(U) CDw128B**

As is described *supra,* CDw 128B binds to the PDZ domains of DLG1, NeDLG, PSD95, and 41.8 in the assays described *supra.* There are two forms for the IL- 8 receptor, IL- 8RA (CDw128A) and IL- 8RB (CDw128B) both of which are members of the G protein- coupled receptor superfamily and chemokine receptor branch of rhodopsin family. CDw 128A and CDw128B both binds IL- 8 with equal affinity but only CDw128B, also binds three other IL- 8- related CXC chemokines: melanoma growth- stimulating activity (GRO/ MGSA) , neutrophil- activating peptide 2 (NAP- 2) and ENA-78. See, e.g., Ahuja, SK and Murphy, PM. 1996. J Biol Chem 271: 20545- 50.

CDw128B is expressed on all granulocytes, a subset of T cells, monocytes, endothelial cells, keratinocytes, erythrocytes, and melanoma cells. IL-8 induces chemotaxis of neutrophils, basophils, and T lymphocytes but diminished relative to IL8RA and increases neutrophil and monocyte adhesion to endothelial cells. The binding of IL-8A to its receptor induces a transient increase in intracellular calcium levelsand granule release but does not induce activation of phospholipase D or a respiratory burst in neutrophils. This pro-inflammatory response is effective in normal immune responses. Inhibitors of CDw128B are useful for treatment of psoriasis, rheumatoid arthritis, polyarthritis, and for control of angiogenesis-dependent disorders such as melanomas and breast cancer.

**(V) DOCK2**

The DOCK family are a group of morphogenetic transmembrane proteins that interacts with cytoskeleton to affect cell shape changes. Members of this new family include Drosophila myoblast city (mbc), DOCK180, DOCK2, DOCK3, CED5, KIAA0209 and CLASP. The prototypical molecule, DOCK1 or DOCK180 is the human homolog of C. elegans gene, CED5 which is involved in the engulfment and phagacytosis by macrophages of apoptotic cells. DOCK2 is found in peripheral blood lymphocytes and can convert a flatten cell into a rounded morphology upon transfection (Nagase, et. al. 1996. DNA Res 3:321-329, Nishihara, H. 1999. Hokkaido Igaku Zasshi 74:157).

As shown supra, DOCK2 is a PL and binds to PDZ proteins. Using PDZ proteins as an adaptor, DOCK2 complexes with A,B,C to control cell shape in preparation for transit of lymphocytes for vascular circulation. Modulation of DOCK2 by agonists and antagonists of its PDZ protein interaction can be used to treat, but is not limited to, acute leukemia, blast crisis, post-myocardial infarction inflammation, post-traumatic inflammation.

### W. CD34

As is shown *supra,* CD34 binds DLG1, PSD95, and NeDLG. CD34 is expressed on a small subpopulation of bone marrow cells which includes hematopoietic stem cells. CD34 is also present on bone marrow stomal cells and on endothelial cells. The selectins CD62L (L- selectin) and CD62E (E- selectin) bind CD34. CD34 mediates attachment and rolling of leukocytes. The hematopoietic stem cell properties of CD34 include myeloid differentiation of stem cells. Modulation of the CD34- PDZ interaction with agonists and/or antagonists can be used to treat, but is not limited to, myelodysplasia, leukemias, post- traumatic inflammation, post- myocardial infarction inflammation.

### X. Fc epsilon receptor beta I chain (FcεRβI)

The high affinity receptor for human IgE, FcεRI, is composed of an α, β, and disulfide-linked γ homodimer. The α-chain binds the Fc portion of IgE, whereas the β-chain serves to amplify signals that are transduced through the γ-chain homodimer. Both (αβγ2 tetramer and αγ2 trimer complexes exist, but the β-chain amplifies the signal 5- to 7-fold, as measured by Syk activation and calcium mobilization. Additionally, the FcεRβI is a PDZ ligand and is a member of the CD20/ FcεRβI receptor family. As is shown *supra,* FcεRβI binds MINT 1.

As the high-affinity receptor for IgE, FcβRI on basophils and mast cells plays a central role in the initiation of allergic responses. Signaling through the FcβRI begins by crosslinking of a multivalent allergen bound to IgE. The result is vesicular degranulation, release of histamine, leukotrienes and pro-inflammatory cytokines (IL-6 and TNFα), factors responsible for the symptoms of immediate hypersensitivity. Alteration of signaling by targeting PL/PDZ interaction with agonists and antagonists can be used to treat, but is not limited to, asthma, atopic dermatitis, eczema, drug reaction, mastocytosis, urticaria, eosinophilia myalgia syndrome (Turner, H., et. al., 1999, Nature 402 SUPP:B24).

### Y. FAS Ligand (FasL)

CD95 (Fas/Apo- 1) and Fas ligand (FasL) are a receptor- ligand pair critically involved in lymphocyte homeostasis and peripheral tolerance. Binding of Fas by its ligand results in apoptotic cell death, an important major mechanism for safe clearance of unwanted cell during resolution of the acute inflammatory response. FasL is mainly restricted to activated T lymphocytes and is rapidly induced. Fas ligand is frequently up- regulated in breast cancer, as compared with normal breast epithelial cells and benign breast disease. As is shown *supra,* FasL binds the KIAA0561 PDZ domain. The PDZ- PL modifiers are useful for treatment of, but not limited to, tumors, e.g., tumors unresponsive to conventional chemotherapy.

### Z. CDW125 (IL5R)

As is shown *supra,* CDW125 binds PTN-4 and RGS12. CDW125 is an IL-5 receptor expressed on eosinophils and basophils. IL5 promotes growth and differentiation of eosinophil precursors and actives mature eosinophils (Takatsu et al 1994, Adv. Immunol. 57:145-190). The secreted form of CDw125 has antagonistic properties and is able to inhibit IL-5-induced eosinophil proliferation and differentiation. Modulation of CDW125 binding to PDZ domains may be used to treat, but is not limited to, asthma, atopic dermatitis, eczema, drug reaction, urticaria, mastocytosis, eosinophilia.

### AA. Burkitt's lymphoma receptor-1 (BLR-1; CXCR5)

BLR- 1 is a transmembrane receptor detected primarily on B cells, and shown to be upregulated in stimulated T cells (Dobner et al., 1992, Eur J. Immunol. 22: 2795- 2799.; Flynn et al., 1998, J. Exp. Med. 188: 297- 304) . BLR- 1 functions in chemotaxis ofB and T cells into follicles of secondary lymphoid organs (e.g. spleen) for proper development and selection toward antigens (Forster et al., 1996, Cell 87: 1037- 1047. Its ligand is B- lymphocyte chemoattractant (BLC) , which is strongly expressed in the follicles of Peyer's patches, spleen and lymph nodes (Gunn et al., 1998, Nature 391: 799- 803) . Consistent with its chemotactic role is the demonstration that BLR- 1 expression is downregulated in developed, activated B cells (plasma cells) to prevent them from being retained in follicles (Forster et al., 1994, Cell Mol Biol 40: 381- 387) , and blr- /- B cells fail to migrate to B cell follicles (Forster et al., 1996) .

As shown by experiments reported herein, the C-terminal end of BLR-1 binds to the PDZ domain containing protein MINT1. Without intending to be bound by a particular mechanism, the interaction between BLR-1 and MINT1, and BLR-1 and PDZ domains is necessary for the proper distribution and signaling of BLR-1 on the cell surface. When this interaction is disrupted, the chemotactic abilities of lymphoid cells expressing BLR-1 is similarly disrupted. Such a disruption results in a reduced immune response, interference with the ability of lymphocytes to properly circulate and develop responses to antigen. Agonists and antagonists of this interaction can be used to treat, but is not limited to, systemic lupus erythematosus, scleroderma and other autoimmune diseases.

**BB. CD4**

CD4 is a co-receptor with the T cell receptor (TCR) involved in antigen recognition. Both CD4 and TCR belong to the immunoglobulin supergene family. T cells activation is enhanced by increasing the avidity of T cells for effector and target cells. The cytoplasmic domain is involved in signal transduction and association with the tyrosine kinase $p56^{lck}$. CD4 is expressed on most thymocytes, two-thirds of peripheral blood T lymphocytes, monocytes and macrophages.

Human immunodeficiency virus type- 1 (HIV- 1) infects cells by membrane fusion mediated by its envelope glycoproteins (gp120- gp41) and is triggered by the interaction of CD4 and a chemokine co- receptor, CCR5 or CXCR4. Modulation of CD4- PDZ inhibitors with agonists and antagonists can be used to treat, but is not limited to, HIV infection immediately after exposure to HIV, rheumatoid arthritis, multiple sclerosis, scleroderma, systemic lupus erythematosis, psoriasis.

6.5 Agonists and Antagonists of PDZ-PL Interactions

As described herein, interactions between PDZ proteins and PL proteins in cells (e.g., hematopoietic cells, e.g., T cells and B cells) may be disrupted or inhibited by the administration of inhibitors or antagonists. Inhibitors can be identified using screening assays described herein. The motifs disclosed herein are used to design inhibitors. In some aspects of the disclosure, the antagonists have a structure (e.g., peptide sequence) based on the C-terminal residues of PL-domain proteins listed in **TABLE 2**. In some aspects of the disclosure, the antagonists have a structure (e.g., peptide sequence) based on a PL motif disclosed herein.

The PDZ/PL antagonists and antagonists may be any of a large variety of compounds, both naturally occurring and synthetic, organic and inorganic, and including polymers (e.g., oligopeptides, polypeptides, oligonucleotides, and polynucleotides), small molecules, antibodies, sugars, fatty acids, nucleotides and nucleotide analogs, analogs of naturally occurring structures (e.g., peptide mimetics, nucleic acid analogs, and the like), and numerous other compounds. Although, for convenience, the present discussion primarily refers! antagonists of PDZ-PL interactions, it will be recognized that PDZ-PL interaction agonists can also be use in the methods disclosed herein.

In one aspect, the peptides and peptide mimetics or analogues contain an amino acid sequence that binds a PDZ domain in hematopoietic cells such as T cells and B cells, or otherwise inhibits the association of PL proteins and PDZ proteins. In one embodiment, the antagonists comprise a peptide that has a sequence corresponding to the carboxy-terminal sequence of a PL protein listed in **TABLE 2,** e.g., a peptide listed **TABLE 4.** Typically, the peptide comprises at least the C-terminal four (4) residues of the PL protein, and often the inhibitory peptide comprises more than four residues (e.g., at least five, six, seven, eight, nine, ten, twelve or fifteen residues) from the PL protein C-terminus. See, e.g. Section 6.5.1, *infra.* Moreover, the C-terminal domains of specific surface receptors expressed by hematopoietic system and endothelial cells may themselves be used as inhibitors, and may be used as the basis for rational design of non-peptide inhibitors. See Section 6.6, *infra.*

In some aspects, the antagonist is a fusion protein comprising such a sequence. Fusion proteins containing a transmembrane transporter amino acid sequence are particularly useful. See, e.g. Section 6.5.2, *infra.*

In some aspects, the inhibitor is conserved variant of the PL C-terminal protein sequence having inhibitory activity. See, e.g. Section 6.5.3, *infra.*

In some aspects, the antagonist is a peptide mimetic of a PL C-terminal sequence. See, e.g. Section 6.5.4, *infra.*

In some aspects, the inhibitor is a small molecule (i.e., having a molecular weight less than 1 kD). See, e.g. Section 6.5.5, *infra.*

6.5.1 Peptide Antagonists

In one aspect, the antagonists comprise a peptide that has a sequence of a PL protein carboxy-terminus listed in **TABLE 2**. The peptide comprises at least the C-terminal two (2) residues of the PL protein, and typically, the inhibitory peptide comprises more than two residues (e.g, at least three, four, five, six, seven, eight, nine, ten, twelve or fifteen residues) from the PL protein C-terminus. Most often, the residues shared by the inhibitory peptide with the PL protein are found at the C-terminus of the peptide. However, in some aspects, the sequence is internal. Similarly, in some cases, the inhibitory peptide comprises residues from a PL sequence that is near, but not at the c-terminus of a PL protein (see, Gee et al., 1998, J Biological Chem. 273:21980-87).

For example, the "core PDZ domain binding motif sequence" of a hematopoietic cell surface receptor at its C-terminus contains the last four amino acids, this sequence may be used to target PDZ domains in hematopoietic cells. The four amino acid core of a PDZ domain binding motif sequence may contain additional amino acids at its amino terminus to further increase its binding affinity and/or stability. In one embodiment, the PDZ domain binding motif sequence peptide can be from four amino acids up to 15 amino acids. It is preferred that the length of the sequence to be 6-10 amino acids. More preferably, the PDZ domain binding motif sequence contains 8 amino acids. Additional amino acids at the amino terminal end of the core sequence may be derived from the natural sequence in each hematopoietic cell surface receptor or a synthetic linker. The additional amino acids may also be conservatively substituted. When the third residue from the C-terminus is S, T or Y, this residue may be phosphorylated prior to the use of the peptide.

In some aspects, the peptide and nonpeptide inhibitors of the are small, e.g., fewer than ten amino acid residues in length if a peptide. Further, it is reported that a limited number of ligand amino acids directly contact the PDZ domain (generally less than eight) (Kozlov et al., 2000, Biochemistry 39, 2572; Doyle et al., 1996, Cell 85, 1067) and that peptides as short as the C-terminal three amino acids often retain similar binding properties to longer (> 15) amino acids peptides (Yanagisawa et al., 1997, J. Biol. Chem. 272, 8539).

**FIGURES 3A- H** show the use of peptides to inhibit PL- PDZ interactions using the G assay described *supra.* In **FIGURE 3A and B,** the inhibiton assays were carried out using GST fusion proteins containing PDZ domains from DLG1 or PSD95 (see *supra* and **TABLE 3**) . Binding of biotinylated PL peptides for Clasp 2, CD46, Fas, or KV1.3 (as listed in **TABLE 4**) was determined in the presence of various competitor peptides (at a concentration of 100 uM) or in the absence of a competitor (equalized as 100% binding) . The competitor peptides were 8- mers peptides having the sequence of C- terminus of Clasp 2 (MTSSSSVV) , CD46 (REVKFTSL) , or Fas (RNEIQSLV) , a unlabeled 19- mer having the sequence of c- terminus of KV1.3 (i.e., non- biotinylated AA33L as listed in **TABLE 3**) , or a peptide having the sequence of residues 64- 76 of hemoglobin (Vidal et al., 1999, J. Immunol. 163, 4811) , i.e., an unrelated competitor. The binding of biotinylated peptide (10 uM for Fas and KV1.3, 20 uM for Clasp 2 and CD46) to GST alone was subtracted from the binding to the fusion proteins to obtain the net signal for each experimental condition. This net signal was then normalized by dividing by the signal in the absence of competitor peptide and the data were plotted. Error bars indicated the standard deviation of duplicate measurements. Specific inhibition of Clasp 2 PL- DLG PDZ binding was observed with the CLASP- 2 8- mer, the CD46 8- mer, the FAS- 8- mer, and the KV13 peptide, but not in the absence of peptide or using an unrelated peptide.

**FIGURES 3C-F** show similar assays using shorter peptides to inhibit (e.g., a 3-mer and a 5-mer). Figures 3C-E show binding of biotinylated PL peptides for Clasp 2, CD46, Fas, or KV1.3, at the indicated concentration (as listed in **TABLE 3**) to GST fusion proteins containing PDZ domains from NeDLG, DLG1, or PSD95 in the absence or presence of 1 mM 3-mer peptide having the sequence of the C-terminus of Clasp 2 (SVV). (Table 3). **FIGURE 3F** shows the effect on binding of a 5-mer CD49E peptide (**ATSDA**) to GST fusion proteins containing a PDZ domain from 41.8Kd

6.5.2 <u>Peptide Variants</u>

Having identified PDZ binding peptides and PDZ-PL interaction inhibitory sequences, variations of these sequences can be made and the resulting peptide variants can be tested for PDZ domain binding or PDZ-PL inhibitory activity. In further aspects, the variants have the same or a different ability to bind a PDZ domain as the parent peptide. Typically, such amino acid substitutions are conservative, i.e., the amino acid residues are replaced with other amino acid residues having physical and/or chemical properties similar to the residues they are replacing. Preferably, conservative amino acid substitutions are those wherein an amino acid is replaced with another amino acid encompassed within the same designated class.

6.5.3 <u>Peptide Mimetics</u>

Having identified PDZ binding peptides and PDZ-PL interaction inhibitory sequences, peptide mimetics can be prepared using routine methods, and the inhibitory activity of the mimetics can be confirmed using the assays of the invention. Thus, in some further aspects, the antagonist is a peptide mimetic of a PL C-terminal sequence. The skilled artisan will recognize that individual synthetic residues and polypeptides incorporating mimetics can be synthesized using a variety of procedures and methodologies, which are well described in the scientific and patent literature, e.g., Organic Syntheses Collective Volumes, Gilman et al. (Eds) John Wiley & Sons, Inc., NY. Polypeptides incorporating mimetics can also be made using solid phase synthetic procedures, as described, e.g., by Di Marchi, et al., U.S. Pat.

No. 5,422,426. Mimetics can also be synthesized using combinatorial methodologies. Various techniques for generation of peptide and peptidomimetic libraries are well known, and include, e.g., multipin, tea bag, and split-couple-mix techniques; see, e.g., al-Obeidi (1998) Mol. Biotechnol. 9:205-223; Hruby (1997) Curr. Opin. Chem. Biol. 1:114-119; Ostergaard (1997) Mol. Divers. 3:17-27; Ostresh (1996) Methods Enzymol. 267:220-234.

6.5.4 Small Molecules

In some aspects, the inhibitor is a small molecule (i.e., having a molecular weight less than 1 kD). Methods for screening small molecules are well known in the art and include those described *supra* at Section 6.4.

6.6 Cell Surface Receptors and PDZ-Domain Binding Sequences

The following sections describe specific surface receptors expressed by different cell types in the hematopoietic and immune response system. The C-termini of these receptors are used as inhibitors, or serve as the basis for designing PDZ domain binding motif sequence peptides, variants, fusion proteins, peptidomimetics, and small molecules for use in inhibiting PDZ-PL interactions. In a preferred aspect, the peptides are tested in an assay for inhibitory or modulatory activity (also see, **TABLE 4**, and discussion *supra).*

6.6.1 I PDZ domain binding Motif Sequences of T Cell Surface Receptors

A number of surface receptors expressed by T cells contain a PDZ domain binding motif sequence (PL sequence). These molecules include CD3η, CD4, CD6, CD38, CD49e, CD49f, CD53, CD83, CD90, CD95, CD97, CD98, CDw137 (41BB), CD166, CDwl28 (IL8 R), DNAM-1, Fas ligand (FasL) and LPAP (Barclay et al., 1997, The Leucocyte Antigen Facts Book, second edition, Academic Press), CLASP-1, CLASP-2, CLASP-4, KV 1.3, and DOCK2.

The C-terminal core sequence of CD3 is SSQL (SEQ ID NO:4). When naturally-occurring residues are added to the core sequence, SSSQL (SEQ ID NO:5), SSSSQL (SEQ ID NO:6), PSSSSQL (SEQ ID NO:7), and PPSSSSQL (SEQ ID NO:8) may also be used to target a PDZ domain-containing protein in T cells.

The C-terminal core sequence of CD4 is CSPI (SEQ ID NO:9). When naturally-occurring residues are added to the core sequence, TCSPI (SEQ ID NO:10), KTCSPI ; (SEQ ID NO:11), QKTCSPI (SEQ ID NO:12), and FQKTCSPI (SEQ ID NO:13) may also be used to target a PDZ domain-containing protein in T cells.

The C-terminal core sequence of CD6 is ISAA (SEQ ID NO:14). When naturally-occurring residues are added to the core sequence, DISAA (SEQ ID NO:15), DDISAA (SEQ ID NO:16), YDDISAA (SEQ ID NO:17), and DYDDISAA (SEQ ID NO:18) may also be used to target a PDZ domain-containing protein in T cells.

The C-terminal core sequence of CD38 is TSEI (SEQ ID NO:19). When naturally-occurring residues are added to the core sequence, CTSEI (SEQ ID NO:20), SCTSEI (SEQ ID NO:21), SSCTSEI (SEQ ID NO:22), and DSSCTSEI (SEQ ID NO:23) may also be used to target a PDZ domain-containing protein in T cells.

The C-terminal core sequence of CD49e is TSDA (SEQ ID NO:24). When naturally-occurring residues are added to the core sequence, ATSDA (SEQ ID NO:25), PATSDA (SEQ ID NO:26), PPATSDA (SEQ ID NO:27), and KPPATSDA (SEQ ID NO:28) may also be used to target a PDZ domain-containing protein in T cells.

The C-terminal core sequence of CD49f is TSDA (SEQ ID NO:29). When naturally-occurring residues are added to the core sequence, LTSDA (SEQ ID NO:30), RLTSDA (SEQ ID NO:31), ERLTSDA (SEQ ID NO:32), and KERLTSDA (SEQ ID NO:33) may also be used to target a PDZ domain-containing protein in T cells.

The C-terminal core sequence of CD53 is TIGL (SEQ ID NO:34). When naturally-occurring residues are added to the core sequence, QTIGL (SEQ ID NO:35), SQTIGL (SEQ ID NO:36), TSQTIGL (SEQ ID NO:37), and KTSQTIGL (SEQ ID NO:38) may also be used to target a PDZ domain-containing protein in T cells.

The C-terminal core sequence of CD83 is TELV (SEQ. ID. NO:248). When naturally-occurring residues are added to the core sequence, KTELV (SEQ. ID. NO: 249), HKTELV (SEQ. ID. NO:250), PHKTELV (SEQ. ID. NO:251), and TPHKTELV (SEQ. ID. NO:252) may also be used to target a PDZ domain-containing protein in T cells.

The C-terminal core sequence of CD90 is FMSL (SEQ ID NO:39). When naturally-occurring residues are added to the core sequence, DFMSL (SEQ ID NO:40), TDFMSL (SEQ ID NO:41), ATDFMSL (SEQ ID NO:42), and QATDFMSL (SEQ ID NO:43) may also be used to target a PDZ domain-containing protein in T cells.

The C-terminal core sequence of CD95 is QSLV (SEQ ID NO:44). When naturally-occurring residues are added to the core sequence, IQSLV (SEQ ID NO:45), EIQSLV (SEQ ID NO:46), NEIQSLV (SEQ ID NO:47), and RNEIQSLV (SEQ ID NO:48) may also be used to target a PDZ domain-containing protein in T cells.

The C-terminal core sequence of CD97 is ESGI (SEQ ID NO:49). When naturally-occurring residues are added to the core sequence, SESGI (SEQ ID NO:50), ASESGI (SEQ ID NO:51), RASESGI (SEQ ID NO:52), and LRASESGI (SEQ ID NO:53) may also be used to target a PDZ domain-containing protein in T cells.

The C-terminal core sequence of CD98 is PYAA (SEQ ID NO:54). When naturally-occurring residues are added to the core sequence, FPYAA (SEQ ID NO:55), RFPYAA (SEQ ID NO:56), LRFPYAA (SEQ ID NO:57), and LLRFPYAA (SEQ ID NO:58) may also be used to target a PDZ domain-containing protein in T cells.

The C-terminal core sequence of CDw137 is GCEL (SEQ ID NO:59). When naturally-occurring residues are added to the core sequence, GGCEL (SEQ ID NO:60), EGGCEL (SEQ ID NO:61), EEGGCEL (SEQ ID NO:62), and EEEGGCEL (SEQ ID NO:63) may also be used to target a PDZ domain-containing protein in T cells.

The C-terminal core sequence of CD166 is KTEA (SEQ ID NO:64). When naturally-occurring residues are added to the core sequence, HKTEA (SEQ ID NO:65), NHKTEA (SEQ ID NO:66), NNHKTEA (SEQ ID NO:67), and ENNHKTEA (SEQ ID NO:68) may also be used to target a PDZ domain-containing protein in T cells.

The C-terminal core sequence of CDw128 is SSNL (SEQ ID NO:69). When naturally-occurring residues are added to the core sequence, VSSNL (SEQ ID NO:70), NVSSNL (SEQ ID NO:71), VNVSSNL (SEQ ID NO:72), and SVNVSSNL (SEQ ID NO:73) may also be used to target a PDZ domain-containing protein in T cells.

The C-terminal core sequence of DNAM-1 is KTRV (SEQ ID NO:74). When naturally-occurring residues are added to the core sequence, PKTRV (SEQ ID NO:75), RPKTRV (SEQ ID NO:76), RRPKTRV (SEQ ID NO:77), and SRRPKTRV (SEQ ID NO:78) may also be used to target a PDZ domain-containing protein in T cells.

The C-terminal core sequence of FasL is LYKL (SEQ ID NO:79). When naturally-occurring residues are added to the core sequence, GLYKL (SEQ ID NO:80), FGLYKL (SEQ ID NO:81), FFGLYKL (SEQ ID NO:82), and TFFGLYKL (SEQ ID NO:83) may also be used to target a PDZ domain-containing protein in T cells.

The C-terminal core sequence of LPAP is VTAL (SEQ ID NO:84). When naturally-occurring residues are added to the core sequence, HVTAL (SEQ ID NO:85), LHVTAL (SEQ ID NO:86), GLHVTAL (SEQ ID NO:87), and QGLHVTAL (SEQ ID NO:88) may also be used to target a PDZ domain-containing protein in T cells.

The C-terminal core sequence of CLASP-1 is SAQV (SEQ. ID. NO:218). When naturally-occurring residues are added to the core sequence, SSAQV (SEQ. ID. NO: 219), SSSAQV (SEQ. ID. NO:220), ISSSAQV (SEQ. ID. NO: 221), and SISSSAQV (SEQ. ID. NO:222) may also be used to target a PDZ domain-containing protein in T cells.

The C-terminal core sequence of CLASP-2 is SSVV (SEQ. ID. NO:223). When naturally-occurring residues are added to the core sequence, SSSVV (SEQ. ID. NO: 224), SSSSVV (SEQ. ID. NO:225), TSSSSVV (SEQ. ID. NO: 226), and MTSSSSVV (SEQ. ID. NO:227) may also be used to target a PDZ domain-containing protein in T cells.

The C-terminal core sequence of CLASP-4 is YAEV (SEQ. ID. NO:228). When naturally-occurring residues are added to the core sequence, RYAEV (SEQ. ID. NO: 229), PRYAEV (SEQ. ID. NO:230), SPRYAEV (SEQ. ID. NO: 231), and GSPRYAEV (SEQ. ID. NO:232) may also be used to target a PDZ domain-containing protein in T cells.

The C-terminal core sequence of KV1.3 is FTDV (SEQ. ID. NO:238). When naturally-occurring residues are added to the core sequence, IFTDV (SEQ. ID. NO:239), KIFTDV (SEQ. ID. NO:240), KKIFTDV (SEQ. ID. NO:241), and IKKIFTDV (SEQ. ID. NO: 242) may also be used to target a PDZ domain-containing protein in T cells.

The C-terminal core sequence of DOCK2 is STDL (SEQ. ID. NO:243). When naturally-occurring residues are added to the core sequence, LSTDL (SEQ. ID. NO:244), SLSTDL (SEQ. ID. NO:245), DSLSTDL (SEQ. ID. NO:246), and PDSLSTDL (SEQ. ID. NO:247) may also be used to target a PDZ domain-containing protein in T cells.

6.6.2 PDZ Domain Binding Motif Sequences of B Cell Surface Receptors

A number of surface receptors expressed by B cells contain a PDZ domain motif sequence. These molecules include, but are not limited to, CD38, CD53, CD95, CD97, CD98, CDw137, CD138, CDw125 (IL5R), DNAM-1, LPAP, Syndecan-2 (Barclay ct al., 1997, The Leucocyte Antigen Facts Book, second edition, Academic Press) and BLR-1. The specific motif sequences of CD38, CD53, CD83, CD95, CD97, CD98, CDw137, DNA-1, DOCK2, LPAP, CLASP-1, CLASP-2 and CLASP-4 have been described in the preceding paragraphs.

The C-terminal core sequence of CD138 is EFYA (SEQ ID NO:89). When naturally-occurring residues are added to the core sequence, EEFYA (SEQ ID NO:90), QEEFYA (SEQ ID NO:91), KQEEFYA (SEQ ID NO:92), and TKQEEFYA (SEQ ID NO:93) may also be used to target a PDZ domain-containing protein in B cells.

The C-terminal core sequence of CDw125 is DSVF (SEQ ID NO:94). When naturally-occurring residues are added to the core sequence, EDSVF (SEQ ID NO:95), LEDSVF (SEQ ID NO:96), TLEDSVF (SEQ ID NO:97), and ETLEDSVF (SEQ ID NO:98) may also be used to target a PDZ domain-containing protein in B cells.

The C-tcrminal core sequence of Syndecan-2 is EFYA (SEQ. ID. NO:258). When naturally-occurring residues are added to the core sequence, KEFYA (SEQ. ID. NO: 259), TKEFYA (SEQ. ID. NO:260), PTKEFYA (SEQ. ID. NO: 261), and APTKEFYA (SEQ. ID. NO:262) may also be used to target a PDZ domain-containing protein in B cells.

The C-terminal core sequence of BLR-1 is LTTF (SEQ. ID. NO: 253). When naturally-occurring residues are added to the core sequence, SLTTF (SEQ. ID. NO: 254), TSLTTF (SEQ. ID. NO: 255), ATSLTTF (SEQ. ID. NO: 256), and NATSLTTF (SEQ. ID. NO: 257) may also be used to target a PDZ domain-containing protein in B cells.

6.6.3 PDZ domain binding motif sequences of Natural Killer Cell Surface Receptors

A number of surface receptors expressed by NK cells contain a PDZ domain motif sequence. These molecules include, but are not limited to CD38, CD56, CD98 and DNAM-1. The specific motif sequences of CD38, CD98 and DNAM-1 have been described in the preceding paragraphs.

The C-terminal core sequence of CD56 is ESKA (SEQ ID NO:99). When naturally-occurring residues are added to the core sequence, NESKA (SEQ ID NO:100), ENESKA (SEQ ID NO:101), KENESKA (SEQ ID NO:102), and TKENESKA (SEQ ID N0:103) may also be used to target a PDZ domain-containing protein in NK cells.

6.6.4 PDZ domain binding motif sequences of Monocyte Surface Receptors

A number of surface receptors expressed by cells of the monocytic lineage (monocytes and macrophages) contain a PDZ domain motif sequence. These molecules include, but are not limited to CD38, CD44, CD46, CD49e, CD49f, CD53, CD61, CD95, CD97, CD98, CD148, CDw128, CDw137, Ly-6, DNAM-1 and FcεRIβ. The specific motif sequences of CD38, CD49e, CD49f, CD53, CD95, CD97, CD98, CDw128, CDw137, DNAM-1, Galectin 3 (Mac-2) and Mannose receptor have been described in the preceding paragraphs.

The C-terminal core sequence of CD44 is KIGV (SEQ ID NO:104). When naturally-occurring residues are added to the core sequence, MKIGV (SEQ ID NO:105), DMKIGV (SEQ ID NO:106), VDMKIGV (SEQ ID NO:107) and NVDMKIGV (SEQ ID NO:108) may also be used to target a PDZ domain-containing protein in monocytes.

The C-terminal core sequence of CD46 is FTSL (SEQ ID NO:109). When naturally-occurring residues are added to the core sequence, KFTSL (SEQ ID NO:110), VKFTSL (SEQ ID NO:111), EVKFTSL (SEQ ID NO:112) and REVKFTSL (SEQ ID NO:113) may also be used to target a PDZ domain-containing protein in monocytes.

The C-terminal core sequence of CD61 is KSLV (SEQ ID NO:114). When naturally-occurring residues are added to the core sequence, LKSLV (SEQ ID NO:115), FLKSLV (SEQ ID NO:116), RFLKSLV (SEQ ID NO:117) and GRFLKSLV (SEQ ID NO:118) may also be used to target a PDZ domain-containing protein in monocytes.

The C-terminal core sequence of CD148 is GYIA (SEQ ID NO:119). When naturally-occurring residues are added to the core sequence, NGYIA (SEQ ID NO:120), TNGYIA (SEQ ID NO:121), KTNGYIA (SEQ ID NO:122) and GKTNGYIA (SEQ ID NO:123) may also be used to target a PDZ domain-containing protein in monocytes.

The C-terminal core sequence of Ly-6 is QTLL (SEQ ID NO:124). When naturally-occurring residues are added to the core sequence, LQTLL (SEQ ID NO:125), LLQTLL (SEQ ID NO:126), VLLQTLL (SEQ ID NO:127) and SVLLQTLL (SEQ ID NO:128) may also be used to target a PDZ domain-containing protein in monocytes.

The C-terminal core sequence of FcεRIβ is PIDL (SEQ ID NO:129). When naturally-occurring residues are added to the core sequence, PPIDL (SEQ ID NO:130), SPPIDL (SEQ ID NO:131), MSPPIDL (SEQ ID NO:132) and EMSPPIDL (SEQ ID NO:133) may also be used to target a PDZ domain-containing protein in monocytes.

The C-terminal core sequence of Galectin 3 is YTMI (SEQ ID NO:134). When naturally-occurring residues are added to the core sequence, SYTMI (SEQ ID NO:135), ASYTMI (SEQ ID NO:136), SASYTMI (SEQ ID NO:137) and TSASYTMI (SEQ ID NO:138) may also be used to target a PDZ domain-containing protein in monocytes.

The C-terminal core sequence of mannose receptor is HSVI (SEQ ID NO:139). When naturally-occurring residues are added to the core sequence, EHSVI (SEQ ID NO:140), NEHSVI (SEQ ID NO:141), QNEHSVI (SEQ ID NO:142) and EQNEHSVI (SEQ ID NO:143) may also be used to target a PDZ domain-containing protein in monocytes.

6.6.5 PDZ domain binding motif sequences of Granulocyte Surface Receptors

A number of surface receptors expressed by granulocytes contain a PDZ domain motif sequence. These molecules include, but are not limited to CD53, CD95, CD97, CD98, CD148, CDw125, CDw128, FcεRIβ and G-CSFR. The specific motif sequences of most of these molecules have been described in the preceding paragraphs.

The C-terminal core sequence of G-CSFR is TSVL (SEQ ID NO:144). When naturally-occurring residues are added to the core sequence, ITSVL (SEQ ID NO:145), PITSVL (SEQ ID NO:146), FPITSVL (SEQ ID NO:147) and LFPITSVL (SEQ ID NO:148) may also be used to target a PDZ domain-containing protein in monocytes.

6.6.6 PDZ domain binding motif sequences of Endothelial Cell Surface Receptors

While endothelial cells are not hematopoietic cells, they closely interact with the hematopoietic system as they form the lining of blood vessels. As such, endothelial cells come in contact with the cells of the hematopoietic system. Thus, the ability to regulate endothelial cell function provides for indirect regulation of hematopoietic cells.

A number of surface receptors expressed by endothelial cells contain a PDZ domain motif sequence. These molecules include, but are not limited to CD34, CD46, CD66b, CD66c, CD105, CD106, CD62e (E- selectin) and VCAM1.

The C-terminal core sequence of CD34 is DTEL (SEQ ID NO:149). When naturally-occurring residues are added to the core sequence, ADTEL (SEQ ID NO:150), VADTEL (SEQ ID NO:151), VVADTEL (SEQ ID NO:152) and HVVADTEL (SEQ ID NO:153) may also be used to target a PDZ domain-containing protein in endothelial cells.

The C-terminal core sequence of CD66b and CD66c is VALI (SEQ ID NO:154). When naturally-occurring residues are added to the core sequence, RVALI (SEQ ID NO:155), ARVALI (SEQ ID NO:156), LARVALI (SEQ ID NO: 157) and VLARVALI (SEQ ID NO:158) may also be used to target a PDZ domain-containing protein in endothelial cells.

The C-terminal core sequence of CD105 is SSMA (SEQ ID NO:159). When naturally-occurring residues are added to the core sequence, TSSMA (SEQ ID NO:160), STSSMA (SEQ ID NO:161), CSTSSMA (SEQ ID NO:162) and PCSTSSMA (SEQ ID NO: 162) may also be used to target a PDZ domain-containing protein in endothelial cells.

The C-terminal core sequence of CD106 is KSKV (SEQ ID NO:163). When naturally-occurring residues are added to the core sequence, QKSKV (SEQ ID NO:164), AQKSKV (SEQ ID NO:165), EAQKSKV (SEQ ID NO:166) and VEAQKSKV (SEQ ID NO:167) may also be used to target a PDZ domain-containing protein in endothelial cells.

The C-terminal core sequence of CD62e is SYIL (SEQ ID NO:168). When naturally-occurring residues are added to the core sequence, PSYIL (SEQ ID NO:169), KPSYIL (SEQ ID NO:170), QKPSYIL (SEQ ID NO:171) and YQKPSYIL (SEQ ID NO:172) may also be used to target a PDZ domain-containing protein in endothelial cells.

The C-terminal core sequence of VCAM1 is KSKV (SEQ. ID. NO:233). When naturally-occurring residues are added to the core sequence, QKSKV (SEQ. ID. NO:234), AQKSKV (SEQ. ID. NO:235), EAQKSKV (SEQ. ID. NO:236), and VEAQKSKV (SEQ. ID. NO:237) may also be used to target a PDZ domain-containing protein in endothelial cells.

6.6.7 Mast Cell. Basophils and Eosinophil Cell Surface Receptors

FcεRIβ, CDw125, CDw128 and IL- 8RB are transmembrane receptors expressed by mast cells, basophils and eosinophils. These receptors play a role in the activation of these cells to result in degranulation and histamine release in allergic reactions. The C- terminal core sequence of FcεRIβ is PIDL (SEQ ID NO: 4) . When naturally- occurring residues are added to the core sequence, PPIDL (SEQ ID NO: 5) , SPPIDL (SEQ ID NO: 6) , MSPPIDL (SEQ ID NO: 7) and EMSPPIDL (SEQ ID NO: 8) may also be used to target a PDZ domain- containing protein in mast cells. In addition, the residue E may be substituted with G to increase its binding affinity.

The C-terminal core sequence of CDw125 is DSVF (SEQ ID NO:9). When naturally-occurring residues are added to the core sequence, EDSVF (SEQ ID NO:10), LEDSVF (SEQ ID NO:11), TLEDSVF (SEQ ID NO:12), and ETLEDSVF (SEQ ID NO:13) may also be used to target a PDZ domain-containing protein in mast cells.

The C-terminal core sequence of CDw128 is SSNL (SEQ ID NO:14). When naturally-occurring residues are added to the core sequence, VSSNL (SEQ ID NO:15), NVSSNL (SEQ ID NO:16), VNVSSNL (SEQ ID NO:17), and SVNVSSNL (SEQ ID NO:18) may also be used to target a PDZ domain-containing protein in mast cells.

The C-terminal core sequence of IL-8RB is STTL (SEQ ID NO:19). When naturally-occurring residues are added to the core sequence TSTTL (SEQ ID NO:20), HTSTTL (SEQ ID NO:21), GHTSTTL (SEQ ID NO:22) and SGHTSTTL (SEQ ID NO:23) may also be used to target a PDZ domain-containing protein in mast cells.

6.6.8 8 Other PDZ domain binding motif sequences

The C-terminal core sequence ofNMDA is ESDV (SEQ. ID. NO:263). When naturally-occurring residues are added to the core sequence, IESDV (SEQ. ID. NO:264), SIESDV (SEQ. ID. NO:265), PSLESDV (SEQ. ID. NO:266), and MPSIESDV (SEQ. ID. NO: 267) may also be used to target a PDZ domain-containing protein in neuronal cells.

The C-terminal core sequence of neurexin is EYYV (SEQ. ID. NO:268). When naturally-occurring residues are added to the core sequence, KEYYV (SEQ. ID. NO: 269), DKEYYV (SEQ. ID. NO:270), KDKEYYV (SEQ. ID. NO:271), and NKDKEYYV (SEQ. ID. NO:272) may also be used to target a PDZ domain-containing protein in neuronal cells.

The C-terminal core sequence of Glycophorin C is EYFI (SEQ. ID. NO:273). When naturally-occurring residues are added to the core sequence, KEYFI (SEQ. ID. NO: 274), RKEYFI (SEQ. ID. NO:275), SRKEYFI (SEQ. ID. NO:276), and SSRKEYFI (SEQ. ID. NO:277) may also be used to target a PDZ domain-containing protein.

The C-terminal core sequence of CD148 is KTIA (SEQ. ID. NO:278). When naturally-occurring residues are added to the core sequence, GKTIA (SEQ. ID. NO:279), FGKTIA (SEQ. ID. NO:280), TFGKTIA (SEQ. ID. NO:281), and TTFGKTIA (SEQ. ID. NO: 282) may also be used to target a PDZ domain-containing protein in epithelial or myeloid cells.

6.7. Preparation of Peptides

6.7.1. Chemical Synthesis

The peptides of the invention or analogues thereof, may be prepared using virtually any art-known technique for the preparation of peptides and peptide analogues. For example, the peptides may be prepared in linear form using conventional solution or solid phase peptide syntheses and cleaved from the resin followed by purification procedures (Creighton, 1983, Protein Structures And Molecular Principles, W.H. Freeman and Co., N.Y.). Suitable procedures for synthesizing the peptides described herein are well known in the art. The composition of the synthetic peptides may be confirmed by amino acid analysis or sequencing (e.g., the Edman degradation procedure and mass spectroscopy).

In addition, analogues and derivatives of the peptides can be chemically synthesized. The linkage between each amino acid of the peptides of the invention may be an amide, a substituted amide or an isostere of amide. Non-classical amino acids or chemical amino acid analogues can be introduced as a substitution or addition into the sequence. Non-classical amino acids include, but are not limited to, the D-isomers of the common amino acids, α-amino isobutyric acid, 4-aminobutyric acid, Abu, 2-amino butyric acid, γ-Abu, ε-Ahx, 6-amino hexanoic acid, Aib, 2-amino isobutyric acid, 3-amino propionic acid, ornithine, norleucine, norvaline, hydroxyproline, sarcosine, citrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogues in general. Furthermore, the amino acid can be D (dextrorotary) or L (levorotary).

6.7.2. Recombinant Synthesis

If the peptide is composed entirely of gene-encoded amino acids, or a portion of it is so composed, the peptide or the relevant portion may also be synthesized using conventional recombinant genetic engineering techniques. For recombinant production, a polynucleotide sequence encoding a linear form of the peptide is inserted into an appropriate expression vehicle, i.e., a vector which contains the necessary elements for the transcription and translation of the inserted coding sequence, or in the case of an RNA viral vector, the necessary elements for replication and translation. The expression vehicle is then transfected into a suitable target cell which will express the peptide. Depending on the expression system used, the expressed peptide is then isolated by procedures well-established in the art. Methods for recombinant protein and peptide production are well known in the art (*see, e.g.,* Maniatis et al., 1989, Molecular Cloning A Laboratory Manual, Cold Spring Harbor Laboratory, N.Y.; and Ausubel et al., 1989, Current Protocols in Molecular Biology, Greene Publishing Associates and Wiley Interscience, N.Y.).

A variety of host-expression vector systems may be utilized to express the peptides described herein. These include, but are not limited to, microorganisms such as bacteria transformed with recombinant bacteriophage DNA or plasmid DNA expression vectors containing an appropriate coding sequence; yeast or filamentous fungi transformed with recombinant yeast or fungi expression vectors containing an appropriate coding sequence; insect cell systems infected with recombinant virus expression vectors (*e.g.*, baculovirus) containing an appropriate coding sequence; plant cell systems infected with recombinant virus expression vectors (*e.g.*, cauliflower mosaic virus or tobacco mosaic virus) or transformed with recombinant plasmid expression vectors (*e.g.*, Ti plasmid) containing an appropriate coding sequence; or animal cell systems.

The expression elements of the expression systems vary in their strength and specificities. Depending on the host/vector system utilized, any of a number of suitable transcription and translation elements, including constitutive and inducible promoters, may be used in the expression vector. For example, when cloning in bacterial systems, inducible promoters such as pL of bacteriophage λ, plac, ptrp, ptac (ptrp-lac hybrid promoter) and the like may be used; when cloning in insect cell systems, promoters such as the baculovirus polyhedron promoter may be used; when cloning in plant cell systems, promoters derived from the genome of plant cells (*e.g.*, heat shock promoters; the promoter for the small subunit of RUBISCO; the promoter for the chlorophyll a/b binding protein) or from plant viruses (*e.g.*, the 35S RNA promoter of CaMV; the coat protein promoter of TMV) may be used; when cloning in mammalian cell systems, promoters derived from the genome of mammalian cells (*e.g.*, metallothionein promoter) or from mammalian viruses (*e.g.*, the adenovirus late promoter; the vaccinia virus 7.5 K promoter) may be used; when generating cell lines that contain multiple copies of expression product, SV40-, BPV- and EBV-based vectors may be used with an appropriate selectable marker.

In cases where plant expression vectors are used, the expression of sequences encoding the peptides of the invention may be driven by any of a number of promoters. For example, viral promoters such as the 35S RNA and 19S RNA promoters of CaMV (Brisson et al., 1984, Nature 310: 511- 514) , or the coat protein promoter of TMV (Takamatsu et al., 1987, EMBO J. 6: 307- 311) may be used; alternatively, plant promoters such as the small subunit of RUBISCO (Coruzzi et al., 1984, EMBO J. 3: 1671- 1680; Broglie et al., 1984, Science 224: 838- 843) or heat shock promoters, *e.g.*, soybean hsp17.5- E or hsp17.3- B (Gurley et al., 1986, Mol. Cell. Biol. 6: 559- 565) may be used. These constructs can be introduced into planleukocytes using Ti plasmids, Ri plasmids, plant virus vectors, direct DNA transformation, microinjection, electroporation, etc. For reviews of such techniques *see, e.g.,* Weissbach & Weissbach, 1988, Methods for Plant Molecular Biology, Academic Press, NY, Section VIII, pp. 421- 463; and Grierson & Corey, 1988, Plant Molecular Biology, 2d Ed., Blackie, London, Ch. 7- 9.

In one insect expression system that may be used to produce the peptides of the invention, *Autographa californica* nuclear polyhidrosis virus (AcNPV) is used as a vector to express the foreign genes. The virus grows in *Spodoptera frugiperda* cells. A coding sequence may be cloned into non- essential regions (for example the polyhedron gene) of the virus and placed under control of an AcNPV promoter (for example, the polyhedron promoter) . Successful insertion of a coding sequence will result in inactivation of the polyhedron gene and production of non- occluded recombinant virus (*i.e.*, virus lacking the proteinaceous coat coded for by the polyhedron gene) . These recombinant viruses are then used to infect *Spodoptera frugiperda* cells in which the inserted gene is expressed. (*e.g.*, see Smith et al., 1983, J. Virol. 46: 584; Smith, U.S. Patent No. 4, 215, 051) . Further examples of this expression system may be found in Current Protocols in Molecular Biology, Vol. 2, Ausubel et al., eds., Greene Publish. Assoc. & Wiley Interscience.

In mammalian host cells, a number of viral based expression systems may be utilized. In cases where an adenovirus is used as an expression vector, a coding sequence may be ligated to an adenovirus transcription/translation control complex, *e.g*., the late promoter and tripartite leader sequence. This chimeric gene may then be inserted in the adenovirus genome by *in vitro* or *in vivo* recombination. Insertion in a non-essential region of the viral genome (*e.g.*, region E1 or E3) will result in a recombinant virus that is viable and capable of expressing peptide in infected hosts. (*e.g.,* See Logan & Shenk, 1984, Proc. Natl. Acad. Sci. USA 81:3655-3659). Alternatively, the vaccinia 7.5 K promoter may be used, (*see, e.g.,* Mackett et al., 1982, Proc. Natl. Acad. Sci. USA 79:7415-7419; Mackett et al., 1984, J. Virol. 49:857-864; Panicali et al., 1982, Proc. Natl. Acad. Sci. USA 79:4927-4931).

Other expression systems for producing linear peptides of the invention will be apparent to those having skill in the art.

6.7.3. <u>Purification of the Peptides and Peptide Analogues</u>

The peptides and peptide analogues of the invention can be purified by art-known techniques such as high performance liquid chromatography, ion exchange chromatography, gel electrophoresis, affinity chromatography and the like. The actual conditions used to purify a particular peptide or analogue will depend, in part, on factors such as net charge, hydrophobicity, hydrophilicity, etc., and will be apparent to those having skill in the art. The purified peptides can be identified by assays based on their physical or functional properties, including radioactive labeling followed by gel electrophoresis, radioimmuno-assays, ELISA, bioassays, and the like.

For affinity chromatography purification, any antibody which specifically binds the peptides or peptide analogues may be used. For the production of antibodies, various host animals, including but not limited to rabbits, mice, rats, etc., may be immunized by injection with a peptide. The peptide may be attached to a suitable carrier, such as BSA or KLH, by means of a side chain functional group or linkers attached to a side chain functional group. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacilli Calmette-Guerin) and *Corynebacterium parvum.*

Monoclonal antibodies to a peptide may be prepared using any technique which provides for the production of antibody molecules by continuous cell lines in culture. These include but are not limited to the hybridoma technique originally described by Koehler and Milstein, 1975, Nature 256:495-497, the human B-cell hybridoma technique, Kosbor et al., 1983, Immunology Today 4:72; Cote et al., 1983, Proc. Natl. Acad. Sci. U.S.A. 80:2026-2030 and the EBV-hybridoma technique (Cole et al., 1985, Monoclonal Antibodies and Cancer Therapy, Alan R. Liss, Inc., pp. 77-96 (1985)). In addition, techniques developed for the production of "chimeric antibodies" (Morrison et al., 1984, Proc. Natl. Acad. Sci. U.S.A. 81:6851-6855; Neuberger et al., 1984, Nature 312:604-608; Takeda et al., 1985, Nature 314:452-454) by splicing the genes from a mouse antibody molecule of appropriate antigen specificity together with genes from a human antibody molecule of appropriate biological activity can be used. Alternatively, techniques described for the production of single chain antibodies (U.S. Patent No. 4,946,778) can be adapted to produce peptide-specific single chain antibodies.

Antibody fragments which contain deletions of specific binding sites may be generated by known techniques. For example, such fragments include but are not limited to $F(ab')_2$ fragments, which can be produced by pepsin digestion of the antibody molecule and Fab fragments, which can be generated by reducing the disulfide bridges of the $F(ab')_2$ fragments. Alternatively, Fab expression libraries may be constructed (Huse et al., 1989, Science 246:1275-1281) to allow rapid and easy identification of monoclonal Fab fragments with the desired specificity for the peptide of interest.

The antibody or antibody fragment specific for the desired peptide can be attached, for example, to agarose, and the antibody-agarose complex is used in immunochromatography to purify peptides of the invention. *See,* Scopes, 1984, Protein Purification: Principles and Practice, Springer-Verlag New York, Inc., NY, Livingstone, 1974, Methods Enzymology: Immunoaffinity Chromatography of Proteins 34:723-731.

6.8. <u>Uses of PDZ Domain Binding and Antagonist Compounds</u>

In one aspect of the invention, the PDZ domain binding and PDZ-PL inhibitory compounds of the present invention are useful in regulating diverse activities of hematopoietic cells (e.g., T cells and B cells) and other cells.involved in the immune response.

In one aspect of the disclosure, the compounds are used to inhibit leukocyte activation, which is manifested in measurable events including but not limited to, cytokine production, cell adhesion, expansion of cell numbers, apoptosis and cytotoxicity. As a corollary, the compounds may be used to treat diverse conditions associated with undesirable leukocyte activation, including but not limited to, acute and chronic inflammation, graft-versus-host disease, transplantation rejection, hypersensitivities and autoimmunity such as multiple sclerosis, rheumatoid arthritis, peridontal disease, systemic lupus erythematosis, juvenile diabetes mellitis, non-insulin-dependent diabetes, and allergies, and other conditions listed herein (see, e.g., Section 6.4, *supra*).

Thus, an aspect of the disclosure also relates to methods of using such compositions in modulating leukocyte activation as measured by, for example, cytotoxicity, cytokine production, cell proliferation, and apoptosis. Assays for activation are well known. For example, PDZ/PL interaction antagonists can be evaluated in the following: (1) cytotoxic T lymphocytes can be incubated with radioactively labeled target cells and the antigen- specific lysis of these target cells detected by the release of radioactivity, (2) helper T lymphocytes can be incubated with antigens and antigen presenting cells and the synthesis and secretion of cytokines measured by standard methods (Windhagen A; et al., 1995, Immunity 2 (4) : 373- 80) , (3) antigen presenting cells can be incubated with whole protein antigen and the presentation of that antigen on MHC detected by either T lymphocyte activation assays or biophysical methods (Harding et al., 1989, Proc. Natl. Acad. Sci., 86: 4230- 4) , (4) mast cells can be incubated with reagents that cross- link their Fc- epsilon receptors and histamine release measured by enzyme immunoassay (Siraganian, et al., 1983, TIPS 4: 432- 437) .

Similarly, the effect of PDZ/PL interaction antagonists on products of leukocyte activation in either a model organism (e.g., mouse) or a human patient can also be evaluated by various methods that are well known. For example, (1) the production of antibodies in response to vaccination can be readily detected by standard methods currently used in clinical laboratories, e.g., an ELISA; (2) the migration of immune cells to sites of inflammation can be detected by scratching the surface of skin and placing a sterile container to capture the migrating cells over scratch site (Peters et al., 1988, Blood 72: 1310- 5) ; (3) the proliferation of peripheral blood mononuclear cells in response to mitogens or mixed lymphocyte reaction can be measured using $^3$H- thymidine; (4) the phagocytic capacity of granulocytes, macrophages, and other phagocytes in PBMCs can be measured by placing PMBCs in wells together with labeled particles (Peters et al., 1988) ; and (5) the differentiation of immune system cells can be measured by labeling PBMCs with antibodies to CD molecules such as CD4 and CD8 and measuring the fraction of the PBMCs expressing these markers.

In one exemplary assay, human peripheral blood mononuclear cells (PBMC), human T cell clones (e.g., Jurkat E6, ATCC TIB-152), EBV-transformed B cell clones (e.g., 9D10, ATCC CRL-8752), antigen-specific T cell clones or lines can be used to examine PDZ/PL interaction antagonists in vitro. Inhibition of activation of these cells or cell lines can be used for the evaluation of potential PDZ/PL interaction antagonists.

Standard methods by which hematopoietic cells are stimulated to undergo activation characteristic of an immune response are, for example:

A) Antigen specific stimulation of immune responses. Either pre- immunized or naïve mouse splenocytes can be generated by standard procedures. In addition, antigen- specific T cell clones and hybridomas (e.g., MBP- specific) , and numerous B cell lymphoma cell lines (e.g., CH27) , have been previously characterized and are available for the assays discussed below. Antigen specific splenocytes or B- cells can be mixed with antigen specific T- cells in the presence of antigen to generate an immune response. This can be performed in the presence or absence of PDZ/PL interaction antagonists to assay whether PDZ/PL interaction antagonists modulate the immune response *infra.*

B) Non-specific T cell activation. The following methods can be used to activate T cells in the absence of antigen: 1) cross-linking T cell receptor (TCR) by addition of antibodies against receptor activation molecules (e.g., TCR, CD3, or CD2) together with antibodies against co-stimulator molecules, for example anti-CD28; 2) activating cell surface receptors in a non-specific fashion using lectins such as concanavalin A (con A) and phytohemagglutinin (PHA); 3) mimicking cell surface receptor-mediated activation using pharmacological agents that activate protein kinase C (e.g., phorbol esters) and increase cytoplasmic $Ca^{2+}$ (e.g., ionomycin).

C) Non-specific B cell activation: 1) application of antibodies against cell surface molecules such as IgM, CD20, or CD21. 2) Lipopolysaccharide (LPS), phorbol esters, calcium ionophores and ionomycin can also be used to by-pass receptor triggering.

D) Mixed lymphocyte reaction (MLR). Mix donor PBMC with recipient PBMC to activate lymphocytes by presentation of mismatched tissue antigens, which occurs in all cases except identical twins.

E) Generation of a specific T cell clone or line that recognizes a particular antigen. A standard approach is to generate tetanus toxin-specific T cells from a donor that has recently been boosted with tetanus toxin. Major histocompatability complex- (MHC-) matched antigen presenting cells and a source of tetanus toxin are used to maintain antigen specificity of the cell line or T cell clone (Lanzavecchia, A., et al., 1983, Eur. J. Immun. 13: 733-738).

Assay Quantitation

The assays described above can be quantitated by a variety of well known quantitation methods. For example:

(A) Tyrosine phosphorylation

Tyrosine phosphorylation of early response proteins such as HS1, PLC-r, ZAP-76, and Vav is an early biochemical event following leukocyte activation. The tyrosine phosphorylated proteins can be detected by Western blot using antibodies against phosphorylated tyrosine residues. Tyrosine phosphorylation of these early response proteins can be used as a standard assay for leukocyte activation (J. Biol. Chem., 1997, 272(23): 14562-14570). Any change in the phosphorylation pattern of these or related proteins when immune responses are generated in the presence of potential PDZ/PL interaction antagonists is indicative of a potential PDZ/PL interaction antagonists.

(B) Intracellular Calcium Flux

The kinetics of intracellular $Ca^{2+}$ concentrations are measured over time after stimulation of cells preloaded with a calcium sensitive dye. Upon binding $Ca^{2+}$ the indicator dye (e.g., Fluor-4 (Molecular Probes)), exhibits an increase in fluorescence level using flow cytometry, solution fluorometry, and confocal microscopy. Any change in the level or timing of calcium flux when immune responses are generated in the presence of PDZ/PL interaction antagonists is indicative of an inhibition of this response.

(C) Regulation of early activation markers

Increased and diminished expression/regulation of early lymphocyte activation marker levels such as CD69, IL-2R, MHC class II, B7, and TCR are commonly measured with fluorescently labeled antibodies using flow cytometry. All antibodies are commercially available. Any change in the expression levels of lymphocyte activation markers when immune responses are generated in the presence of the PDZ/PL interaction antagonists is indicative of an inhibition of this response.

(D) Increased metabolic activity/acid release

Activation of most known signal transduction pathways trigger increases in acidic metabolites. This reproducible biological event is measured as the rate of acid release using a microphysiometer (Molecular Devices), and is used as an early activation marker when comparing the treatment of cells with potential biological therapeutics (McConnell, H.M. et al., 1992, Science 257: 1906-1912 and McConnell, H.M., 1995, Proc. Natl. Acad. Sci. 92: 2750-2754). Any statistically significant increase or decrease in acid release of the PDZ/PL interaction antagonist-treated sample, as compared to control sample (no treatment), suggest an effect of the PDZ/PL interaction antagonist on biological function.

(E) Cell proliferation/cell viability assays

(1) $^3$H-thimidine incorporation

Exposure of lymphocytes to antigen or mitogen in vitro induces DNA synthesis and cellular proliferation. The measurement of mitotic activity by $^3$H-thimidine incorporation into newly synthesized DNA is one of the most frequently used assays to quantitative T cell activation. Depending on the cell population and form of stimulation used to activate the T cells, mitotic activity can be measured within 24-72 hrs. in vitro, post 3H-thimidine pulse (Mishell, B. B. and S. M. Shiigi, 1980, Selected Methods in Cellular Immunology, W. H. Freeman and Company and Dutton, R. W. and Pearce, J. D., 1962, Nature 194: 93). Any statistically significant increase or decrease in CPM of the PDZ/PL interaction antagonist-treated sample, as compared to control sample (no treatment), suggest and effect of the PDZ/PL interaction antagonist on biological function.

(2) MTS [5- (3- carboxymethoxyphenyl)- 2- (4, 5- dimethylthiazolyl)- 3 (4- sulfophenyl) tetrazolium, inner salt] is a colorimetric method for determining the number of viable cells in proliferation or cytotoxicity assays (Barltrop, J.A. et al., 1991, Bioorg. & Med. Chem. Lett. 1: 611) . 1- 5 days after lymphocyte activation, MTS tetrazolium compound, Owen's reagent, is bioreduced by cells into a colored formazan product that is soluble in tissue culture media. Color intensity is read at 490 nm minus 650 nm using a microplate reader. Any statistically significant increase or decrease in color intensity of the PDZ/PL interaction antagonist- treated sample, as compared to control sample (no treatment) , can suggest an effect of the PDZ/PL interaction antagonist on biological function (Mosmann, T., 1983, J. Immunol.

Methods 65: 55 and Barltrop, J.A. et al. (1991) ) .

(3) Bromodeoxyuridine (BrdU), a thymidine analogue, readily incorporates into cells undergoing DNA synthesis. BrdU-pulsed cells are labeled with an enzyme-conjugated anti-BrdU antibody (Gratzner, H.G., 1982, Science 218: 474-475.). A colorimetric, soluble substrate is used to visualize proliferating cells that have incorporated BrdU. Reaction is stopped with sulfuric acid and plate is read at 450 nm using a microplate reader. Any statistically significant increase or decrease in color intensity of the PDZ/PL interaction antagonist-treated sample, as compared to control sample (no treatment), suggest an effect of the PDZ/PL interaction antagonist on biological function.

(F) Apoptosis by Annexin V

Programmed cell death or apoptosis is an early event in a cascade of catabolic reactions leading to cell death. A lose in the integrity of the cell membrane allows for the binding of fluorescently conjugated phosphatidylserine. Stained cells can be measured by fluorescence microscopy and flow cytometry (Vermes, I., 1995, J. Immunol. Methods. 180: 39-52). In one embodiment, any statistically significant increase or decrease in apoptotic cell number of the PDZ/PL interaction antagonist-treated sample, as compared to control sample (no treatment), suggest an effect of the PDZ/PL interaction antagonist on biological function. For evaluating apoptosis in situ, assays for evaluating cell death in tissue samples can also be used in vivo studies.

(G) Quantitation of cytokine production

Cell supernatants harvested after cell stimulation for 16-48 hrs are stored at - 80°C until assayed or directly tested for cytokine production. Multiple cytokine assays can be performed on each sample. IL-2, IL-3, IFN-$\gamma$ and other cytokine ELISA Assays are available for mouse, rat, and human (Endogen, Inc. and BioSource). Cytokine production is measured using a standard two-antibody sandwich ELISA protocol as described by the manufacturer. The presence of horseradish peroxidase is detected with 3, 3'5, 5' tertamethyl benziidine (TMB) substrate and the reaction is stopped with sulfuric acid. The absorbency at 450 nm is measured using a microplate reader. Any statistically significant increase or decrease in color intensity of the PDZ/PL interaction antagonist-treated sample, as compared to control sample (no treatment), suggest an effect of the PDZ/PL interaction antagonist on biological function. See also Example 1, *infra*. Detection of intracellular cytokines using anti-cytokine antibodies provides the additional advantage of measuring cytokines fore mixed cell populations. This allows for phenotyping measuring frequency of cytokine producing cell types in suspension or in tissues.

(H) NF-AT can be visualized by Immunostaining

T cell activation requires the import of nuclear factor of activated T cells (NF-AT) to the nucleus. This translocation of NF-AT can be visualized by immunostaining with anti-NF-AT antibody (Cell 1998, 93: 851-861). Therefore, NF-AT nuclear translocation has been used to assay T cell activation. Similarly, NF-AT/luciferase reporter assays have been used as a standard measurement of T cell activation (MCB 1996, 12: 7151-7160). Any statistically significant increase or decrease in the nuclear translocation of NF-AT brought about by the PDZ/PL interaction antagonist-treated sample, as compared to control sample (no treatment), suggest an effect of the PDZ/PL interaction antagonist on biological function. In order to optimize the use of the peptides and peptide analogues disclosed herein in a human subject, various animal models may be used to define certain clinical parameters. For example, the compounds of the invention may be tested in different dosages, formulations and route of administration in a cardiac transplant mouse model to optimize their ability to inhibit rejection responses to solid organ transplants (Fulmer et al., 1963, Am. J. Anat. 113:273; Jockusch et al., 1983, Exp. Neurol. 81:749).

In situations where inhibition of a T cell response is desired, the compounds of the inventions may be used to inhibit PDZ domain interactions with CD3, CD4, CD6 and CDw137. In addition, the compounds of the invention may be used to inhibit PDZ domain interactions with CD53 and CD138 in B cells. In order to inhibit IgE-mediated allergic reactions, the compounds of the invention may be used to inhibit PDZ domain interactions with Fc$\epsilon$RI$\beta$, CDw125 and CDw128. Furthermore, a PDZ domain binding motif sequence (PL sequence) of CD95 may be used to induce apoptosis of lymphomas.

(I) Inflammatory Mediator Release Assays

Assays are well known in the art for inflammatory mediator release to access the effect of compounds or treatments IgE-mediated degranulation. See, e.g. Berger et al., 1997, Measuring Cell Degranulation e.g., Ch 19.6 Immunology Method Manual. Academic Press, Ltd. 1436-1440 and Siraganian, 1983, Histamine Secretion from Mast Cells and Basophil. TIPS 4:432-437.

6.9. Formulation and Route of Administration

6.9.1 Introduction of Agonists or Antagonists (e.g., Peptides and Fusion Proteins) into Cells

In one aspect, the PDZ-PL antagonists are introduced into a cell to modulate (i.e., increase or decrease) a biological function or activity of the cell. Many small organic molecules readily cross the cell membranes (or can be modified by one of skill using routine methods to increase the ability of compounds to enter cells, e.g., by reducing or eliminating charge, increasing lipophilicity, conjugating the molecule to a moiety targeting a cell surface receptor such that after interacting with the receptor). Methods for introducing larger molecules, e.g., peptides and fusion proteins are also well known, including, e.g., injection, liposome-mediated fusion, application of a hydrogel, conjugation to a targeting moiety conjugate endocytozed by the cell, electroporation, and the like).

In one aspect, the antagonist or agent is a fusion polypeptide or derivatized polypeptide. A fusion or derivatized protein may include a targeting moiety that increases the ability of the polypeptide to traverse a cell membrane or causes the polypeptide to be delivered to a specified cell type (e.g., liver cells or tumor cells) preferentially or cell compartment (e.g., nuclear compartment) preferentially. Examples of targeting moieties include lipid tails, amino acid sequences such as antennapoedia peptide or a nuclear localization signal (NLS; e.g., Xenopus nucleoplasmin Robbins et al., 1991, Cell 64:615).

In one aspect, a peptide sequence or peptide analog determined to inhibit a PDZ domain- PL protein binding, in an assay is introduced into a cell by linking the sequence to an amino acid sequence that facilitates its transport through the plasma membrane (a "transmembrane transporter sequence") . The peptides may be used directly or fused to a transmembrane transporter sequence to facilitate their entry into cells. In the case of such a fusion peptide, each peptide may be fused with a heterologous peptide at its amino terminus directly or by using a flexible polylinker such as the pentamer G- G- G- G- S (SEQ ID NO: 1) repeated 1 to 3 times. Such linker has been used in constructing single chain antibodies (scFv) by being inserted between $V_H$ and $V_L$ (Bird et al., 1988, Science 242: 423- 426; Huston et al., 1988, Proc. Natl. Acad Sci. U.S.A. 85: 5979- 5883) . The linker is designed to enable the correct interaction between two beta- sheets forming the variable region of the single chain antibody. Other linkers which may be used include Glu- Gly- Lys- Ser- Ser- Gly- Ser- Gly- Ser- Glu- Ser- Lys- Val- Asp (SEQ ID NO: 2) (Chaudhary et al., 1990, Proc. Natl. Acad. Sci. U.S.A. 87: 1066- 1070) and Lys- Glu- Ser- Gly- Ser- Val- Ser- Ser- Glu- Gln- Leu- Ala- Gln- Phe- Arg- Ser- Leu- Asp (SEQ ID NO: 3) (Bird et al., 1988, Science 242: 423- 426) .

A number of peptide sequences have been described in the art as capable of facilitating the entry of a peptide linked to these sequences into a cell through the plasma membrane (Derossi et al., 1998, Trends in Cell Biol. 8: 84) . For the purpose of this invention, such peptides are collectively referred to as transmembrane transporter peptides. Examples of these peptide include, but are not limited to, tat derived from HIV (Vives et al., 1997, J. Biol. Chem. 272: 16010; Nagahara et al., 1998, Nat. Med. 4: 1449) , antennapedia from Drosophila (Derossi et al., 1994, J. Biol. Chem. 261: 10444) , VP22 from herpes simplex virus (Elliot and D'Hare, 1997, Cell 88: 223- 233) , complementarity- determining regions (CDR) 2 and 3 of anti- DNA antibodies (Avrarneas et al., 1998, Proc. Natl Acad. Sci. U.S.A., 95: 5601- 5606) , 70 KDa heat shock protein (Fujihara, 1999, EMBO J. 18: 411- 419) and transportan (Pooga et al., 1998, FASEB J. 12: 67- 77) . In a preferred embodiment of the invention, a truncated HIV tat peptide having the sequence of GYGRKKRRQRRRG (SEQ ID NO: 173) is used.

It is preferred that a transmembrane transporter sequence is fused to a hematopoietic cell surface receptor carboxyl terminal sequence at its amino-terminus with or without a linker. Generally, the C-terminus of a PDZ domain binding motif sequence (PL sequence) must be free in order to interact with a PDZ domain. The transmembrane transporter sequence may be used in whole or in part as long as it is capable of facilitating entry of the peptide into a cell.

In an alternate aspect of the disclosure, a hematopoietic cell surface receptor C-terminal sequence may be used alone when it is delivered in a manner that allows its entry into cells in the absence of a transmembrane transporter sequence. For example, the peptide may be delivered in a liposome formulation or using a gene therapy approach by delivering a coding sequence for the PDZ domain binding motif alone or as a fusion molecule into a target cell.

The compounds may also be administered via liposomes, which serve to target the conjugates to a particular tissue, such as lymphoid tissue, or targeted selectively to infected cells, as well as increase the half-life of the peptide composition. Liposomes include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic compositions. Thus, liposomes filled with a desired peptide or conjugate of the invention can be directed to the site of lymphoid cells, where the liposomes then deliver the selected inhibitor compositions. Liposomes for use in the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g.,

Szoka et al., Ann. Rev. Biophys. Bioeng. 9:467 (1980), U.S. Pat. Nos. 4,235,871, 4,501,728 and 4,837,028.

The targeting of liposomes using a variety of targeting agents is well known in the art (see, e.g., U.S. Patent Nos. 4, 957, 773 and 4, 603, 044) . For targeting to the immune cells, a ligand to he incorporated into the liposome can include, e.g., antibodies or fragments thereof specific for cell surface determinants of the desired immune system cells. A liposome suspension containing a peptide or conjugate may be administered intravenously, locally, topically, etc. in a dose which varies according to, inter alia, the manner of administration, the conjugate being delivered, and the stage of the disease being treated.

In order to specifically deliver a PDZ domain binding motif sequence (PL sequence) peptide into a specific cell type, the peptide may be linked to a cell-specific targeting moiety, which include but are not limited to, ligands for diverse leukocyte surface molecules such as growth factors, hormones and cytokines, as well as antibodies or antigen-binding fragments thereof. Since a large number of cell surface receptors have been identified in leukocytes, ligands or antibodies specific for these receptors may be used as cell-specific targeting moieties. For example, interleukin-2, B7-1 (CD80), B7-2 (CD86) and CD40 or peptide fragments thereof may be used to specifically target activated T cells (The Leucocyte Antigen Facts Book, 1997, Barclay et al. (eds.), Academic Press). CD28, CTLA-4 and CD40L or peptide fragments thereof may be used to specifically target B cells. Furthermore, Fc domains may be used to target certain Fc receptor-expressing cells such as monocytes.

Antibodies are the most versatile cell-specific targeting moieties because they can be generated against any cell surface antigen. Monoclonal antibodies have been generated against leukocyte lineage-specific markers such as certain CD antigens. Antibody variable region genes can be readily isolated from hybridoma cells by methods well known in the art. However, since antibodies are assembled between two heavy chains and two light chains, it is preferred that a scFv be used as a cell-specific targeting moiety. Such scFv are comprised of $V_H$ and $V_L$ domains linked into a single polypeptide chain by a flexible linker peptide.

The PDZ domain binding motif sequence (PL sequence) may be linked to a transmembrane transporter sequence and a cell-specific targeting moiety to produce a tri-fusion molecule. This molecule can bind to a leukocyte surface molecule, passes through the membrane and targets PDZ domains. Alternatively, a PDZ domain binding motif sequence (PL sequence) may be linked to a cell-specific targeting moiety that binds to a surface molecule that internalizes the fusion peptide.

In an other approach, microspheres of artificial polymers of mixed amino acids (proteinoids) have been used to deliver pharmaceuticals. For example, U.S. Pat. No. 4,925,673 describes drug-containing proteinoid microsphere carriers as well as methods for their preparation and use. These proteinoid microspheres are useful for the delivery of a number of active agents. Also see, U.S. Patent Nos. 5,907,030 and 6,033,884.

6.9.2 Introduction of Polynucleotides into Cells

A polynucleotide encoding a surface receptor C-terminal peptide may be useful in the treatment of various leukocyte activation-associated abnormal conditions. By introducing gene sequences into cells, gene therapy can be used to treat conditions in which leukocytes are activated to result in deleterious consequences. In one aspect, a polynucleotide that encodes a PL sequence peptide of the invention is introduced into a cell where it is expressed. The expressed peptide then inhibits the interaction of PDZ proteins and PL proteins in the cell.

Thus, in one aspect, the polypeptides of the invention are expressed in a cell by introducing a nucleic acid (e.g., a DNA expression vector or mRNA) encoding the desired protein or peptide into the cell. Expression may be either constitutive or inducible depending on the vector and choice of promoter. Methods for introduction and expression of nucleic acids into a cell are well known in the art and described herein.

In a specific aspect, nucleic acids comprising a sequence encoding a peptide disclosed herein, are administered to a human subject. In this aspect, the nucleic acid produces its encoded product that mediates a therapeutic effect by inhibiting leukocyte activation. Any of the methods for gene therapy available in the art can be used. Exemplary methods are described below.

For general reviews of the methods of gene therapy, see Goldspiel et al., 1993, Clinical Pharmacy 12: 488-505; Wu and Wu, 1991, Biotherapy 3: 87- 95; Tolstoshev, 1993, Ann. Rev. Pharmacol. Toxicol. 32: 573- 596; Mulligan, 1993, Science 260: 926- 932; and Morgan and Anderson, 1993, Ann. Rev. Biochem. 62: 191- 217; May, 1993, TIBTECH 11 (5) : 155- 215. Methods commonly known in the art of recombinant DNA technology which can be used are described in Ausubel et al. (eds.) , 1993, Current Protocols in Molecular Biology, John Wiley & Sons, NY; and Kriegler, 1990, Gene Transfer and Expression, A Laboratory Manual, Stockton Press, NY.

In a preferred aspect, the therapeutic composition comprises a coding sequence that is part of an expression vector. In particular, such a nucleic acid has a promoter operably linked to the coding sequence, said promoter being inducible or constitutive, and, optionally, tissue-specific. In another specific embodiment, a nucleic acid molecule is used in which the coding sequence and any other desired sequences are flanked by regions that promote homologous recombination at a desired site in the genome, thus providing for intrachromosomal expression of the nucleic acid (Koller and Smithies, 1989, Proc. Natl. Acad. Sci. USA 86:8932-8935; Zijlstra et al., 1989, Nature 342:435-438).

Delivery of the nucleic acid into a patient may be either direct, in which case the patient is directly exposed to the nucleic acid or nucleic acid-carrying vector, or indirect, in which case, cells are first transformed with the nucleic acid *in vitro,* then transplanted into the patient. These two approaches are known, respectively, as *in vivo* or *ex vivo* gene therapy.

In a specific aspect, the nucleic acid is directly administered *in vivo*, where it is expressed to produce the encoded product. This can be accomplished by any methods known in the art, *e.g.*, by constructing it as part of an appropriate nucleic acid expression vector and administering it so that it becomes intracellular, *e.g.*, by infection using a defective or attenuated retroviral or other viral vector (see U.S. Patent No. 4, 980, 286) , by direct injection of naked DNA, by use of microparticle bombardment (e.g., a gene gun; Biolistic, Dupont) , by coating with lipids or cell- surface receptors or transfecting agents, by encapsulation in liposomes, microparticles, or microcapsules, by administering it in linkage to a peptide which is known to enter the nucleus, or by administering it in linkage to a ligand subject to receptor-mediated endocytosis (see e.g.,  Wu and Wu, 1987, J. Biol. Chem. 262: 4429- 4432) which can be used to target cell types specifically expressing the receptors. In another aspect, a nucleic acid- ligand complex can be formed in which the ligand comprises a fusogenic viral peptide to disrupt endosomes, allowing the nucleic acid to avoid lysosomal degradation. In yet another aspect, the nucleic acid can be targeted *in vivo* for cell specific uptake and expression, by targeting a specific receptor (see, e.g., PCT Publications WO 92/06180 dated April 16, 1992; WO 92/22635 dated December 23, 1992; WO92/20316 dated November 26, 1992; WO93/14188 dated July 22, 1993; WO 93/20221 dated October 14, 1993) . Alternatively, the nucleic acid can be introduced intracellularly and incorporated within host cell DNA for expression, by homologous recombination (Koller and Smithies, 1989, Proc. Natl. Acad. Sci. USA 86: 8932- 8935; Zijlstra et al., 1989, Nature 342: 435- 438) .

In a preferred aspect, adenoviruses as viral vectors can be used in gene therapy. Adenoviruses have the advantage of being capable of infecting non-dividing cells (Kozarsky and Wilson, 1993, Current Opinion in Genetics and Development 3:499-503). Other instances of the use of adenoviruses in gene therapy can be found in Rosenfeld et al., 1991, Science 252:431-434; Rosenfeld et al., 1992, Cell 68:143-155; and Mastrangeli et al., 1993, J. Clin. Invest. 91: 225-234. Furthermore, adenoviral vectors with modified tropism may be used for cell specific targeting (WO98/40508) Adeno-associated virus (AAV) has also been proposed for use in gene therapy (Walsh et al., 1993, Proc. Soc. Exp. Biol. Med. 204:289-300).

In addition, retroviral vectors (see Miller et al., 1993, Meth. Enzymol. 217:581-599) have been modified to delete retroviral sequences that are not necessary for packaging of the viral genome and integration into host cell DNA. The coding sequence to be used in gene therapy is cloned into the vector, which facilitates delivery of the gene into a patient. More detail about retroviral vectors can be found in Boesen et al., 1994, Biotherapy 6:291-302, which describes the use of a retroviral vector to deliver the *mdr1* gene to hematopoietic stem cells in order to make the stem cells more resistant to chemotherapy. Other references illustrating the use of retroviral vectors in gene therapy are: Clowes et al., 1994, J. Clin. Invest. 93:644-651; Kiem et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Another approach to gene therapy involves transferring a gene to cells in tissue culture. Usually, the method of transfer includes the transfer of a selectable marker to the cells. The cells are then placed under selection to isolate those cells that have taken up and are expressing the transferred gene. Those cells are then delivered to a patient.

In this aspect, the nucleic acid is introduced into a cell prior to administration *in vivo* of the resulting recombinant cell. Such introduction can be carried out by any method known in the art, including but not limited to transfection, electroporation, lipofection, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see e.g., Loeffler and Behr, 1993, Meth. Enzymol. 217:599-618; Cohen et al., 1993, Meth. Enzymol. 217:618-644; Cline, 1985, Pharmac. Ther. 29: 69-92) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny. In a preferred embodiment, the cell used for gene therapy is autologous to the patient.

In a specific aspect, the nucleic acid to be introduced for purposes of gene therapy comprises an inducible promoter operably linked to the coding sequence, such that expression of the nucleic acid is controllable by controlling the presence or absence of the appropriate inducer of transcription.

Oligonucleotides such as anti-sense RNA and DNA molecules, and ribozymes that function to inhibit the translation of a leukocyte surface receptor mRNA, especially its C-terminus are also within the scope of the invention. Antisense RNA and DNA molecules act to directly block the translation of mRNA by binding to targeted mRNA and preventing protein translation. In regard to antisense DNA, oligodeoxyribonucleotides derived from the translation initiation site, *e.g.*, between -10 and +10 regions of a nucleotide sequence, are preferred.

The antisense oligonucleotide may comprise at least one modified base moiety which is selected from the group including, but not limited to, 5- fluorouracil, 5- bromouracil, 5- chlorouracil, 5- iodouracil, hypoxanthine, xanthine, 4- acetylcytosine, 5- (carboxyhydroxylmethyl) uracil, 5- carboxymethylaminomethyl- 2- thiouridine, 5- carboxymethylaminomethyluracil, dihydrouracil, beta- D- galactosylqueosine, inosine, N6- isopentenyladenine, 1- methylguanine, 1-methylinosine, 2, 2- dimethylguanine, 2- methyladenine, 2- methylguanine, 3- methylcytosine, 5- methylcytosine, N6-adenine, 7- methylguanine, 5- methylaminomethyluracil, 5- methoxyaminomethyl- 2- thiouracil, beta- D- mannosylqueosine, 5'- methoxycarboxymethyluracil, 5- methoxyuracil, 2- methylthio- N6- isopentenyladenine, uracil- 5- oxyacetic acid (v) , wybutoxosine, pseudouracil, queosine, 2- thiocytosine, 5- methyl- 2- thiouracil, 2- thiouracil, 4- thiouracil, 5-methyluracil, uracil- 5- oxyacetic acid methylester, uracil- 5- oxyacetic acid (v) , 5- methyl- 2- thiouracil, 3- (3- amino- 3-N- 2- carboxypropyl) uracil, (acp3) w, and 2, 6- diaminopurine.

Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Within the scope of the invention are engineered hammerhead motif ribozyme molecules that specifically and efficiently catalyze endonucleolytic cleavage of leukocyte surface receptor RNA sequences.

Specific ribozyme cleavage sites within any potential RNA target are initially identified by scanning the target molecule for ribozyme cleavage sites which include the following sequences, GUA, GUU and GUC. Once identified, short RNA sequences of between 15 and 20 ribonucleotides corresponding to the region of the target gene containing the cleavage site may be evaluated for predicted structural features such as secondary structure that may render the oligonucleotide sequence unsuitable. The suitability of candidate targets may also be evaluated by testing their accessibility to hybridization with complementary oligonucleotides, using ribonuclease protection assays.

The anti-sense RNA and DNA molecules and ribozymes may be prepared by any method known in the art for the synthesis of nucleic acid molecules. These include techniques for chemically synthesizing oligodeoxyribonucleotides well known in the art such as for example solid phase phosphoramidite chemical synthesis. Alternatively, RNA molecules may be generated by *in vitro* and *in vivo* transcription of DNA sequences encoding the RNA molecule. Such DNA sequences may be incorporated into a wide variety of vectors which contain suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Various modifications to the DNA molecules may be introduced as a means of increasing intracellular stability and half-life. Possible modifications include, but are not limited to, the addition of flanking sequences of ribo- or deoxy-nucleotides to the 5' and/or 3' ends of the molecule or the use of phosphorothioate or 2' O-methyl rather than phosphodiesterase linkages within the oligodeoxyribonucleotide backbone.

6.9.3 Other Pharmaceutical Compositions

The compounds described herein may be administered to a subject *per se* or in the form of a sterile composition or a pharmaceutical composition. Pharmaceutical compositions comprising the compounds of the invention may be manufactured by means of conventional mixing, dissolving, granulating, dragee-making, levigating, emulsifying, encapsulating, entrapping or lyophilizing processes. Pharmaceutical compositions may be formulated in conventional manner using one or more physiologically acceptable carriers, diluents, excipients or auxiliaries that facilitate processing of the active peptides or peptide analogues into preparations which can be used pharmaceutically. Proper formulation is dependent upon the route of administration chosen.

For topical administration the compounds described herein may be formulated as solutions, gels, ointments, creams, suspensions, etc. as are well-known in the art.

Systemic formulations include those designed for administration by injection, e.g. subcutaneous, intravenous, intramuscular, intrathecal or intraperitoneal injection, as well as those designed for transdermal, transmucosal, oral or pulmonary administration.

For injection, the compounds described herein may be formulated in aqueous solutions, preferably in physiologically compatible buffers such as Hanks's solution, Ringer's solution, or physiological saline buffer. The solution may contain formulatory agents such as suspending, stabilizing and/or dispersing agents.

Alternatively, the compounds may be in powder form for constitution with a suitable vehicle, e.g., sterile pyrogen-free water, before use.

For transmucosal administration, penetrants appropriate to the barrier to be permeated are used in the formulation. Such penetrants are generally known in the art. This route of administration may be used to deliver the compounds to the nasal cavity.

For oral administration, the compounds can be readily formulated by combining the active peptides or peptide analogues with pharmaceutically acceptable barriers well known in the art. Such carriers enable the compounds of the invention to be formulated as tablets, pills, dragees, capsules, liquids, gels, syrups, slurries, suspensions and the like, for oral ingestion by a patient to be treated. For oral solid formulations such as, for example, powders, capsules and tablets, suitable excipients include fillers such as sugars, such as lactose, sucrose, mannitol and sorbitol; cellulose preparations such as maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP); granulating agents; and binding agents. If desired, disintegrating agents may be added, such as the cross-linked polyvinylpyrrolidone, agar, or alginic acid or a salt thereof such as sodium alginate.

If desired, solid dosage forms may be sugar-coated or enteric-coated using standard techniques.

For oral liquid preparations such as, for example, suspensions, elixirs and solutions, suitable carriers, excipients or diluents include water, glycols, oils, alcohols, etc. Additionally, flavoring agents, preservatives, coloring agents and the like may be added.

For buccal administration, the compounds may take the form of tablets, lozenges, etc. formulated in conventional manner.

For administration by inhalation, the compounds for use according to the present disclosure are conveniently delivered in the form of an aerosol spray from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

The compounds may also be formulated in rectal or vaginal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

Alternatively, other pharmaceutical delivery systems may be employed. Liposomes and emulsions are well known examples of delivery vehicles that may be used to deliver peptides and peptide analogues. Certain organic solvents such as dimethylsulfoxide also may be employed, although usually at the cost of greater toxicity. Additionally, the compounds may be delivered using a sustained- release system, such as semipermeable matrices of solid polymers containing the therapeutic agent. Various of sustained- release materials have been established and are well known by those skilled in the art. Sustained- release capsules may, depending on their chemical nature, release the compounds for a few weeks up to over 100 days. Depending on the chemical nature and the biological stability of the therapeutic reagent, additional strategies for protein stabilization may be employed.

As the compounds described herein may contain charged side chains or termini, they may be included in any of the above-described formulations as the free acids or bases or as pharmaceutically acceptable salts. Pharmaceutically acceptable salts are those salts which substantially retain the biologic activity of the free bases and which are prepared by reaction with inorganic acids. Pharmaceutical salts tend to be more soluble in aqueous and other protic solvents than are the corresponding free base forms.

6.10. <u>Effective Dosages</u>

The compounds described herein will generally be used in an amount effective to achieve the intended purpose. For use to inhibit leukocyte activation-associated disorders, the compounds of the invention or pharmaceutical compositions thereof, are administered or applied in a therapeutically effective amount. By therapeutically effective amount is meant an amount effective ameliorate or prevent the symptoms, or prolong the survival of, the patient being treated. Determination of a therapeutically effective amount is well within the capabilities of those skilled in the art, especially in light of the detailed disclosure provided herein. An "inhibitory amount" or "inhibitory concentration" of a PL-PDZ binding inhibitor is an amount that reduces binding by at least about 40%, preferably at least about 50%, often at least about 70%, and even as much as at least about 90%. Binding can as measured *in vitro* (e.g., in an A assay or G assay) or in situ.

For systemic administration, a therapeutically effective dose can be estimated initially from *in vitro* assays. For example, a dose can be formulated in animal models to achieve a circulating concentration range that includes the $IC_{50}$ as determined in cell culture (*i.e*., the concentration of test compound that inhibits 50% of leukocyte surface receptor-PDZ domain-containing protein interactions). Such information can be used to more accurately determine useful doses in humans.

Initial dosages can also be estimated from *in vivo* data, e.g., animal models, using techniques that are well known in the art. One having ordinary skill in the art could readily optimize administration to humans based on animal data.

Dosage amount and interval may be adjusted individually to provide plasma levels of the compounds that are sufficient to maintain therapeutic effect. Usual patient dosages for administration by injection range from about 0.1 to 5 mg/kg/day, preferably from about 0.5 to 1 mg/kg/day. Therapeutically effective serum levels may be achieved by administering multiple doses each day.

In cases of local administration or selective uptake, the effective local concentration of the compounds may not be related to plasma concentration. One having skill in the art will be able to optimize therapeutically effective local dosages without undue experimentation.

The amount of compound administered will, of course, be dependent on the subject being treated, on the subject's weight, the severity of the affliction, the manner of administration and the judgment of the prescribing physician.

The therapy may be repeated intermittently while symptoms detectable or even when they are not detectable. The therapy may be provided alone or in combination with other drugs. In the case of conditions associated with leukocyte activation such as transplantation rejection and autoimmunity, the drugs that may be used in combination with the compounds of the invention include, but are not limited to, steroid and non-steroid anti-inflammatory agents.

6.10.1 Toxicity

Preferably, a therapeutically effective dose of the compounds described herein will provide therapeutic benefit without causing substantial toxicity.

Toxicity of the compounds described herein can be determined by standard pharmaceutical procedures in cell cultures or experimental animals, e.g., by determining the $LD_{50}$ (the dose lethal to 50% of the population) or the $LD_{100}$ (the dose lethal to 100% of the population). The dose ratio between toxic and therapeutic effect is the therapeutic index. Compounds which exhibit high therapeutic indices are preferred. The data obtained from these cell culture assays and animal studies can be used in formulating a dosage range that is not toxic for use in human. The dosage of the compounds described herein lies preferably within a range of circulating concentrations that include the effective dose with little or no toxicity. The dosage may vary within this range depending upon the dosage form employed and the route of administration utilized. The exact formulation, route of administration and dosage can be chosen by the individual physician in view of the patient's condition. (*See, e.g.,* Fingl et al., 1975, In: The Pharmacological Basis of Therapeutics, Ch.1, p.1).

6.11. **EXAMPLE 1**: TAT-T CELL SURFACE RECEPTOR CARBOXYL TERMINUS FUSION PEPTIDES INHIBITED T CELL ACTIVATION

6.11.1. Materials And Methods

6.11.1.1. Peptide Synthesis

All peptides were chemically synthesized by standard procedures. The Tat- CD3 carboxyl terminus fusion peptide, (GYGRKKRRQRRRGPPSSSSGL, SEQ ID NO: 174) ; Tat- CLASP1 carboxyl terminus fusion peptide, (GYGRKKRRQRRRGSISSSAEV, SEQ ID NO: 175) ; Tat- CLASP2 carboxyl terminus fusion peptide, (GYGRKKRRQRRRGMTSSSSVV, SEQ ID NO: 176) ; and Tat peptide, (GYGRKKRRQRRRG, SEQ ID NO: _) ; were dissolved at 1 mM in PBS, pH 7, or dH2O. Stock MBPAc1- 16 peptide, (AcASQKRPSQRHGSKYLA) , was dissolved at 5 mM. All peptides were aliquoted and stored at- 80°C until tested.

6.11.1.2 Cell Cultures

Cells were maintained and tested in RPMI 1640 media supplemented with 10% fetal calf serum (HyClone), 2 mM glutamine, 10 mM Hepes, 100 U/ml penicillin, 100 μg/ml streptomycin, 0.1 mM non-essential amino acids, 1 mM sodium pyruvate, and 50 μM beta mercaptoethanol.

6.11.1.3 T Cell Stimulation Assay

Supernatants were assayed for cytokine production following activation of T cell lines. Mouse T cell lines were stimulated using two different methods, either with antigen and antigen presenting cells or anti-mouse CD3.

Antigen- specific mouse T cells, BR4.2, were activated with the N- terminal 16 amino acid sequences of myelin basic protein (MBPAc1- 16) and syngenic mouse splenocytes in 96- well plates. Mitomycin C- treated antigen presenting cells, 2 x $10^5$ B10.BR, were added to each row of serially diluted MBPAc1- 16 ranging from 0 to 200 μM. Next, 10 μM Tat- peptides or media alone was added to each row. Finally, 2 x $10^4$ MBPAc1- 16- specific T cell, pre- loaded with 10 μM Tat- peptides (see above) , were added to all wells (Rabinowitz et al., 1997, . Proc. Natl. Acad. Sci. U.S.A., 94: 8702-8707) . Cells were activated during an overnight incubation at 5% CO2, 37°C. Cell supernatant was collected and stored at- 80°C until assayed for cytokine production. The final volume was 200 μl/ well.

Antibody against mouse CD3 (Pharmigen #145-2C11) was coated overnight at 4°C using 96-well flat bottom Elisa plates at a final concentration of 0.5 μg/ml, diluted in PBS. Just prior to use, plates were washed three times with

200 μl/well PBS to remove excess anti-CD3. To ensure that cells were given sufficient time to transduce Tat-peptides before activation, T cells ($5x10^5$ cells/ml) were pre-treated with or without 10 μM Tat-peptides for two hours at 5% CO2, 37°C and then diluted in media with or without 10 μM Tat-peptides to a final concentration of $2x10^4$ cells per well in a final volume of 200 μl. Cells were then treated as described above.

6.11.1.4 Cytokine ELISA

IFNγ was measured from cell supernatants, described above, at ambient temperature using the Endogen, Inc. ELISA protocol 3. Briefly, 96-well, flat bottom, high binding ELISA plates were preincubated overnight with coating antibody (MM700). Plates were washed with 50 mM TRIS, 0.2% tween-20, pH 8 and they blocked for one hour with PBS plus 2% BSA. Washed plates were then incubated one hour with 25 μl of cell supernatant and 25 μl blocking buffer, or with 50 μl IFNγ standard. The presence of IFNγ was detected with a biotin-labeled anti-mouse IFNγ monoclonal antibody (MM700B, Endogen, Inc.,). Quantitative amounts of detection antibody are revealed with horseradishperoxidase-conjugated streptavidin. The enzymatic, color, substrate for HRP, tetramethylbenzidine (TMB), was developed for up to 30 minutes and stopped with 1.0 M $H_2SO_4$. The absorbance at 450 nm was measured using a microtiter plate reader (Thermo Max, Molecular Devices) and the concentration of unknown IFNγ from cell supernatants was calculated from a standard curve generated by Softmax Pro. software (Molecular Devices).

6.11.2 Results

Peptides containing Tat transporter sequences linked to C-terminal sequences of various PLs were testing for their ability to inhibit T cell activation. **FIGURE 4A** shows that the Tat-CD3 fusion peptide inhibits T cell activation mediated by peptide:MHC as compared to controls of Tat-peptide alone or no peptide. **FIGURE 4B** shows that Tat-CLASP2 carboxyl terminus fusion peptide inhibited T cell activation mediated by monoclonal anti-CD3 as compared to Tat-peptide alone. Tat-CLASP1 fusion peptide did not inhibit T cell activation in this experiment. These results indicate that peptides containing potential inhibitory sequences can be transported into T cells through transporter peptide such as Tat to disrupt surface receptor organization mediated by PDZ proteins. Disruption of PDZ-mediated surface receptor organization leads to blockage of T cell activation in response to antigen.

6.12. EXAMPLE 2: DESIGN OF AN INHIBITOR OF DLG 1-LIGAND BINDING WITH GREATER THAN 100 Um POTENCY

A GST/DLG1 fusion protein (See **TABLE 3**) and a biotin-labeled peptide corresponding to the C-terminal 20 amino acids of the CLASP-2 protein, peptide AA2L (See **TABLE 4**), were synthesized and purified by standard techniques well known in the art as described supra. This PDZ-ligand combination was then shown to bind specifically using both the "A" assay and the "G" assay (See **TABLE 2**). Once specific binding was demonstrated, the apparent affinity of the binding interaction was determined using Approach 1 of the section entitled "Measurement of PDZ-ligand binding affinity" (see **FIGURE 2A**). The measured apparent affinity was 21 uM. This implies that 21 uM labeled CLASP-2 peptide AA2L filled 50% of the binding sites for CLASP-2 on DLG1. Thus, 21 uM unlabeled CLASP-2 peptide should be able to block the binding of a given ligand to DLG1 by approximately 50%, assuming that the given ligand (1) binds to the same site (s) on DLG1 as Qasp~2 and (2) is not added at sufficient concentration to reduce significantly the binding of the CLASP-2 peptide (i.e. cannot out-compete the CLASP-2 peptide).

To detect such inhibition, it was necessary to synthesize an analogue of the CLASP2peptide AA2L that (1) retained similar DLG1 binding properties and (2) would not itself generate a signal in the assay selected to measure inhibition. Because most molecular interactions between PDZ proteins and their ligands involve only the C-terminal 6 amino acids of the ligand, an eight amino acid variant of the CLASP-2 peptide, MTSSSSVV, was anticipated to retain similar DLG1 binding properties as the 20 amino acid AA2L CLASP-2 peptide. This eight amino acid CLASP-2 peptide (lacking a functional label) was therefore synthesized and purified by standard techniques as described *supra.* When 100 uM of the (functionally unlabeled) eight amino acid CLASP-2 peptide and 20 uM of the biotin-labeled AA2L CLASP-2 peptide were added simultaneously to DLG1 in a variant of the "G" assay (described supra), the binding of the labeled AA2L CLASP-2 peptide was, as predicted, inhibited by greater than 50% (**FIGURE 3A**). An analogous experiment in which the labeled AA2L CLASP-2 peptide was replaced with another labeled DLG1 ligand, labeled AAI3L Fas peptide demonstrated similar inhibition by the eight amino acid CLASP-2 peptide (**FIGURE 3A**). Thus, an effective inhibitor of DLG1-ligand binding (i.e. the eight amino acid CLASP-2 peptide MTSSSSVV) with a known potency range (order of magnitude 21 uM) was designed based on knowledge of the affinity, 21 uM, with which a particular labeled ligand, the CLASP-2 peptide AA2L, bound to DLG1.

The present invention is not to be limited in scope by the exemplified embodiments which are intended as illustrations of single aspects of the invention and any sequences which are functionally equivalent are within the scope of the invention. Indeed, various modifications of the invention in addition to those shown and described herein will become apparent to those skilled in the art from the foregoing description and accompanying drawings. Such modifications are intended to fall within the scope of the appended claims.

## Claims

**1.** A fusion peptide or peptide analogue thereof for modulating the binding of a PDZ protein and a PL protein comprising:

(i) a transmembrane transporter amino acid sequence capable of facilitating the entry of the peptide linked thereto into a cell through the plasma membrane,
(ii) an optional, flexible polylinker peptide sequence, and
(iii) a carboxyl terminal peptide sequence comprising a PDZ domain binding motif at the C-terminal, the PDZ domain binding motif sequence comprising
X*-S/T-D/E-V/I/L, wherein X* is any non-aromatic amino acid.

**2.** The fusion peptide or peptide analogue of claim 1, wherein the modulating activity functions to inhibit the binding of the PDZ protein to the PL protein.

**3.** The fusion peptide or peptide analogue of claim 2, wherein the C-terminal sequence comprises either of the sequences E-S-D-V or E-T-D-V.

**4.** The fusion peptide or peptide analogue of any of claims 1 to 3, wherein the carboxyl terminal peptide sequence comprises from 4 to 15 amino acids.

**5.** The fusion peptide or peptide analogue of any of claims 1 to 3, wherein the transmembrane transporter amino acid sequence is selected from (a) tat derived from HIV, (b) antennapedia from Drosophila, and (c) VP22 from herpes simplex.

**6.** The fusion peptide or peptide analogue of claim 5, wherein the carboxyl terminal peptide sequence comprises from 4 to 15 amino acids.

**7.** The fusion peptide or peptide analogue of any of claims 1 to 3, wherein the transmembrane transporter amino acid sequence is a tat peptide comprising the sequence GYGRKKRRQRRRG.

**8.** The fusion peptide or peptide analogue of claim 3, wherein the transmembrane transporter amino acid sequence is a tat sequence derived from HIV, and wherein the carboxyl terminal peptide sequence contains from 4 to 15 amino acids.

## Patentansprüche

**1.** Ein Fusionspeptid oder ein Peptidanalogon von diesem zum Modulieren der Bindung eines PDZ-Proteins und eines PL-Proteins, umfassend:

(i) eine Transmembrantransporter-Aminosäuresequenz, die in der Lage ist, den Eintritt eines Peptids, das damit verknüpft ist, in eine Zelle durch die Plasmamembran zu erleichtern,
(ii) eine fakultative, flexible Polylinker-Peptidsequenz und
(iii) eine carboxylterminale Peptidsequenz, die ein PDZ-Domänen-Bindungsmotiv an dem C-Terminus umfasst, wobei die PDZ-Domänen-Bindungsmotivsequenz X*-S/T-D/E-V/I/L umfasst, wobei X* irgendeine nicht aromatische Aminosäure ist.

**2.** Das Fusionspeptid oder Peptidanalogon nach Anspruch 1, wobei die modulierende Aktivität so funktioniert, dass die Bindung des PDZ-Proteins an das PL-Protein gehemmt wird.

**3.** Das Fusionspeptid oder Peptidanalogon nach Anspruch 2, wobei die C-terminale Sequenz eine der Sequenzen E-S-D-V oder E-T-D-V umfasst.

4. Das Fusionspeptid oder Peptidanalogon nach irgendeinem der Ansprüche 1 bis 3, wobei die carboxylterminale Peptidsequenz von 4 bis 15 Aminosäuren umfasst.

5. Das Fusionspeptid oder Peptidanalogon nach irgendeinem der Ansprüche 1 bis 3, wobei die Transmembrantransporter-Aminosäuresequenz ausgewählt ist aus (a) tat, das von HIV abgeleitet ist, (b) Antennapedia aus Drosophila und (c) VP22 aus Herpes simplex.

6. Das Fusionspeptid oder Peptidanalogon nach Anspruch 5, wobei die carboxylterminale Peptidsequenz von 4 bis 15 Aminosäuren umfasst.

7. Das Fusionspeptid oder Peptidanalogon nach irgendeinem der Ansprüche 1 bis 3, wobei die Transmembrantransporter-Aminosäuresequenz ein tat-Peptid ist, das die Sequenz GYGRKKRRQRRRG umfasst.

8. Das Fusionspeptid oder Peptidanalogon nach Anspruch 3, wobei die Transmembrantransporter-Aminosäuresequenz eine tat-Sequenz ist, die von HIV abgeleitet ist, und wobei die carboxylterminale Peptidsequenz von 4 bis 15 Aminosäuren enthält.

**Revendications**

1. Peptide de fusion ou analogue peptidique de celui-ci pour moduler la liaison d'une protéine PDZ et d'une protéine PL, comprenant :

   (i) une séquence d'acides aminés d'un transporteur transmembranaire capable de faciliter l'entrée d'un peptide lié à celui-ci dans une cellule à travers la membrane plasmatique,
   (ii) une séquence peptidique à lieur multisite flexible facultative, et
   (iii) une séquence peptidique carboxy-terminale comprenant un motif de liaison à un domaine PDZ au niveau de l'extrémité C-terminale, la séquence du motif de liaison au domaine PDZ comprenant X*-S/T-D/E-V/I/L, dans lequel X* est un quelconque acide aminé non aromatique.

2. Peptide de fusion ou analogue peptidique selon la revendication 1, dans lequel l'activité de modulation sert à inhiber la liaison de la protéine PDZ à la protéine PL.

3. Peptide de fusion ou analogue peptidique selon la revendication 2, dans lequel la séquence C-terminale comprend l'une quelconque des séquences E-S-D-V ou E-T-D-V.

4. Peptide de fusion ou analogue peptidique selon l'une quelconque des revendications 1 à 3, dans lequel la séquence peptidique carboxy-terminale comprend entre 4 et 15 acides aminés.

5. Peptide de fusion ou analogue peptidique selon l'une quelconque des revendications 1 à 3, dans lequel la séquence d'acides aminés du transporteur transmembranaire est choisie parmi (a) tat dérivée du VIH, (b) antennapedia de la drosophile et (c) VP22 de l'herpès.

6. Peptide de fusion ou analogue peptidique selon la revendication 5, dans lequel la séquence peptidique carboxy-terminale comprend de 4 à 15 acides aminés.

7. Peptide de fusion ou analogue peptidique selon l'une quelconque des revendications 1 à 3, dans lequel la séquence d'acides aminés du transporteur transmembranaire est un peptide tat comprenant la séquence GYGRKKRRQRRRG.

8. Peptide de fusion ou analogue peptidique selon la revendication 3, dans lequel la séquence d'acides aminés du transporteur transmembranaire est une séquence tat dérivée du VIH, et dans lequel la séquence peptidique carboxy-terminale contient de 4 à 15 acides aminés.

FIG. 1A

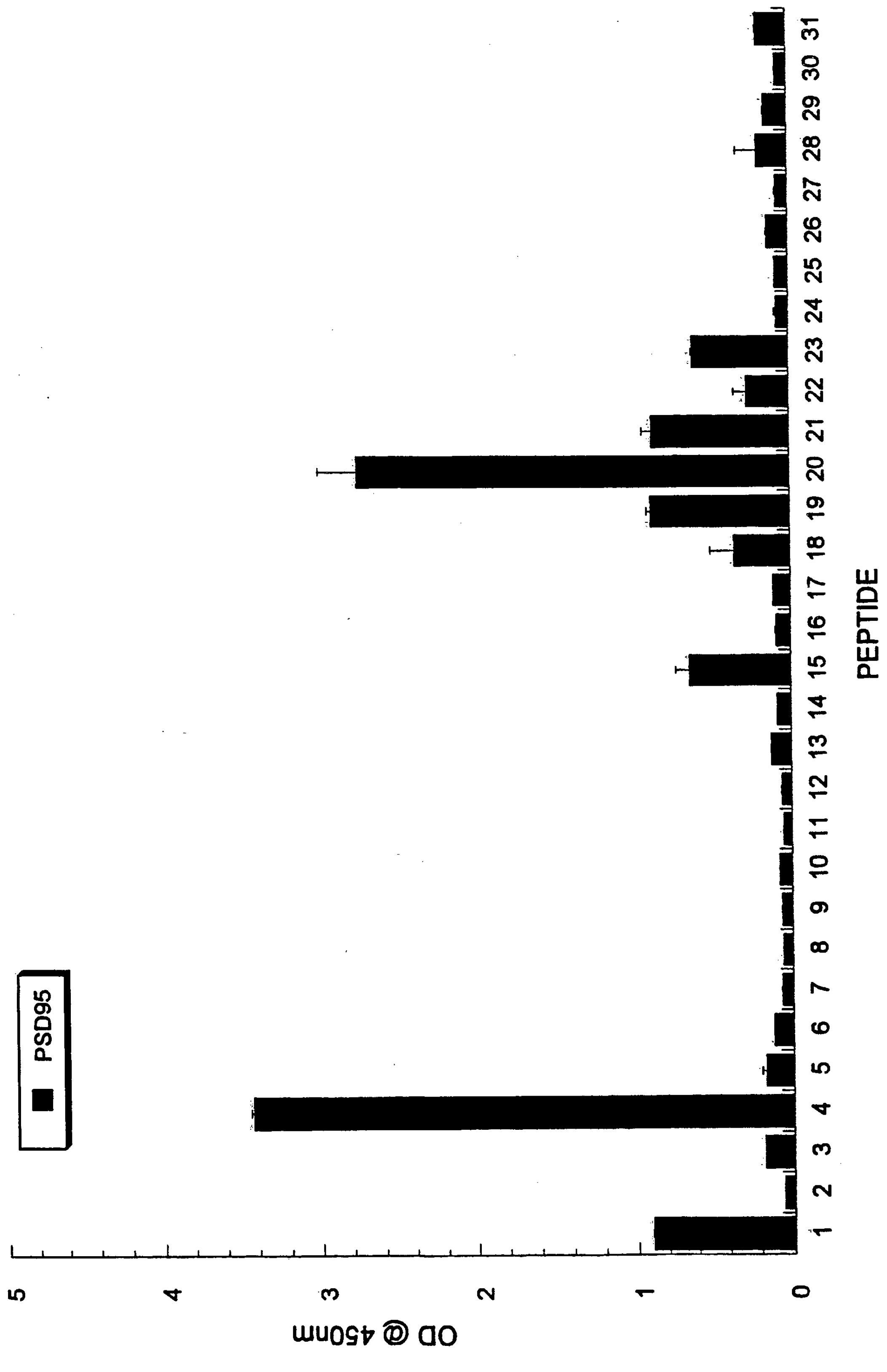
PSD95
OD @ 450nm
0
1
2
3
4
5
PEPTIDE
1 2 3 4 5 6 7 8 9 10 11 12 13 14 15 16 17 18 19 20 21 22 23 24 25 26 27 28 29 30 31

FIG. 1B

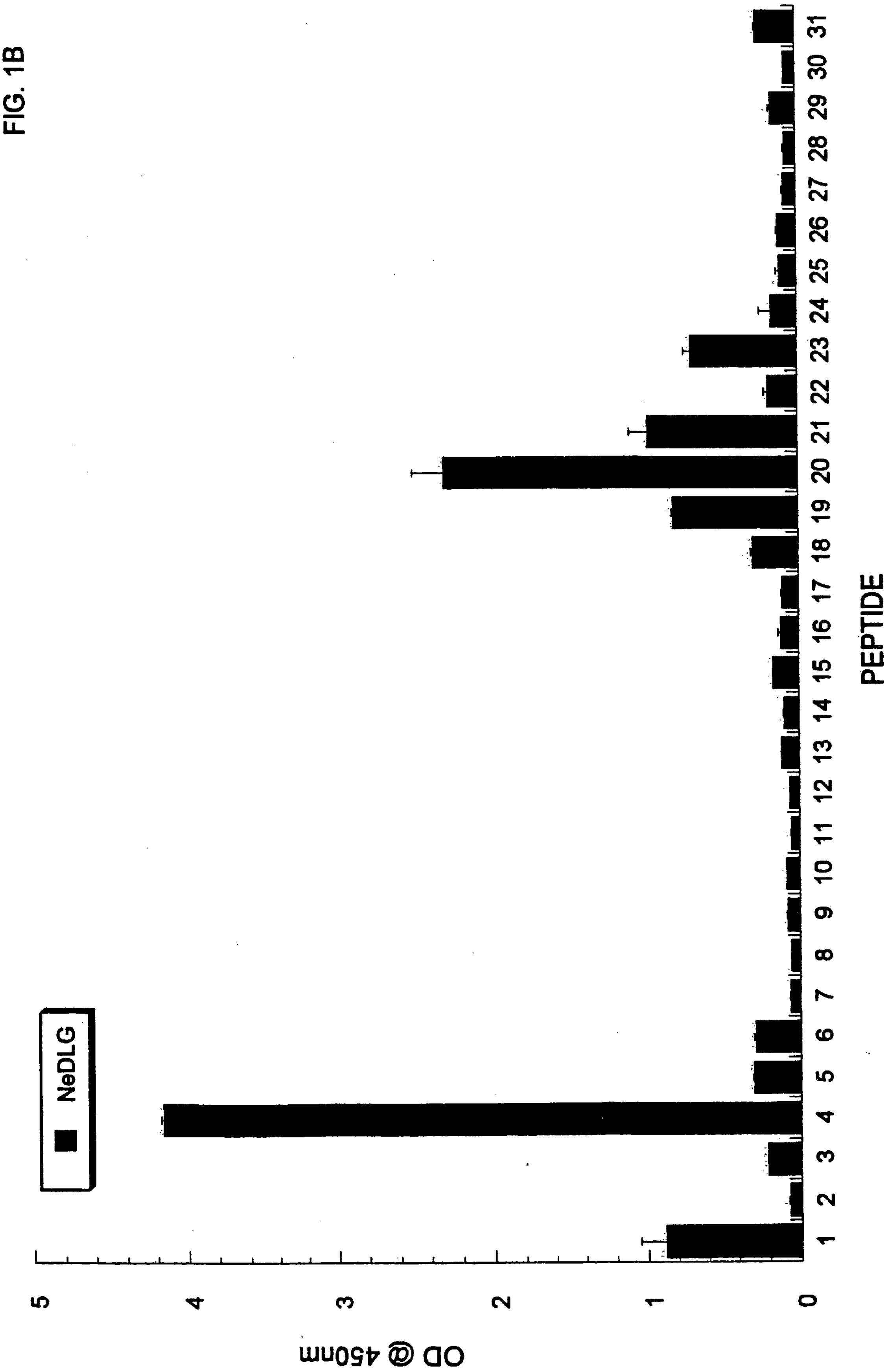
NeDLG
OD @ 450nm
0
1
2
3
4
5
PEPTIDE
1 2 3 4 5 6 7 8 9 10 11 12 13 14 15 16 17 18 19 20 21 22 23 24 25 26 27 28 29 30 31

FIG. 1C

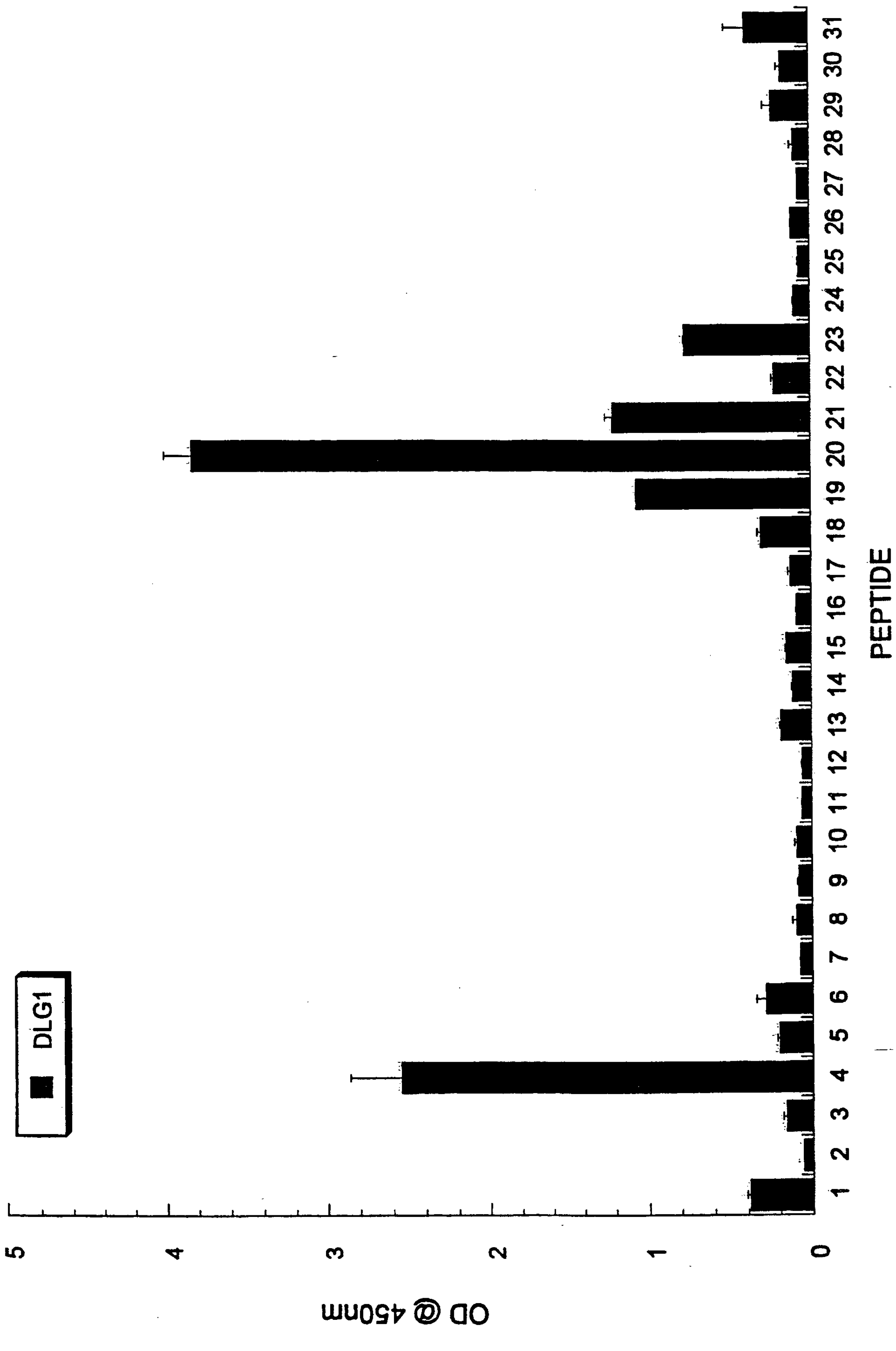
DLG1
OD @ 450nm
0
1
2
3
4
5
PEPTIDE
1 2 3 4 5 6 7 8 9 10 11 12 13 14 15 16 17 18 19 20 21 22 23 24 25 26 27 28 29 30 31

FIG. 1D

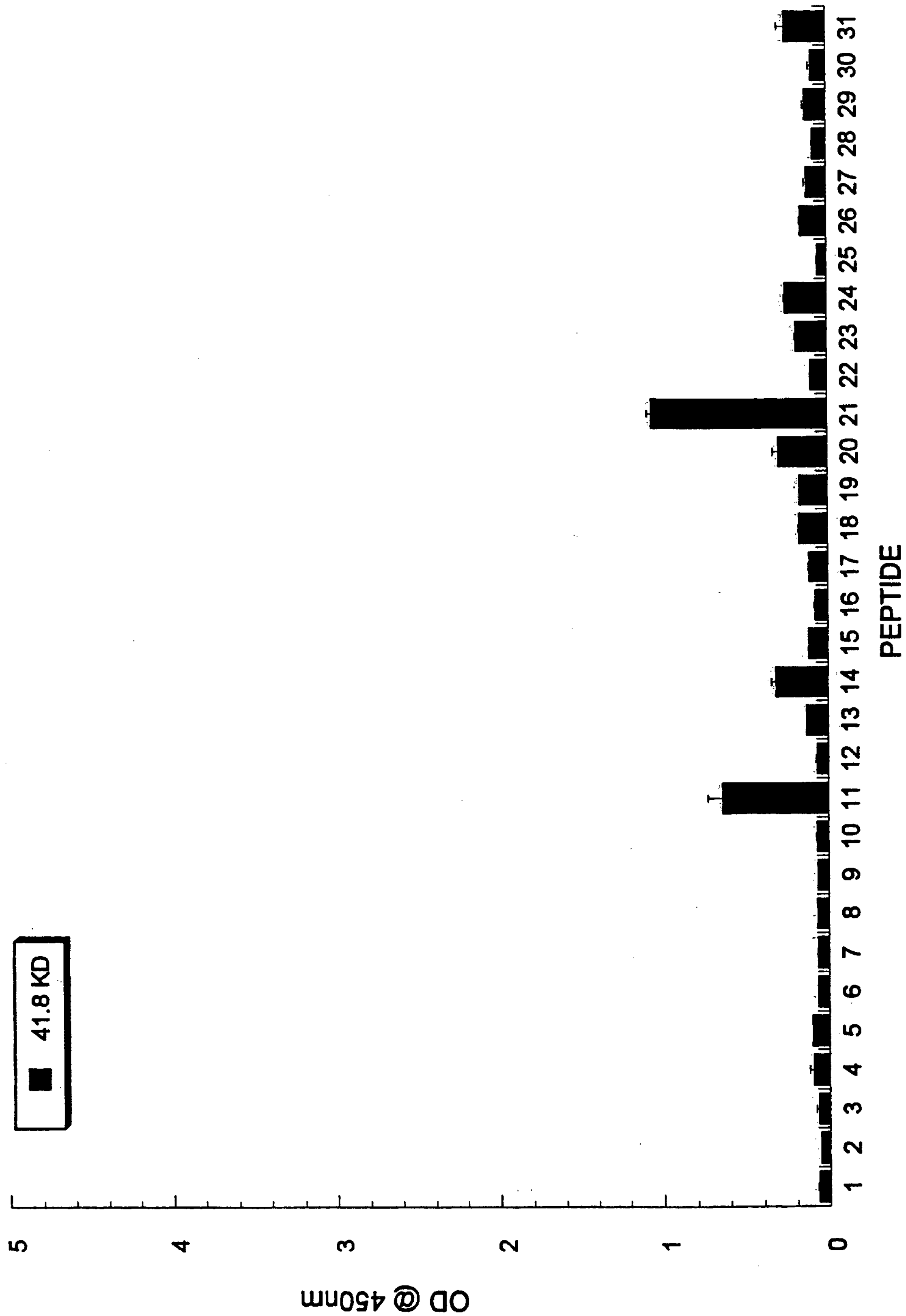
41.8 KD
OD @ 450nm
0
1
2
3
4
5
PEPTIDE
1 2 3 4 5 6 7 8 9 10 11 12 13 14 15 16 17 18 19 20 21 22 23 24 25 26 27 28 29 30 31

FIG. 2A

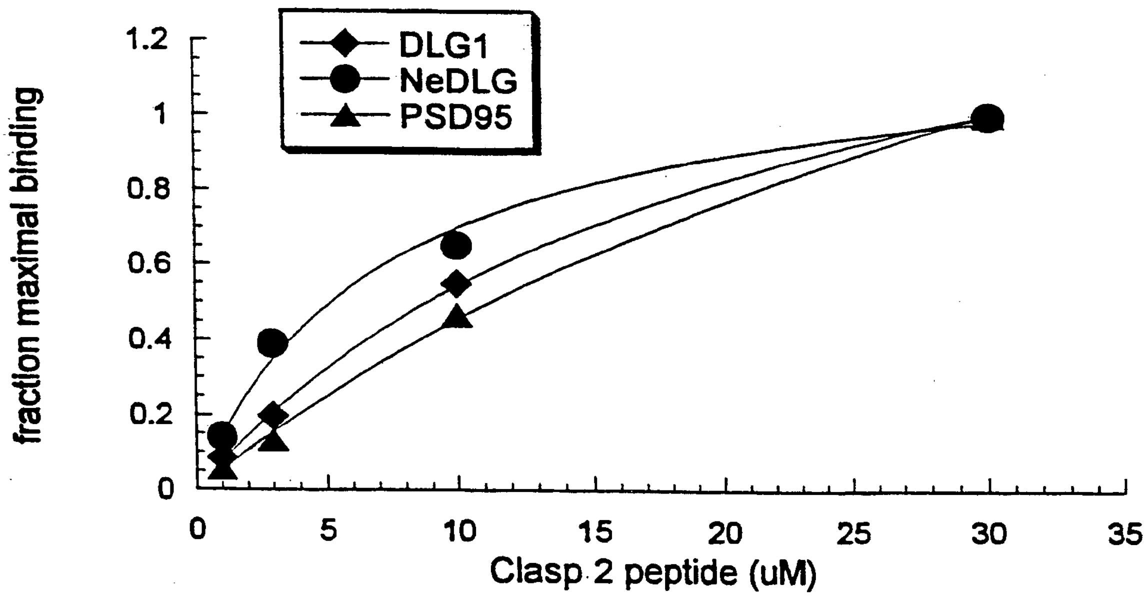
DLG1
NeDLG
PSD95
fraction maximal binding
1.2
1
0.8
0.6
0.4
0.2
0
0
5
10
15
20
25
30
35
Clasp 2 peptide (uM)

FIG. 2B

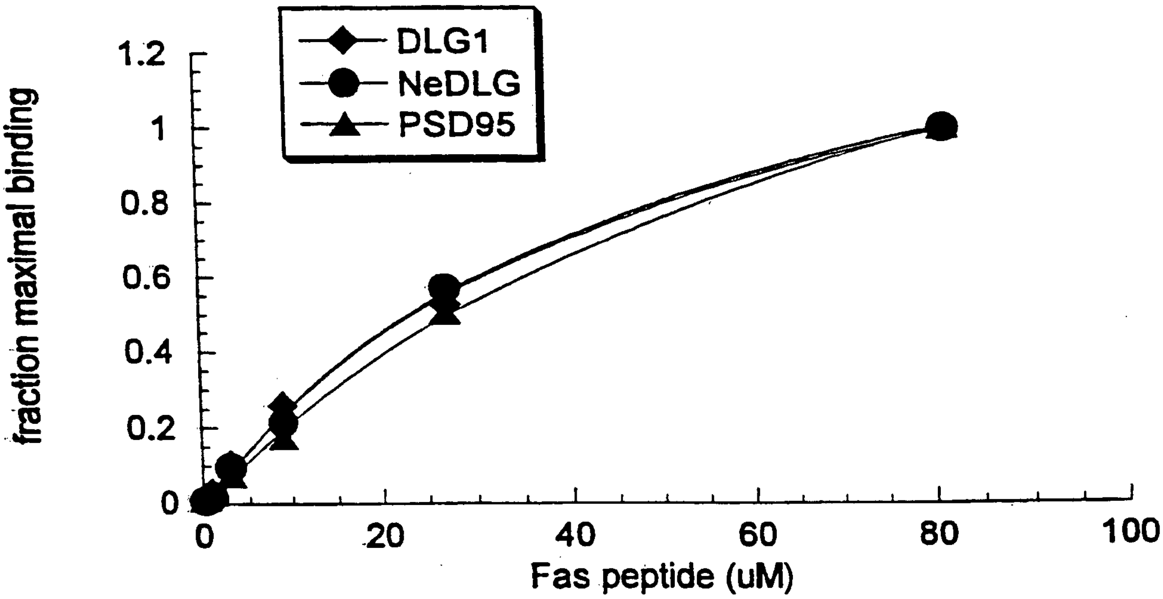
DLG1
NeDLG
PSD95
fraction maximal binding
1.2
1
0.8
0.6
0.4
0.2
0
0
20
40
60
80
100
Fas peptide (uM)

FIG. 3A

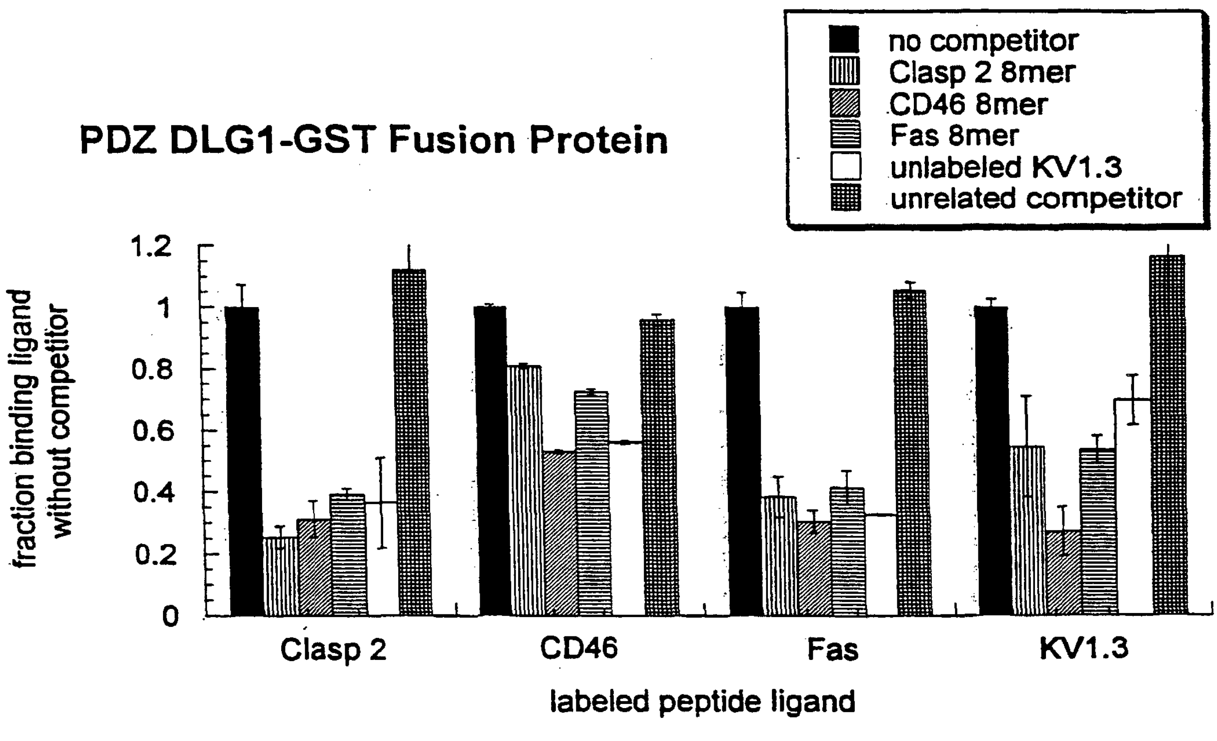
PDZ DLG1-GST Fusion Protein
no competitor
Clasp 2 8mer
CD46 8mer
Fas 8mer
unlabeled KV1.3
unrelated competitor
fraction binding ligand without competitor
1.2
1
0.8
0.6
0.4
0.2
0
Clasp 2
CD46
Fas
KV1.3
labeled peptide ligand

FIG. 3B

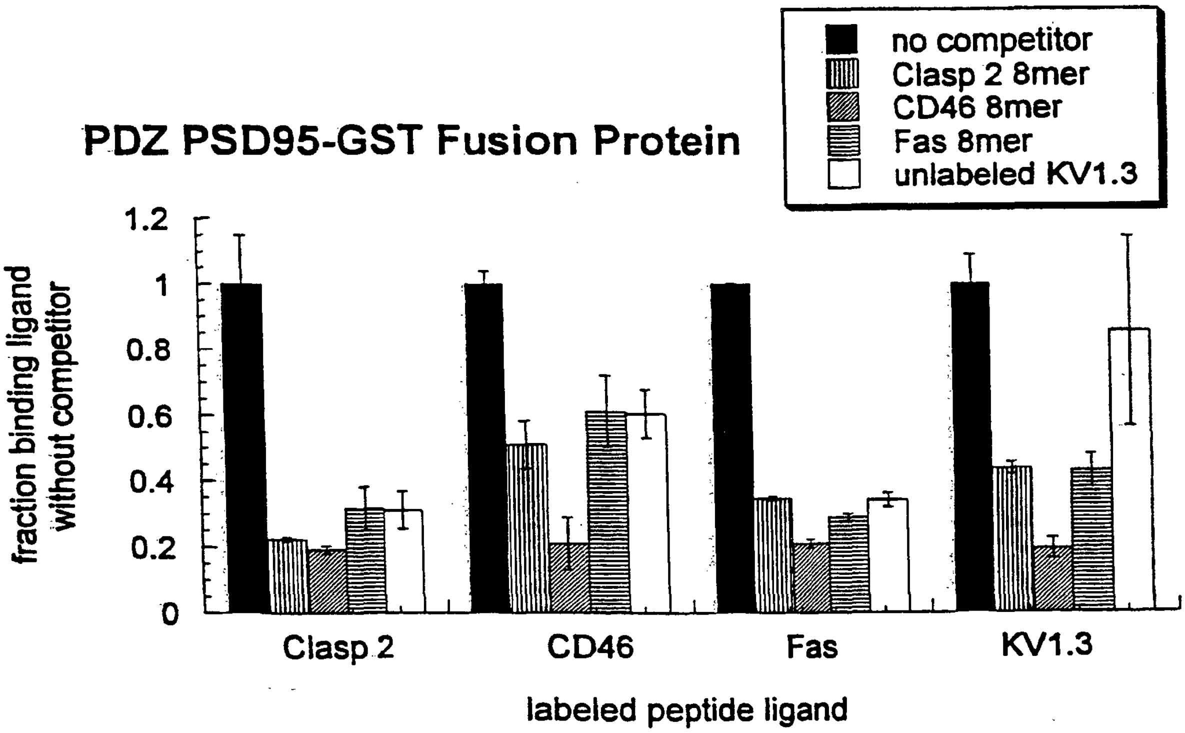
PDZ PSD95-GST Fusion Protein
no competitor
Clasp 2 8mer
CD46 8mer
Fas 8mer
unlabeled KV1.3
fraction binding ligand without competitor
1.2
1
0.8
0.6
0.4
0.2
0
Clasp 2
CD46
Fas
KV1.3
labeled peptide ligand

FIG. 3C

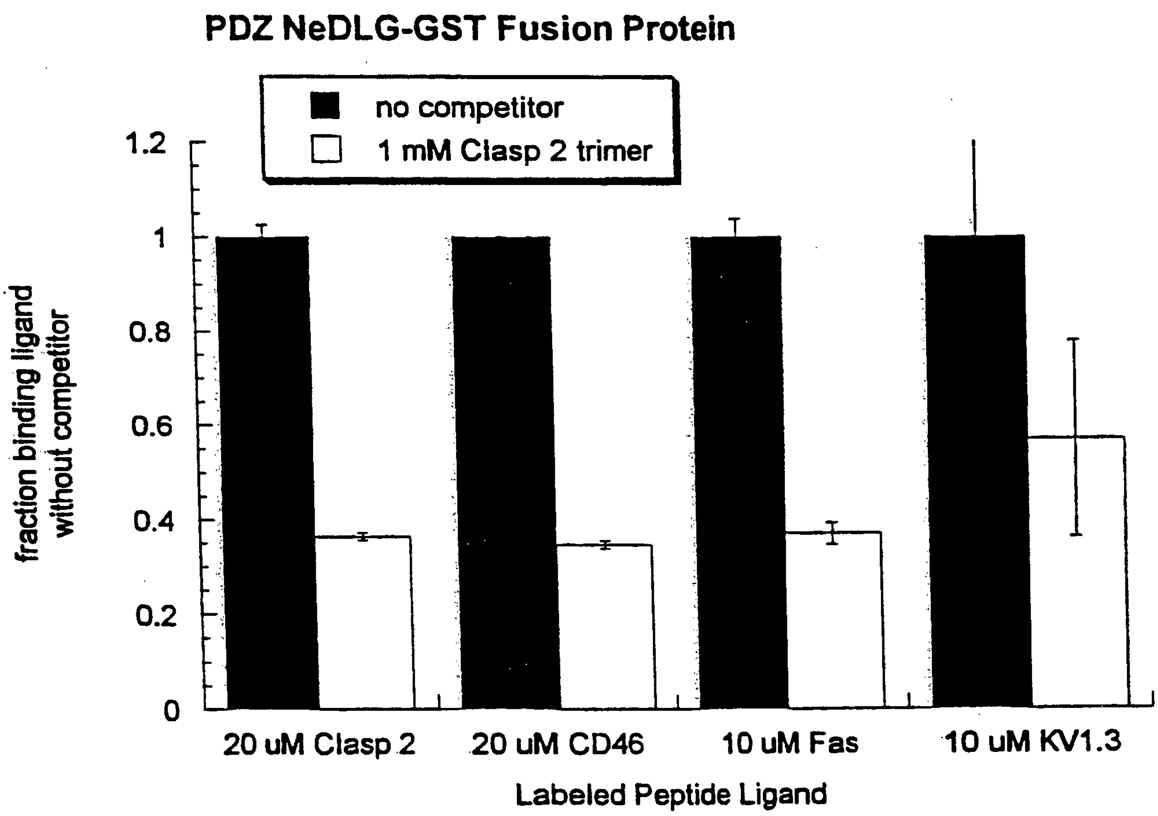
PDZ NeDLG-GST Fusion Protein
no competitor
1 mM Clasp 2 trimer
1.2
1
0.8
0.6
0.4
0.2
0
fraction binding ligand without competitor
20 uM Clasp 2
20 uM CD46
10 uM Fas
10 uM KV1.3
Labeled Peptide Ligand

FIG. 3D

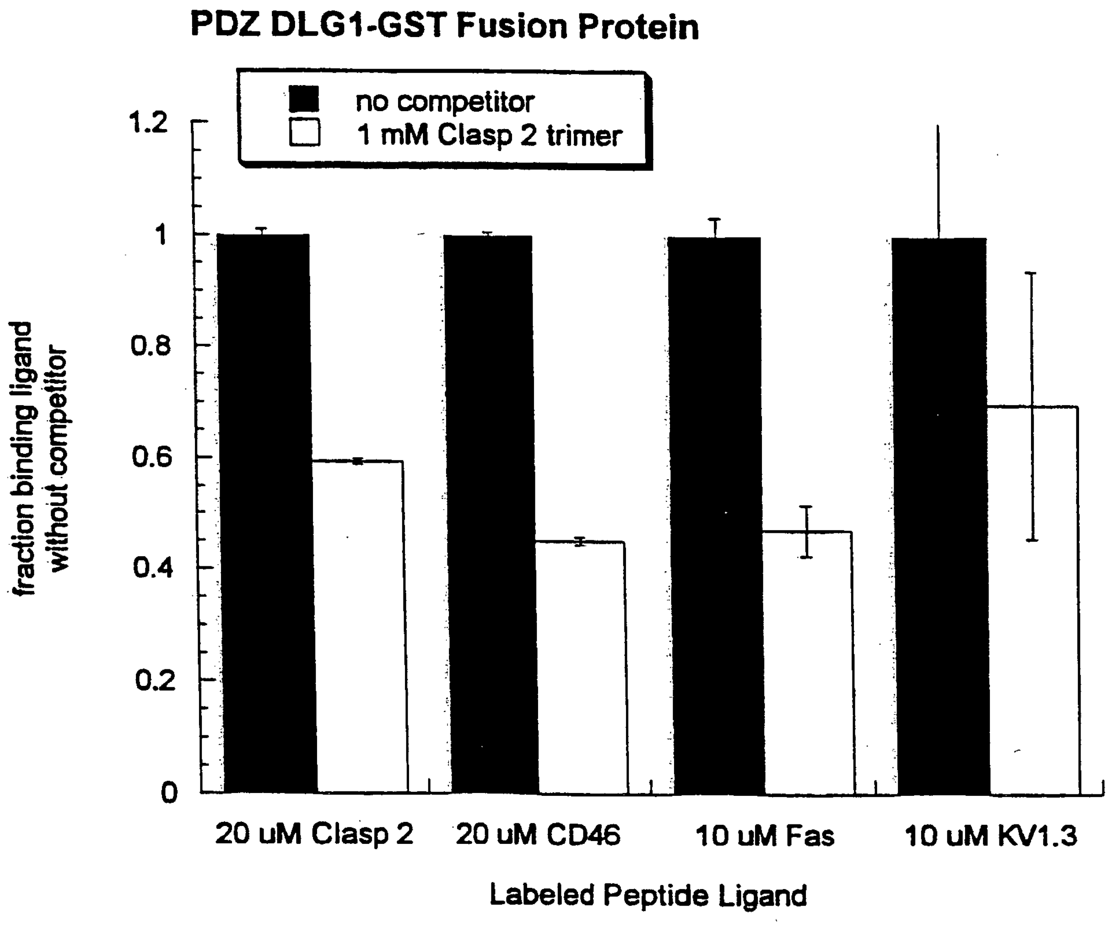
PDZ DLG1-GST Fusion Protein
no competitor
1 mM Clasp 2 trimer
1.2
1
0.8
0.6
0.4
0.2
0
fraction binding ligand without competitor
20 uM Clasp 2
20 uM CD46
10 uM Fas
10 uM KV1.3
Labeled Peptide Ligand

FIG. 3E

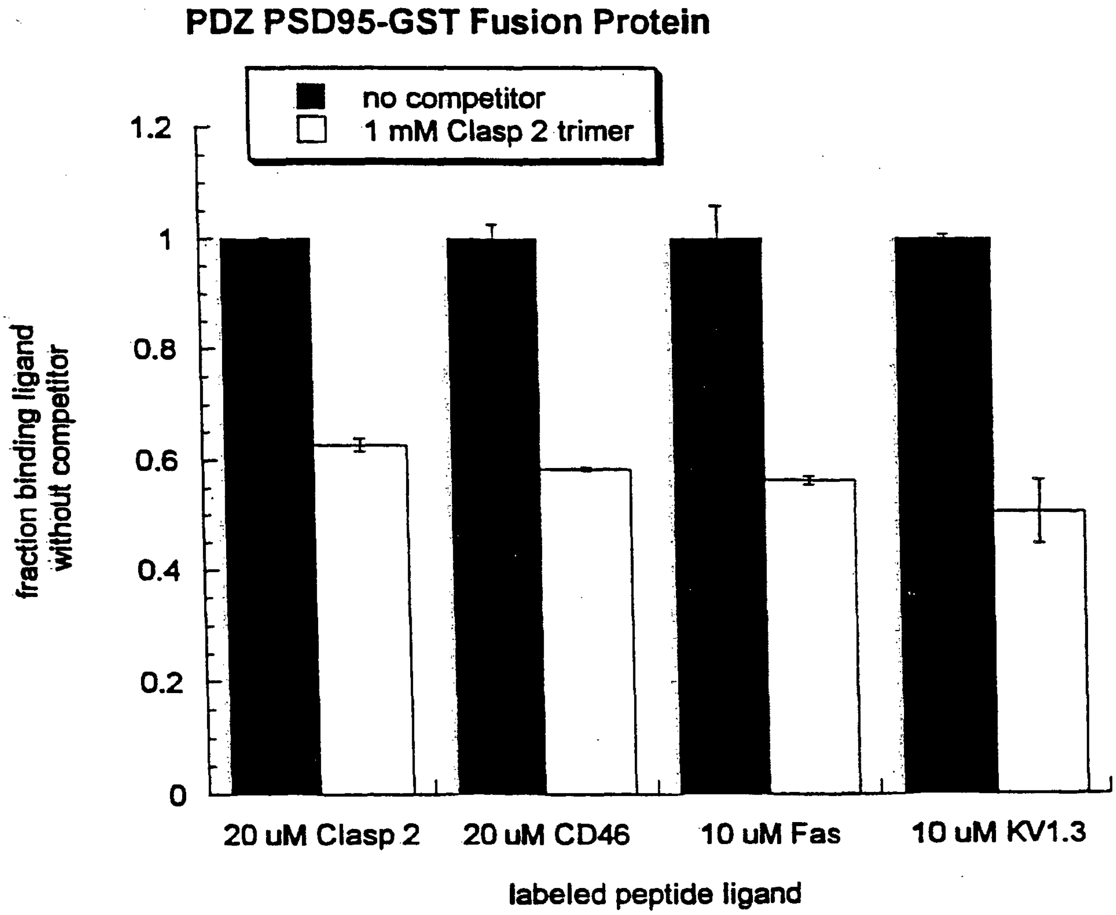
PDZ PSD95-GST Fusion Protein
no competitor
1 mM Clasp 2 trimer
1.2
1
0.8
0.6
0.4
0.2
0
fraction binding ligand without competitor
20 uM Clasp 2
20 uM CD46
10 uM Fas
10 uM KV1.3
labeled peptide ligand

FIG. 3F

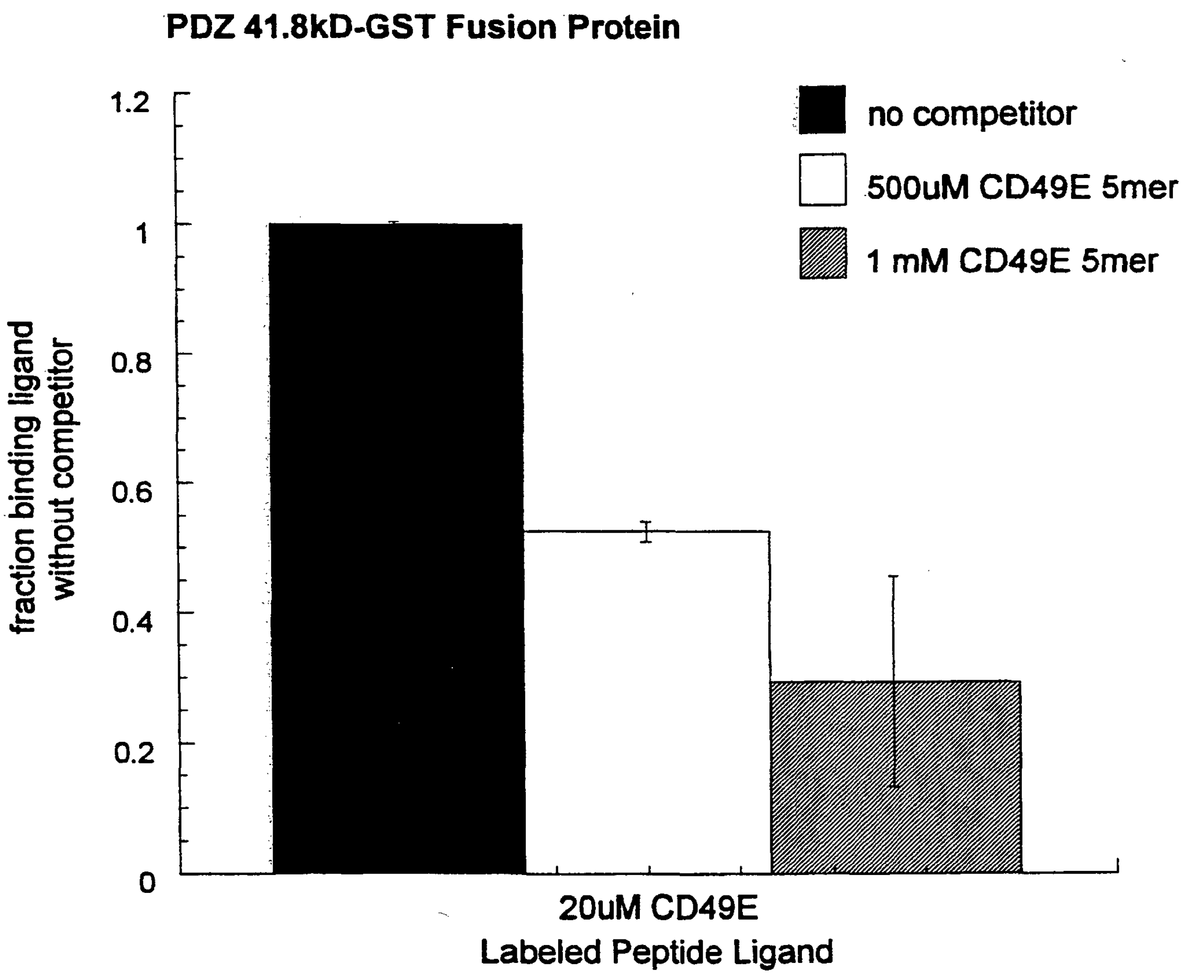
PDZ 41.8kD-GST Fusion Protein
1.2
1
0.8
0.6
0.4
0.2
0
fraction binding ligand without competitor
no competitor
500uM CD49E 5mer
1 mM CD49E 5mer
20uM CD49E
Labeled Peptide Ligand

FIG. 4A

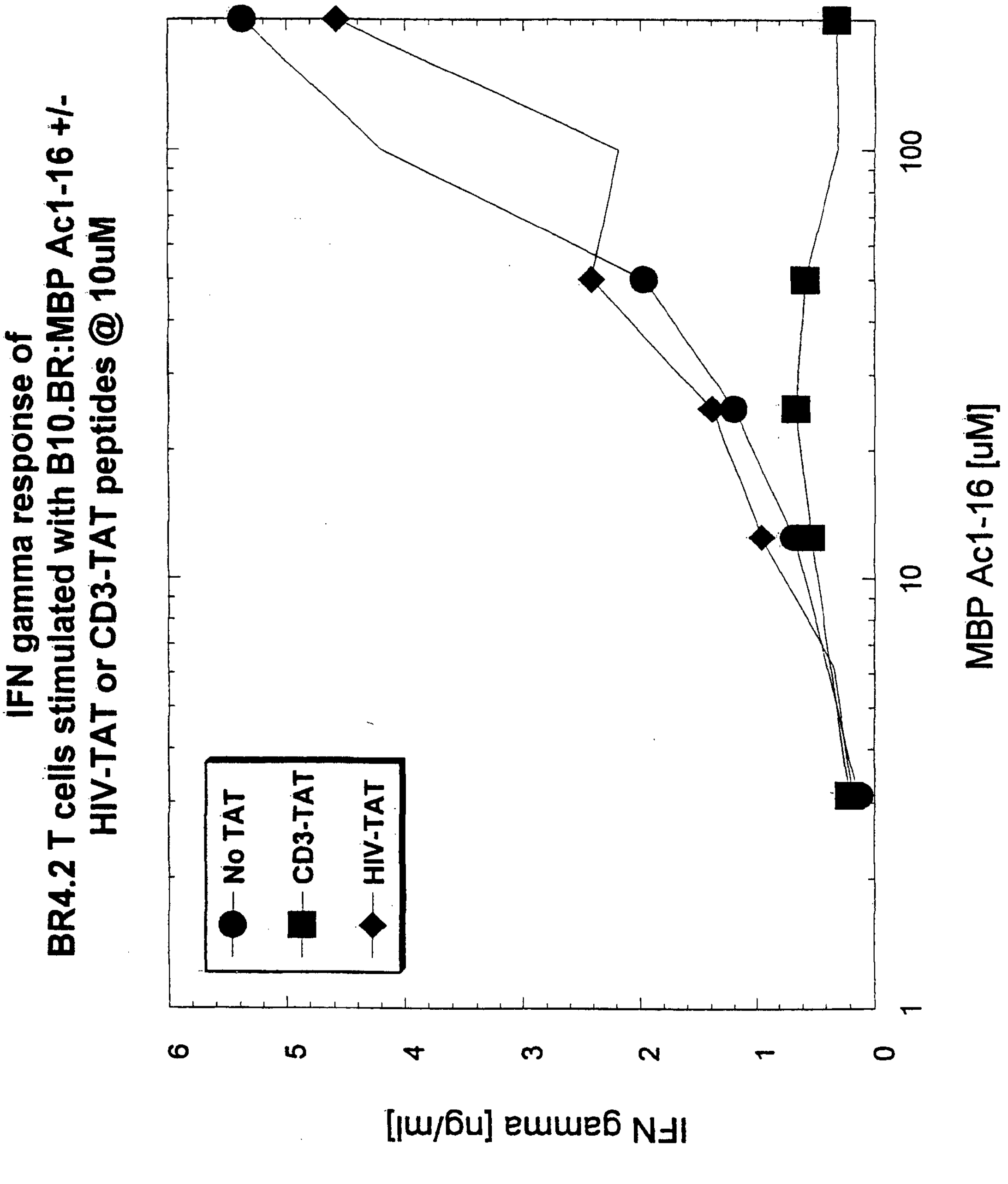
IFN gamma response of
BR4.2 T cells stimulated with B10.BR:MBP Ac1-16 +/-
HIV-TAT or CD3-TAT peptides @ 10uM
No TAT
CD3-TAT
HIV-TAT
IFN gamma [ng/ml]
0
1
2
3
4
5
6
MBP Ac1-16 [uM]
1
10
100

FIG. 4B

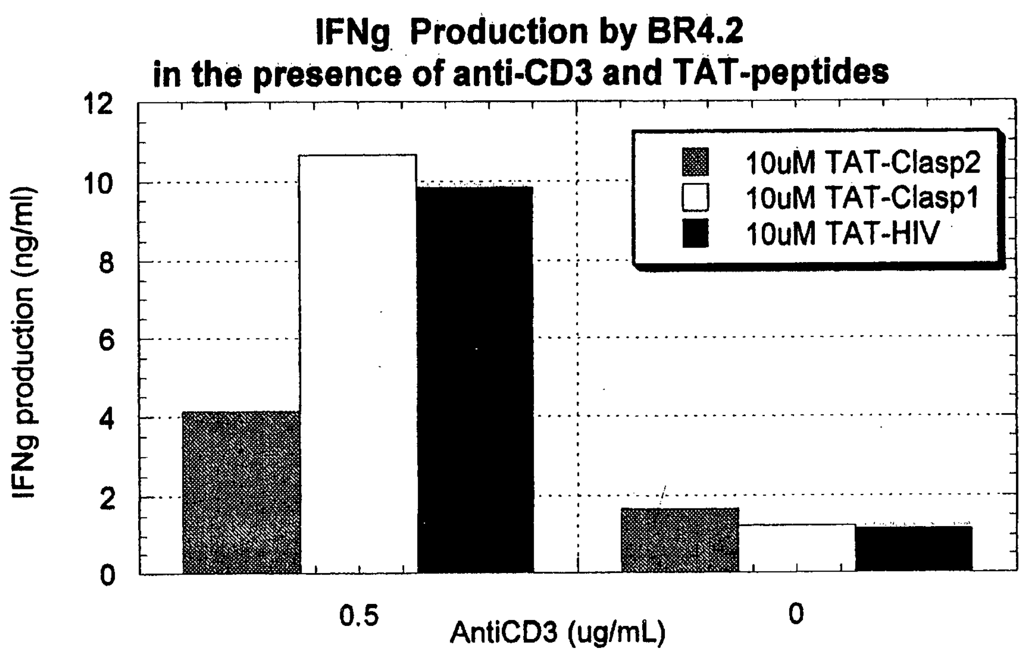
IFNg Production by BR4.2
in the presence of anti-CD3 and TAT-peptides
IFNg production (ng/ml)
12
10
8
6
4
2
0
10uM TAT-Clasp2
10uM TAT-Clasp1
10uM TAT-HIV
0.5
0
AntiCD3 (ug/mL)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9823781 A1
- US 5612895 A, Balaji
- US 3817837 A
- US 3850752 A
- US 3939350 A
- US 3996345 A
- US 4277437 A
- US 4275149 A
- US 4366241 A
- WO 0020434 A
- US SN09547276 A
- US 4108846 A
- US 5096815 A
- US 5223409 A
- US 5198346 A, Ladner
- WO 9418318 A
- WO 9320242 A
- WO 9105058 A
- US 9922996 W
- US 19652700 P
- US 09547276 B
- WO 0010158 A
- WO 0010156 A
- US 5422426 A, Di Marchi
- US 4215051 A, Smith
- US 4946778 A
- US 4235871 A
- US 4501728 A
- US 4837028 A
- US 4957773 A
- US 4603044 A
- US 4925673 A
- US 5907030 A
- US 6033884 A
- US 4980286 A
- WO 9206180 A
- WO 9222635 A
- WO 9220316 A
- WO 9314188 A
- WO 9320221 A
- WO 9840508 A


### Non-patent literature cited in the description


- **GOMPERTS et al.** *Cell,* 1996, vol. 84, 659-662
- **SONGYANG et al.** *Science,* 1997, vol. 275, 73
- **DOYLE et al.** *Cell,* 1996, vol. 88, 1067-1076
- **BARCLAY et al.** The Leucocyte Antigen Facts Book. Academic Press, 1997
- Leucocyte Typing. Springer-Verlag, 1984
- Leukocyte Typing II. Springer-Verlag, 1986
- Leukocyte Typing III. Oxford University Press, 1987
- Leukocyte Typing IV. Oxford University Press, 1989
- VI Internat. Workshop and Conference on Human Leukocyte Differentiation Antigens. *CD Antigens,* 1996, http://www.ncbi.nlm. nih.gov/prow
- **DOYLE, D. A.** *Cell,* 1996, vol. 85, 1067-1076
- A Peptide Backbone Modifications. **SPATOLA.** Chemistry and Biochemistry of Amino Acids, Peptides and Proteins. Marcell Dekker, 1983, vol. 7, 267-357
- **NAGAI.** *Tet. Lett.,* 1985, vol. 26, 647-650
- **FEIGL.** *J. Amer. Chem. Soc,* 1986, vol. 108, 181-182
- **KAHN.** *J. Amer. Chem. Soc.,* 1988, vol. 110, 1638-1639
- **KEMP.** *Tet. Lett.,* 1988, vol. 29, 5057-5060
- **KAHN.** *J. Molec. Recognition,* 1988, vol. 1, 75-79
- **SMITH.** *J. Amer. Chem. Soc,* 1992, vol. 114, 10672-10674
- **BEUSEN.** *Biopolymers,* 1995, vol. 36, 181-200
- **BANERJEE.** *Biopolymers,* 1996, vol. 39, 769-777
- **HIGGINS.** *J. Pept. Res.,* 1997, vol. 50, 421-435
- **HRUBY.** *Biopolymers,* 1997, vol. 43, 219-266
- **FASMAN.** CRC Practical Handbook of Biochemistry and Molecular Biology. CRC Press, Inc, 1989
- **PEARSON, W.R. ; LIPMAN, D.J.** *Proc. Natl. Acad. Sci. U.S.A.,* 1988, vol. 85, 2444
- **W. R. PEARSON.** *Methods Enzymol.,* 1996, vol. 266, 227-258
- **SAMBROOK et al.** MOLECULAR CLONING: A LABORATORY MANUAL. Cold Spring Harbor Laboratory, 1989, vol. 1-3

- **AUSUBEL et al.** CURRENT PROTOCOLS IN MOLECULAR BIOLOGY. Greene Publishing and Wiley-Interscience, 1997
- **CARUTHERS et al.** *Nucleic Acids Res. Symp. Ser.,* 1980, 215-223
- **HORN et al.** *Nucleic Acids Res. Symp. Ser.,* 1980, 225-232
- **ROBERGE et al.** *Science,* 1995, vol. 269, 202
- **GUY ; FIELDS.** *Meth. Enz,* 1997, vol. 289, 67-83
- **WELLINGS ; ATHERTON.** *Meth. Enz,* 1997, vol. 289, 44-67
- **DOYLE et al.** *Cell,* 1996, vol. 85, 1067
- **MARFATIA et al.** *J. Biol. Chem.,* 1997, vol. 272, 24191
- **PARMLEY ; SMITH.** *Adv. Exp. Med. Biol,* 1989, vol. 251, 215-218
- **SCOTT ; SMITH.** *Science,* 1990, vol. 249, 386-390
- **FOWLKES et al.** *BioTechniques,* 1992, vol. 13, 422-427
- **OLDENBURG et al.** *Proc. Natl. Acad. Sci. USA,* 1992, vol. 89, 5393-5397
- **YU et al.** *Cell,* 1994, vol. 76, 933-945
- **STAUDT et al.** *Science,* 1988, vol. 241, 577-580
- **BOCK et al.** *Nature,* 1992, vol. 355, 564-566
- **TUERK et al.** *Proc. Natl. Acad. Sci. USA,* 1992, vol. 89, 6988-6992
- **ELLINGTON et al.** *Nature,* 1992, vol. 355, 850-852
- **REBAR ; PABO.** *Science,* 1993, vol. 263, 671-673
- **PARMLEY ; SMITH.** *Gene,* 1988, vol. 73, 305-318
- **FIELDS ; SONG.** *Nature,* 1989, vol. 340, 245-246
- **CHIEN et al.** *Proc. Natl. Acad. Sci. USA,* 1991, vol. 88, 9578-9582
- **FODOR et al.** *Science,* 1991, vol. 251, 767-773
- **HOUGHTEN et al.** *Nature,* 1991, vol. 354, 84-86
- **LAM et al.** *Nature,* 1991, vol. 354, 82-84
- **MEDYNSKI.** *Bio/Technology,* 1994, vol. 12, 709-710
- **GALLOP et al.** *J. Medicinal Chemistry,* 1994, vol. 37 (9), 1233-1251
- **OHLMEYER et al.** *Proc. Natl. Acad. Sci. USA,* 1993, vol. 90, 10922-10926
- **ERB et al.** *Proc. Natl. Acad. Sci. USA,* 1994, vol. 91, 11422-11426
- **HOUGHTEN et al.** *Biotechniques,* 1992, vol. 13, 412
- **JAYAWICKREME et al.** *Proc. Natl. Acad. Sci. USA,* 1994, vol. 91, 1614-1618
- **SALMON et al.** *Proc. Natl. Acad. Sci. USA,* 1993, vol. 90, 11708-11712
- **BRENNER ; LERNER.** *Proc. Natl. Acad. Sci. USA,* 1992, vol. 89, 5381-5383
- **DEVLIN et al.** *Science,* 1990, vol. 249, 404-406
- **CHRISTIAN, R.B. et al.** *J. Mol. Biol.,* 1992, vol. 227, 711-718
- **LENSTRA.** *J. Immunol. Meth,* 1992, vol. 152, 149-157
- **KAY et al.** *Gene,* 1993, vol. 128, 59-65
- **MATTHEAKIS et al.** *Proc. Natl. Acad. Sci. USA,* 1994, vol. 91, 9022-9026
- **BUNIN et al.** *Proc. Natl. Acad. Sci. USA,* 1994, vol. 91, 4708-4712
- **SIMON et al.** *Proc. Natl. Acad. Sci. USA,* 1992, vol. 89, 9367-9371
- **OSTRESH et al.** *Proc. Natl. Acad. Sci. USA,* 1994, vol. 91, 11138-11142
- **CRESS et al.** *Cancer Metastasis R,* 1995
- **COHEN et al.** *J. Cell. Biol.,* 1998, vol. 142, 129-138
- **GROOTJANS et al.** *PNAS,* 1997, vol. 94, 13683-13688
- **HSUEH et al.** *J. Cell. Biol.,* 1998, vol. 142, 139-151
- **SALOMON et al.** *Blood,* 1997, vol. 89, 2461-2471
- **ST-PIERRE et al.** *Eur. J. Immunol.,* 1996, vol. 26, 2050-2055
- **BELL et al.** *Int. Immunol.,* 1995, vol. 7, 1861-1871
- **PASLOSKE et al.** *Ann Rev Med,* 1994, vol. 45, 283
- **OCKENHOUSE et al.** *J. Exp. Med.,* 1992, vol. 176, 1183
- **SOLEZK et al.** *Kidney Int.,* 1997, vol. 51, 1476
- **TEDLA et al.** *Clin. Exp. Immunol.,* 1999, vol. 117, 92-99
- **KUSTERER et al.** *Exp Clin Endocrinol Diabetes,* 1999, vol. 107, 102-107
- **BONOMINI et al.** *Nephron,* 1998, vol. 79, 399
- **SUASSUNA et al.** *Kidney Int.,* 1994, vol. 46, 443
- **FERRI et al.** *Hypertension,* 1999, vol. 34, 568
- **LEWIS ; CAHALAN.** *Ann. Rev. Immunol.,* 1995, vol. 13, 623
- *J. Biol. Chem.,* 1997, vol. 272, 26899
- **SHIBUYA et al.** *Immunity,* 1999, vol. 11, 615-623
- **SHIBUYA et al.** *J. Immun.,* 1998, vol. 161, 1671-1676
- **HANADA et al.** *J Biol Chem,* 1997, vol. 272, 26899
- **ZHOU ; TEDDER.** *J. Immunol.,* 1995, vol. 154, 3821-3835
- **ZHOU et al.** *J. Immunol.,* 1992, vol. 149, 735-742

- **CZERNIECKI et al.** *J. Immunol.,* 1997, vol. 159, 3823-3837
- **BARCLAY et al.** The Leukocyte Antigen Facts Book. Academic Press, 1997
- **BAJORATH.** *Proteins,* 2000, vol. 39, 103-111
- **TAHER et al.** *J. Biol. Chem.,* 1996, vol. 271, 2863-2867
- **ARUFFO.** *J. Clin. Invest,* 1996, vol. 98, 2191-2192
- **EICHLER et al.** *Scand. J Immunol.,* 1994, vol. 39, 111-115
- **PICKL et al.** *Leukocyte Typing,* 1995, vol. V, 1151-1153
- **LE VAN KIM et al.** *J. Biol. Chem.,* 1989, vol. 264, 20407-20414
- **MARFATIA et al.** *J. Biol. Chem.,* 1997, vol. 272, 24191-24197
- **REID et al.** *Blood,* 1987, vol. 69, 1068-1072
- **AHUJA, SK ; MURPHY, PM.** *J Biol Chem,* 1996, vol. 271, 20545-50
- **WEISS ; LITTMAN.** *Cell,* 1994, vol. 76, 263-274
- **BARCLAY et al.** The Leucocyte antigen facts book. Academic Press, 1997
- **LISZEWSKI et al.** *Adv. Immunol.,* 1999, vol. 61, 201-283
- **MANCHESTER et al.** *Proc. Natl Acad. Sci. USA,* 1994, vol. 91, 2161
- **OKADA et al.** *Proc. Natl Acad. Sci. USA,* 1995, vol. 92, 2489-2493
- **JURIANZ et al.** *Mol Immunol,* 1999, vol. 36, 929-939
- **AHUJA, SK ; MURPHY, PM.** *J Biol Chem,* 1996, vol. 271, 20545-50
- **NAGASE.** *DNA Res,* 1996, vol. 3, 321-329
- **NISHIHARA, H.** *Hokkaido Igaku Zasshi,* 1999, vol. 74, 157
- **TURNER, H.** *Nature,* 1999, vol. 402, B24
- **TAKATSU et al.** *Adv. Immunol.,* 1994, vol. 57, 145-190
- **DOBNER et al.** *Eur J. Immunol.,* 1992, vol. 22, 2795-2799
- **FLYNN et al.** *J. Exp. Med.,* 1998, vol. 188, 297-304
- **FORSTER et al.** *Cell,* 1996, vol. 87, 1037-1047
- **GUNN et al.** *Nature,* 1998, vol. 391, 799-803
- **FORSTER et al.** *Cell Mol Biol,* 1994, vol. 40, 381-387
- **GEE et al.** *J Biological Chem.,* 1998, vol. 273, 21980-87
- **KOZLOV et al.** *Biochemistry,* 2000, vol. 39, 2572
- **YANAGISAWA et al.** *J. Biol. Chem.,* 1997, vol. 272, 8539
- **VIDAL et al.** *J. Immunol.,* 1999, vol. 163, 4811
- Organic Syntheses Collective. John Wiley & Sons, Inc,
- **AL-OBEIDI.** *Mol. Biotechnol.,* 1998, vol. 9, 205-223
- **HRUBY.** *Curr. Opin. Chem. Biol.,* 1997, vol. 1, 114-119
- **OSTERGAARD.** *Mol. Divers,* 1997, vol. 3, 17-27
- **OSTRESH.** *Methods Enzymol.,* 1996, vol. 267, 220-234
- **CREIGHTON.** Protein Structures And Molecular Principles. W.H. Freeman and Co, 1983
- **MANIATIS et al.** Molecular Cloning A Laboratory Manual. Cold Spring Harbor Laboratory, 1989
- **AUSUBEL et al.** Current Protocols in Molecular Biology. Greene Publishing Associates and Wiley Interscience, 1989
- **BRISSON et al.** *Nature,* 1984, vol. 310, 511-514
- **TAKAMATSU et al.** *EMBO J.,* 1987, vol. 6, 307-311
- **CORUZZI et al.** *EMBO J.,* 1984, vol. 3, 1671-1680
- **BROGLIE et al.** *Science,* 1984, vol. 224, 838-843
- **GURLEY et al.** *Mol. Cell. Biol,* 1986, vol. 6, 559-565
- **WEISSBACH ; WEISSBACH.** Methods for Plant Molecular Biology. Academic Press, 1988, 421-463
- **GRIERSON ; COREY.** Plant Molecular Biology. Blackie, 1988
- **SMITH et al.** *J. Virol.,* 1983, vol. 46, 584
- Current Protocols in Molecular Biology. Greene Publish. Assoc. & Wiley Interscience, vol. 2
- **LOGAN ; SHENK.** *Proc. Natl. Acad. Sci. USA,* 1984, vol. 81, 3655-3659
- **MACKETT et al.** *Proc. Natl. Acad. Sci. USA,* 1982, vol. 79, 7415-7419
- **MACKETT et al.** *J. Virol,* 1984, vol. 49, 857-864
- **PANICALI et al.** *Proc. Natl. Acad. Sci. USA,* 1982, vol. 79, 4927-4931
- **KOEHLER ; MILSTEIN.** *Nature,* 1975, vol. 256, 495-497
- **KOSBOR et al.** *Immunology Today,* 1983, vol. 4, 72
- **COTE et al.** *Proc. Natl. Acad. Sci. U.S.A.,* 1983, vol. 80, 2026-2030
- **COLE et al.** Monoclonal Antibodies and Cancer Therapy. Alan R. Liss, Inc, 1985, 77-96
- **MORRISON et al.** *Proc. Natl. Acad. Sci. U.S.A.,* 1984, vol. 81, 6851-6855
- **NEUBERGER et al.** *Nature,* 1984, vol. 312, 604-608
- **TAKEDA et al.** *Nature,* 1985, vol. 314, 452-454
- **HUSE et al.** *Science,* 1989, vol. 246, 1275-1281

- **SCOPES.** Protein Purification: Principles and Practice. Springer-Verlag, 1984
- **LIVINGSTONE.** Methods Enzymology: Immunoaffinity Chromatography of Proteins. 1974, vol. 34, 723-731
- **WINDHAGEN A et al.** *Immunity,* 1995, vol. 2 (4), 373-80
- **HARDING et al.** *Proc. Natl. Acad. Sci.,* 1989, vol. 86, 4230-4
- **SIRAGANIAN et al.** *TIPS,* 1983, vol. 4, 432-437
- **PETERS et al.** *Blood,* 1988, vol. 72, 1310-5
- **LANZAVECCHIA, A. et al.** *Eur. J. Immun.,* 1983, vol. 13, 733-738
- *J. Biol. Chem.,* 1997, vol. 272 (23), 14562-14570
- **MCCONNELL, H.M. et al.** *Science,* 1992, vol. 257, 1906-1912
- **MCCONNELL, H.M.** *Proc. Natl. Acad. Sci.,* 1995, vol. 92, 2750-2754
- **MISHELL, B. B. ; S. M. SHIIGI.** Selected Methods in Cellular Immunology. W. H. Freeman and Company, 1980
- **DUTTON, R. W. ; PEARCE, J. D.** *Nature,* 1962, vol. 194, 93
- **BARLTROP, J.A. et al.** *Bioorg. & Med. Chem. Lett.,* 1991, vol. 1, 611
- **MOSMANN, T.** *J. Immunol. Methods,* 1983, vol. 65, 55
- **GRATZNER, H.G.** *Science,* 1982, vol. 218, 474-475
- **VERMES, I.** *J. Immunol. Methods,* 1995, vol. 180, 39-52
- *Cell,* 1998, vol. 93, 851-861
- *MCB,* 1996, vol. 12, 7151-7160
- **FULMER et al.** *Am. J. Anat.,* 1963, vol. 113, 273
- **JOCKUSCH et al.** *Exp. Neurol.,* 1983, vol. 81, 749
- Measuring Cell Degranulation. **BERGE et al.** Immunology Method Manual. Academic Press, Ltd, 1997, 1436-1440
- **SIRAGANIAN.** Histamine Secretion from Mast Cells and Basophil. *TIPS,* 1983, vol. 4, 432-437
- **ROBBINS et al.** *Cell,* 1991, vol. 64, 615
- **BIRD et al.** *Science,* 1988, vol. 242, 423-426
- **HUSTON et al.** *Proc. Natl. Acad Sci. U.S.A.,* 1988, vol. 85, 5979-5883
- **CHAUDHARY et al.** *Proc. Natl. Acad. Sci. U.S.A.,* 1990, vol. 87, 1066-1070
- **DEROSSI et al.** *Trends in Cell Biol.,* 1998, vol. 8, 84
- **VIVES et al.** *J. Biol. Chem.,* 1997, vol. 272, 16010
- **NAGAHARA et al.** *Nat. Med.,* 1998, vol. 4, 1449
- **DEROSSI et al.** *J. Biol. Chem.,* 1994, vol. 261, 10444
- **ELLIOT ; D'HARE.** *Cell,* 1997, vol. 88, 223-233
- **AVRARNEAS et al.** *Proc. Natl Acad. Sci. U.S.A.,* 1998, vol. 95, 5601-5606
- **FUJIHARA.** *EMBO J.,* 1999, vol. 18, 411-419
- **POOGA et al.** *FASEB J.,* 1998, vol. 12, 67-77
- **SZOKA et al.** *Ann. Rev. Biophys. Bioeng.,* 1980, vol. 9, 467
- The Leucocyte Antigen Facts Book. Academic Press, 1997
- **GOLDSPIEL et al.** *Clinical Pharmacy,* 1993, vol. 12, 488-505
- **WU ; WU.** *Biotherapy,* 1991, vol. 3, 87-95
- **TOLSTOSHEV.** *Ann. Rev. Pharmacol. Toxicol,* 1993, vol. 32, 573-596
- **MULLIGAN.** *Science,* 1993, vol. 260, 926-932
- **MORGAN ; ANDERSON.** *Ann. Rev. Biochem,* 1993, vol. 62, 191-217
- *TIBTECH,* May 1993, vol. 11 (5), 155-215
- Current Protocols in Molecular Biology. John Wiley & Sons, 1993
- **KRIEGLER.** Gene Transfer and Expression, A Laboratory Manual. Stockton Press, 1990
- **KOLLER ; SMITHIES.** *Proc. Natl. Acad. Sci. USA,* 1989, vol. 86, 8932-8935
- **ZIJLSTRA et al.** *Nature,* 1989, vol. 342, 435-438
- **WU ; WU.** *J. Biol. Chem.,* 1987, vol. 262, 4429-4432
- **KOZARSKY ; WILSON.** *Current Opinion in Genetics and Development,* 1993, vol. 3, 499-503
- **ROSENFELD et al.** *Science,* 1991, vol. 252, 431-434
- **ROSENFELD et al.** *Cell,* 1992, vol. 68, 143-155
- **MASTRANGELI et al.** *J. Clin. Invest,* 1993, vol. 91, 225-234
- **WALSH et al.** *Proc. Soc. Exp. Biol. Med,* 1993, vol. 204, 289-300
- **MILLER et al.** *Meth. Enzymol.,* 1993, vol. 217, 581-599
- **BOESEN et al.** *Biotherapy,* 1994, vol. 6, 291-302
- **CLOWES et al.** *J. Clin. Invest,* 1994, vol. 93, 644-651
- **KIEM et al.** *Blood,* 1994, vol. 83, 1467-1473
- **SALMONS ; GUNZBERG.** *Human Gene Therapy,* 1993, vol. 4, 129-141
- **GROSSMAN ; WILSON.** *Curr. Opin. in Genetics and Devel.,* 1993, vol. 3, 110-114
- **LOEFFLER ; BEHR.** *Meth. Enzymol.,* 1993, vol. 217, 599-618
- **COHEN et al.** *Meth. Enzymol.,* 1993, vol. 217, 618-644
- **CLINE.** *Pharmac. Ther.,* 1985, vol. 29, 69-92
- **FINGL et al.** The Pharmacological Basis of Therapeutics. 1975, 1

- **RABINOWITZ et al.** *Proc. Natl. Acad. Sci. U.S.A.,* 1997, vol. 94, 8702-8707